# EUROPEAN PATENT SPECIFICATION

(11) **EP 1 633 729 B1**
(45) Date of publication and mention of the grant of the patent: **30.07.2008**
(21) Application number: 04748975.2
(22) Date of filing: 02.06.2004
(51) Int. Cl.: C07D 241/20, C07D 403/12, C07D 417/12, C07D 401/12, C07D 413/12, A61K 31/4965, A61K 31/497, A61P 11/06, A61P 29/00

(54) **SULPHONAMIDE COMPOUNDS THAT MODULATE CHEMOKINE RECEPTOR ACTIVITY (CCR4)**
SULPHONAMIDVERBINDUNGEN, DIE DIE CHEMOKINREZEPTORAKTIVITÄT (CCR4) MODULIEREN
COMPOSES SULFAMIDES MODULANT L'ACTIVITE DES RECEPTEURS DES CHIMIOKINES (CCR4)

(30) Priority: 05.06.2003 SE 0301653
(43) Date of publication of application: 15.03.2006
(73) Proprietor: AstraZeneca AB, 151 85 Södertälje (SE)
(72) Inventor: HARRISON, Richard AstraZeneca R & D Charnwood, Loughborough Leicestershire LE11 5RH (GB); METE, Antonio AstraZeneca R & D Charnwood, Loughborough Leicestershire LE11 5RH (GB); TEOBALD, Barry AstraZeneca R & D Charnwood, Loughborough Leicestershire LE11 5RH (GB); WALTERS, Iain AstraZeneca R & D Charnwood, Loughborough Leicestershire LE11 5RH (GB)
(86) International application number: PCT/SE2004/000850
(87) International publication number: WO 2004/108692

(56) References cited:
- WO-A1-02/24665
- WO-A1-03/051870
- WO-A1-03/059893
- WO-A2-02/30357
- US-B1- 6 420 567

## Description

The present invention relates to sulphonamide compounds, processes and intermediates used in their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small-secreted molecules are a growing superfamily of 8-14 kDa proteins characterised by a conserved four cysteine motif. At the present time, the chemokine superfamily comprises three groups exhibiting characteristic structural motifs, the Cys-X-Cys (C-X-C), Cys-Cys (C-C) and Cys-X₃-Cys (C-X₃-C) families. The C-X-C and C-C families have sequence similarity and are distinguished from one another on the basis of a single amino acid insertion between the NH-proximal pair of cysteine residues. The C-X₃-C family is distinguished from the other two families on the basis of having a triple amino acid insertion between the NH-proximal pair of cysteine residues.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils. Examples include human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β), Thymus and Activation Regulated Chemokine (TARC, CCL17) and Macrophage Derived Chemokine (MDC, CCL22).

The C-X₃-C chemokine (also known as fractalkine) is a potent chemoattractant and activator of microglia in the central nervous system (CNS) as well as of monocytes, T cells, NK cells and mast cells.

Studies have demonstrated that the actions of chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family. These receptors represent good targets for drug development since agents which modulate these receptors would be useful in the treatment of disorders and diseases such as those mentioned above.

The present invention therefore provides a compound of formula (I) and pharmaceutically acceptable salts, solvates or N-oxides thereof: 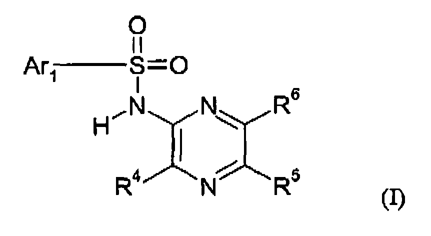 in which:
Ar¹ is phenyl or thienyl, each of which is optionally substituted by one to three substituents R¹, R² and R³ selected from halogen, cyano, CF₃, OCF₃, OC₁₋₆ alkyl or C₁₋₆ alkyl;
R⁴ is C₁₋₆ alkoxy where the alkyl group may form a 3-6 membered saturated ring or may be substituted with 1-3 fluorine atoms or a cyano group; or
OC₁₋₆ alkylR¹¹, or OC₂₋₆ alkyl-X-R¹¹ where the alkyl group may form a 3-6 membered saturated ring and is optionally substituted with 1-3 groups selected from hydroxy, halogen, NR¹⁴R¹⁵, SR¹³, S(O)₂R¹³, S(O)R¹³ or COR¹³;
one of R⁵ or R⁶ is XCH₂C₁₋₄ alkyl where the alkyl group is substituted at one position by R¹¹ and either NR¹⁴R¹⁵ or hydroxy, or R⁵/R⁶ is XR¹⁶ where R¹⁶ is a 4-8 membered saturated ring containing 1-3 heteroatoms selected from nitrogen, oxygen or sulphur and optionally substituted with 1-3 groups selected from hydroxy, cyano, halogen and =O, and R¹⁶ is substituted by R¹¹;
and the other is hydrogen, halogen, amino, NHC₁₋₆ alkyl, N(C₁₋₆ alkyl)₂, C₁₋₆ alkoxy or C₁₋₆ alkyl optionally substituted by one or more fluoro or hydroxyl groups;
X is NR¹³, O, S, S(O) or S(O)₂;
R¹¹ is an aryl group or a 5-7 membered heteroaromatic ring containing 1-4 heteroatoms selected from nitrogen, oxygen or sulphur, which aryl group or heteroaromatic ring can be optionally substituted by 1-3 groups selected from halogen, C(O)NR¹⁴R¹⁵, C(O)OR¹², hydroxy, =O, =S, CN, NO₂, COR¹³, NR¹⁴R¹⁵, X(CH₂)_{q}NR¹⁴R¹⁵, (CH₂)nNR¹⁴R¹⁵, (CH₂)nOH, SR¹³, S(O)R¹³, S(O)₂R¹³
C₁₋₆ alkyl-X-C₁₋₆ alkyl, C₁₋₆ alkyl or C₁₋₆ alkoxy where the alkyl group may form a 3-6 membered ring or is optionally substituted with 1-3 groups selected from hydroxy, halogen, NR¹⁴R¹⁵, SR¹³, S(O)R¹³, S(O)₂R¹³; or
R¹¹ is C(O)NR¹⁴R¹⁵, C(O)OR¹², CH₂OR¹²
R¹² and R¹³ are independently hydrogen or C₁₋₆ alkyl where the alkyl group may be substituted with 1-3 fluorine atoms or may form a saturated 3-6 membered ring;
R¹⁴ and R¹⁵ are independently hydrogen, C₁₋₆ alkyl, C₃₋₆ cycloalkyl, (CH₂)qOH or (CH₂)qNH₂,
or R¹⁴ and R¹⁵ together with the nitrogen atom to which they are attached form a 4-8 membered saturated ring containing 1-3 heteroatoms selected from nitrogen, oxygen and sulphur and optionally substituted by C₁₋₆ alkyl, C₁₋₆ alkyl-OH, or hydroxy; and
n is 1 ,2, 3, 4 or 5; and
q is 2, 3, 4, 5 or 6.

The term aryl includes phenyl and naphthyl. The term alkyl, whether alone or as part of another group, includes straight chain and branched chain alkyl groups. Examples of 5- to 7-membered heteroaromatic rings containing 1 to 4 heteroatoms include thienyl, furanyl, pyrrolyl, imidazolyl, pyridyl, pyrazinyl, pyrimidyl, pyridazinyl, triazinyl, oxazolyl, thiazolyl, isoxazolyl, pyrazolyl, oxadiazolyl, thiadiazolyl, triazolyl and tetrazolyl. Examples of saturated 4- to 8-membered rings containing 1 to 3 heteroatoms include morpholine, piperidine and azetidine. Substituents on any rings can be present in any suitable ring position including suitable substituents on nitrogen atoms.

Certain compounds of formula (I) are capable of existing in stereoisomeric forms- It will be understood that the invention encompasses all geometric and optical isomers of the compounds of formula (I) and mixtures thereof including racemates. Tautomers and mixtures thereof also form an aspect of the present invention.

Preferably Ar¹ is phenyl, more preferably substituted by one or more halogen atoms. Preferred halogen groups for R¹, R² and R³ are chloro, bromo and fluoro. Preferably one of R¹, R² and R³ is hydrogen and the others are chloro, bromo or methyl. More preferably R¹ and R² are chloro at the 2- and 3-positions of the phenyl ring and R³ is hydrogen (i.e. 2,3-dichlorophenyl), R¹ and R³ are chloro at the 2- and 4-positions of the phenyl ring and R² is hydrogen (i.e. 2,4-dichlorophenyl) or R¹ is chloro at the 2-position and R² is methyl at the 3-position of the phenyl ring and R³ is hydrogen (i.e 2-chloro-3-methylphenyl). Most preferably R¹ and R² are chloro at the 2- and 3-positions of the phenyl ring and R³ is hydrogen (i.e. 2,3-dichlorophenyl).

Preferably R⁴ is methoxy.

For R⁵ examples of NR¹⁴R¹⁵ include morpholine, pyrrolidine, NMe₂, NH₂, NHMe, and the groups below:

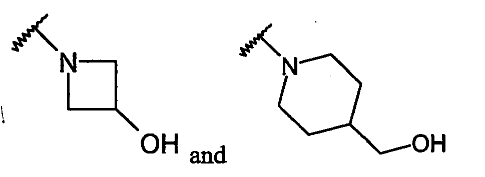

Examples of XCH₂C₁₋₄ alkyl where the alkyl group is substituted at any position by the two groups R¹¹ and either NR¹⁴R¹⁵ or hydroxy include SCH₂CH(Ph)NH₂ and OCH₂CH(pyridyl)OH. Examples of XR¹⁶ where R¹⁶ is substituted by R¹¹ include the groups below:

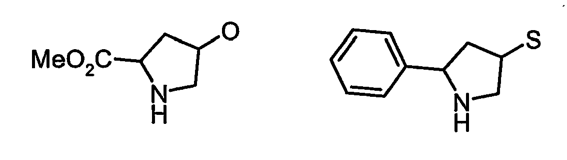

Preferably R⁵ is XCH₂CH(R¹¹)NR¹⁴R¹⁵ where R¹¹ is CO₂Me or CONHMe or a 5 or 6-membered heterocycle and NR¹⁴R¹⁵ is NH₂ or NHMe and X is S or O. More preferably R⁵ is XCH₂CH(R¹¹)NR¹⁴R¹⁵ where R¹¹ is CO₂Me, 2-thiazole or C(O)NHMe, and NR¹⁴R¹⁵ is NH₂ and X is S.

Preferably R⁶ is hydrogen, chloro or methyl.

Preferably X is NR¹³, O, S, S(O) or S(O)₂.

### Preferred compounds of the invention include:

S-[5-[[(2,3-Dichlorophenyl)sulfonyl]amino]-6-methoxypyrazinyl]-D-cysteine, methyl ester
S-[5-[[(2,3-Dichlorophenyl)sulfonyl]amino]-6-methoxypyrazinyl]-L-cysteine, methyl ester
S-[5-[[(2,3-dichlorophenyl)sulfonyl]amino]-6-methoxypyrazinyl]-L-cysteine
(2*R*)-2-amino-3-[[5-[[(2,3-dichlorophenyl)sulfonyl]amino]-6-methoxypyrazinyl]thio]propanamide
(2*R*)-2-amino-3-[[5-[[(2,3-dichlorophenyl)sulfonyl]amino]-6-methoxypyrazinyl]thio]propanamide
(2*R*)-2-amino-3-[[5-[[(2,3-dichlorophenyl)sulfonyl]amino]-6-methoxypyrazinyl]thio]*-N,N-*dimethylpropanamide
*N*-[5-[[(2*R*)-2-amino-3-hydroxypropyl]thio]-3-methoxypyrazinyl]-2,3-dichloro benzenesulfonamide
*S*-[3-chloro-5-[[(2,3-dichlorophenyl)sulfonyl]amino]-6-methoxypyrazinyl]-L-cysteine, methyl ester
*S*-[3-chloro-5-[[(2,3-dichlorophenyl)sulfonyl]amino]-6-methoxypyrazinyl]-L-cysteine
*N*-[5-[[(2*R*)-2-amino-3-hydroxypropyl]thio]-6-chloro-3-methoxypyrazinyl]-2,3-dichlorobenzenesulfonamide
*S*-[5-[[(2,3-Dichlorophenyl)sulfonyl]amino]-6-methoxy-3-methylpyrazinyl]-L-cysteine, methyl ester
*S*-[5-[[(2,3-dichlorophenyl)sulfonyl]amino]-6-methoxy-3-methylpyrazinyl]-L-cysteine,
*N*-(2-Aminoethyl)-*S*-[5-[[(2,3-dichlorophenyl)sulfonyl]amino]-6-methoxypyrazinyl]-L-cysteine, ethyl ester
*N*-[5-[(2R)-2-amino-2-phenylethoxy]-3-methoxypyrazinyl]-2,3-dichlorobenzenesulfonamide
2,3-Dichloro-*N*-[5-[[2-hydroxy-2-(2-thiazolyl)ethyl]thio]-3-methoxypyrazinyl]-benzenesulfonamide
2,3-Dichloro-*N*-[5-[[2-hydroxy-2-(1-methyl-1*H*-imidazol-2-yl)ethyl]thio]-3-methoxypyrazinyl]-benzenesulfonamide, potassium salt
2,3-Dichloro-*N*-[5-[[2-hydroxy-2-(2-oxazolyl)ethyl]thio]-3-methoxypyrazinyl]-benzenesulfonamide
*N*-[5-[[2-amino-2-(2-oxazolyl)ethyl]thio]-3-methoxypyrazinyl]-2,3-dichlorobenzenesulfonamide
2,3-dichloro-*N*-[5-[(2,3-dihydroxypropyl)thio]-3-methoxypyrazinyl] benzenesulfonamide
2,3-dichloro-*N*-[5-[(2-hydroxy-2-phenylethyl)thio]-3-methoxypyrazinyl] benzenesulfonamide
2,3-dichloro-*N*-[5-[[2-hydroxy-2-(3-pyridinyl)ethyl]thio]-3-methoxypyrazinyl] benzenesulfonamide
*N*-[5-[[2-amino-2-(3-pyridinyl)ethyl]thio]-3-methoxypyrazinyl]-2,3-dichloro benzenesulfonamide
2,3-dichloro-*N*-[5-[[2-hydroxy-2-(4-pyridinyl)ethyl]thio]-3-methoxypyrazinyl]benzenesulfonamide
2,3-dichloro-*N*-[5-[[2-hydroxy-2-(2-pyridinyl)ethyl]thio]-3-methoxypyrazinyl]benzenesulfonamide
3-[[5-[[(2,3-dichlorophenyl)sulfonyl]amino]-6-methoxypyrazinyl]thio]-(2R)-2-hydroxypropanoic acid, methyl ester
*N*-[5-[[2-amino-2-(2-pyridinyl)ethyl]thio]-3-methoxypyrazinyl]-2,3-dichlorobenzenesulfonamide
*N*-[5-[[2-amino-2-(1-methyl-1*H*-imidazol-2-yl)ethyl]thio]-3-methoxypyrazinyl]-2,3-dichlorobenzenesulfonamide
(2*R*)-2-amino-3-[[3-chloro-5-[[(2,3-dichlorophenyl)sulfonyl]amino]-6-methoxypyrazinyl]thio]-*N*-methylpropanamide
(2*R*)-2-amino-3-[[5-[[(2,3-dichlorophenyl)sulfonyl]amino]-6-methoxy-3-methylpyrazinyl]thio]-*N*-methylpropanamide
*N*-[5-[[2-amino-2-(2-thiazolyl)ethyl]thio]-3-methoxypyrazinyl]-2,3-dichlorobenzenesulfonamide
*N*-[5-[[(2*R*)-2-Amino-3-hydroxypropyl]oxy]-3-methoxypyrazinyl]-2,3-dichlorobenzenesulfonamide, monohydrochloride
2,3-Dichloro-*N*-[5-[[(2*S*)-2,3-dihydroxypropyl]oxy]-3-methoxypyrazinyl]-benzenesulfonamide
2,3-Dichloro-*N*-[5-[[(2*R*)-2,3-dihydroxypropyl]oxy]-3-methoxypyrazinyl]-benzenesulfonamide
*N*-[5-[[(2*R*)-2-Amino-2-(3-methyl-1,2,4-oxadiazol-5-yl)ethyl]thio]-3-methoxypyrazinyl]-2,3-dichlorobenzenesulfonamide
*N*-[5-[[(2*R*)-2-Amino-2-(3-methyl-1,2,4-oxadiazol-5-yl)ethyl]thio]-3-methoxy-6-methylpyrazinyl]-2,3-dichlorobenzenesulfonamide
*N*-[5-[[(2*R*)-2-Amino-2-(3-methyl-1,2,4-oxadiazol-5-yl)ethyl]thio]-6-chloro-3-methoxypyrazinyl]-2,3-dichlorobenzenesulfonamide
   and pharmaceutically acceptable salts and solvates thereof.

According to the invention there is also provided a process for the preparation of compound (I) which comprises reaction of a compound of formula (II): 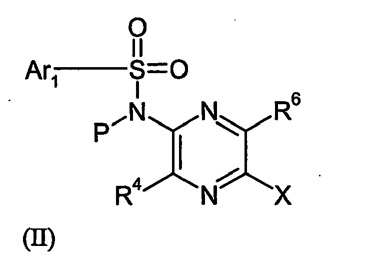 where Ar¹, R⁴ and R⁶ are as defined above, P is a suitable protecting group (e.g. trimethylsilylethoxymethyl (SEM)) and X is a leaving group (e.g. chloro or bromo) with a compound R⁵-H in the presence of a base, and optionally thereafter,
• removing any protecting groups
• forming a pharmaceutically acceptable salt

The reaction may conveniently be carried out in a solvent (e.g. acetonitrile) at room temperature using a base (e.g. caesium carbonate). The protecting group P is typically removed using an acid such as trifluoroacetic acid in a solvent such as dichloromethane at room temperature.

### Compounds of formula (II) may be prepared from compounds of formula (III)

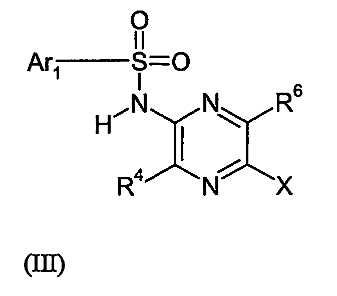

where Ar¹, R⁴, R⁶ and X are as defined above by reaction with a suitable protecting reagent such as 2-(trimethylsilyl)ethoxymethyl chloride (SEM-Cl). The reaction may be performed in a solvent such as dichloromethane in the presence of a base such as diisopropylamine at room temperature.

Compounds of formula (III) where Ar¹, R⁴ and R⁶ are as defined in formula (I) or are protected derivatives thereof and X is a leaving group such as chloro or bromo may be prepared either:-
(a) from compounds of formula (IV): 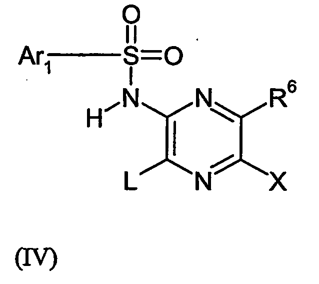 where Ar¹ and R⁶ are as defined above and X and L are leaving groups such as chloro or bromo by reaction with a compound R⁴-H in the presence of a suitable base. The compound R⁴-H may be used as the solvent, such as methanol, in which case a suitable base would be sodium methoxide, and the reaction may be performed at a temperature between 20°C and 100°C, or
(b) from compounds of formula (V) where R⁴ and R⁶ are as defined above and X is a leaving group such as chloro or bromo by reaction with a sulphonyl chloride of formula (VI) where Ar¹ is as defined in formula (I). The reaction may be carried out in a solvent such as dimethoxyethane, tetrahydrofuran or *N-*methylpyrrolidinone in the presence of a base such as sodium hydride or potassium *tert*-butoxide at room temperature.

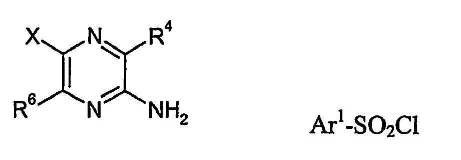

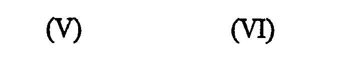

Compounds of formula (IV) may be prepared from compounds of formula (VII) where R⁶, X and L are as defined above by reaction with a sulphonyl chloride of formula (VI). The reaction may be carried out in a solvent such as dimethoxyethane, tetrahydrofuran ot *N-*methylpyrrolidinone in the presence of a base such as sodium hydride or potassium *tert-*butoxide at room temperature.

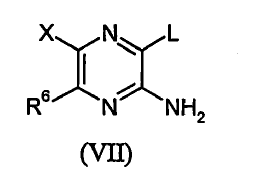

Compounds of formula (V) where R⁴ and R⁶ are as defined in formula (I) and X is a leaving group such as chloro or bromo may be prepared from compounds of formula (VII) where R⁶ and X are as defined above and L is a leaving group such as chloro or bromo by reaction with a compound R⁴-H in the presence of a suitable base. The compound R⁴-H may be used as the solvent, such as methanol, in which case a suitable base would be sodium methoxide, and the reaction may be performed at a temperature between 20°C and 100°C.

Compounds of formula (VII) where R⁶ is as defined in formula (I) and X and L are leaving groups such as bromo may be prepared from the reaction of compounds of the formula (VIII) where R⁶ is as defined above with a brominating agent such as bromine or *N-*bromosuccinimide. The reaction may be performed in a solvent such as chloroform at room temperature or reflux in the presence of a base such as pyridine.

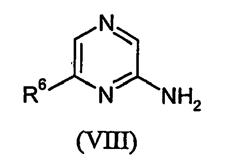

Compounds of formula (VIM) are either commercially available or if R⁶ is methyl may be prepared from compounds of formula (IX) where L is a leaving group such as chloro by reaction with dimethylzinc in the presence of *bis*(diphenylphosphino)propane]nickel(II) chloride in a solvent such as dioxan at reflux.

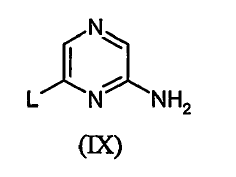

Compounds of formula (VI) and (IX) are commercially available.

In addition, in the case where compound (I) has the formula 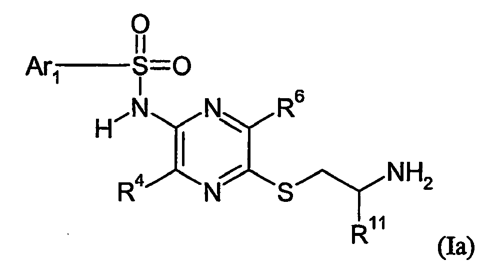 , wherein Ar¹, R⁴, R⁶ and R¹¹ are as defined above, there is provided a process for its preparation which comprises reacting a compound of formula (X), 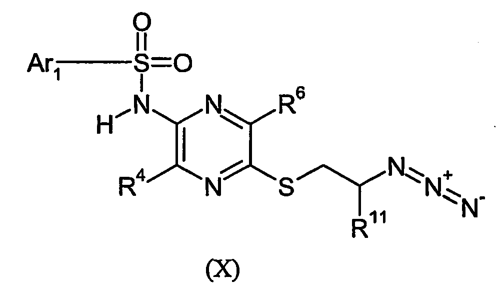 wherein Ar¹, R⁴, R⁶ and R¹¹ are as defined above, with a suitable reducing agent such as triphenylphosphine in the presence of water. The reaction may conveniently be performed in a solvent, for example tetrahydrofuran, at room temperature.

Compounds of formula (X) where Ar¹, R⁴, R⁶ and R¹¹ are as defined above may be prepared from compounds of formula (XI) where Ar¹, R⁴, R⁶ and R¹¹ are as defined above and P is a suitable protecting group such as trimethylsilylethoxymethyl (SEM) by treatment with a suitable acid such as trifluoroacetic acid in a solvent such as dichloromethane. The reaction may be performed at room temperature.

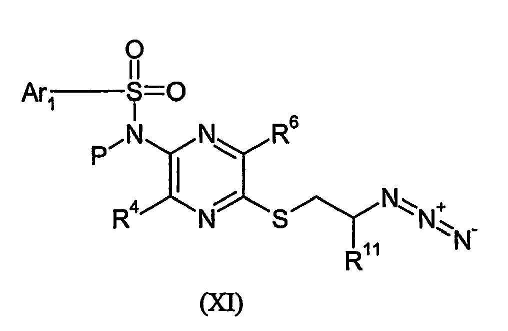

Compounds of formula (XI) where Ar¹, R⁴, R⁶, R¹¹ and P are as defined above may be prepared from compounds of formula (XII) where Ar¹, R⁴, R⁶, R¹¹ and P are as defined above and X is a suitable leaving group such as chloro by treatment with a suitable metal azide such as sodium azide. The reaction may be performed in a solvent such as dimethylformamide at a temperature between 20°C and 100°C.

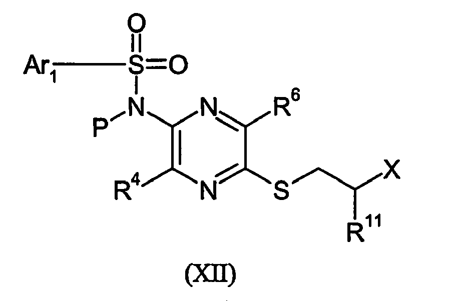

Compounds of formula (XII) where Ar¹, R⁴, R⁶, R¹¹ and P are as defined above and X is chloro may be prepared from compounds of formula (XII) where Ar¹, R⁴, R⁶, R¹¹ and P are as defined above and X is hydroxy by treatment with a suitable chlorinating agent such as methanesulphonyl chloride in the presence of a base such as triethylamine. The reaction may be carried out in a solvent such as dichloromethane at room temperature.

Compounds of formula (XII) where where Ar¹, R⁴, R⁶, R¹¹ and P are as defined above and X is hydroxy may be prepared by either:-
(a) treatment of compounds of formula (XIII) : 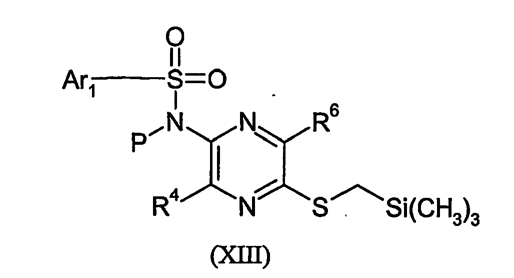 where Ar¹, R⁴, R⁶ and P are as defined above with an aldehyde R¹¹CHO in the presence of a fluoride source such as tetrabutylammonium fluoride. The reaction may be performed in a solvent such as tetrahydrofuran at room temperature.
   Compounds of formula (XIII) where Ar¹, R⁴, R⁶ and P are as defined above may be prepared from compounds of formula (II) where Ar¹, R⁴, R⁶ and P are as defined above by treatment with trimethylsilylmethanethiol in the presence of a base such as cesium carbonate. The reaction may be performed in a solvent such as acetonitrile at room temperature.
(b) treatment of compounds of formula (XIV): 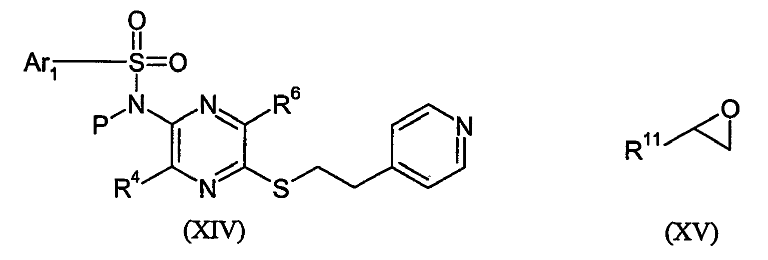 where Ar¹, R⁴, R⁶ and P are as defined above with an epoxide of formula (XV) in the presence of a base such as potassium *tert*-butoxide. The reaction may be performed in a solvent such as tetrahydrofuran at room temperature.
   Compounds of formula (XIV) where Ar¹, R⁴, R⁶ and P are as defined above may be prepared from compounds of formula (II) where Ar¹, R⁴, R⁶ and P are as defined above by treatment with 4-pyridineethanethiol in the presence of a base such as cesium carbonate. The reaction may be performed in a solvent such as acetonitrile at room temperature.
(c) treatment of compounds of formula (XVI): 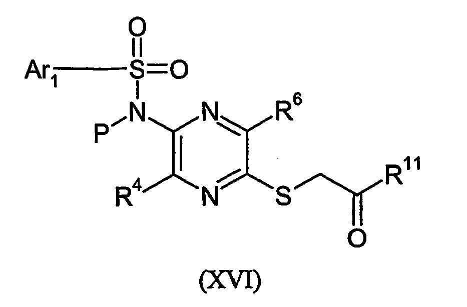
where Ar¹, R⁴, R⁶ and P are as defined above with a reducing agent such as sodium borohydride. The reaction may be performed in a solvent such as ethanol at room temperature.

Compounds of formula (XVI) where Ar¹, R⁴, R⁶, R¹¹ and P are as defined above may be prepared from compounds of formula (XIV) where Ar¹, R⁴, R⁶ and P are as defined above by treatment with an alkylating agent of formula (XVU) where R¹¹ is as defined above and X is a leaving group such as chloro or bromo. The reaction may be performed in a solvent such as tetrahydrofuran at room temperature in the presence of a base such as potassium *tert*-butoxide.

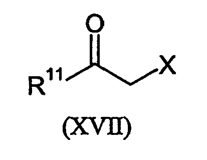

It will be appreciated by those skilled in the art that in the processes of the present invention certain functional groups such as hydroxyl or amino groups in the starting reagents or intermediate compound may need to be protected by protecting groups. Thus, the preparation of the compound of formula (I) may involve, at an appropriate stage, the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley-Interscience (1991).

The compounds of formula (I) above may be converted to a pharmaceutically acceptable salt or solvate thereof, preferably a basic addition salt such as sodium, potassium, calcium, aluminium, lithium, magnesium, zinc, benzathine, chloroprocaine, choline, diethanolamine, ethanolamine, ethyldiamine, meglumine, tromethamine or procaine, or an acid addition salt such as a hydrochloride, hydrobromide, phosphate, acetate, fumarate, maleate, tartrate, citrate, oxalate, methanesulphonate or *p*-toluenesulphonate.

The compounds of formula (I) have activity as pharmaceuticals, in particular as modulators of chemokine receptor (especially CCR4) activity, and may be used in the treatment (therapeutic or prophylactic) of conditions/diseases in human and non-human animals which are exacerbated or caused by excessive or unregulated production of chemokines. Examples of such conditions/diseases include:
(1) (**the respiratory tract**) obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) (**bone and joints**) gout, rheumatoid arthritis, seronegative spondyloarthropathies (including ankylosing spondylitis, psoriatic arthritis and Reiter's disease), Behcet's disease, Sjogren's syndrome and systemic sclerosis;
(3) (**skin**) pruritis, scleroderma, otitus, psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis, lupus;
(4) (**gastrointestinal tract**) Coeliac disease, proctitis, eosinopilic gastro-enteritis, mastocytosis, inflammatory bowel diseases such as Crohn's disease, ulcerative colitis, ileitis and enteritis, food-related allergies which have effects remote from the gut, e.g., migraine, rhinitis and eczema;
(5) (**central and peripheral nervous system**) Neurodegenerative diseases and dementia disorders, e.g. Alzheimer's disease, amyotrophic lateral sclerosis and other motor neuron diseases, Creutzfeldt-Jacob's disease and other prion diseases, HIV encephalopathy (AIDS dementia complex), Huntington's disease, frontotemporal dementia, Lewy body dementia and vascular dementia; polyneuropathies, e.g. Guillain-Barré syndrome, chronic inflammatory demyelinating polyradiculoneuropathy, multifocal motor neuropathy, plexopathies; CNS demyelination, e.g. multiple sclerosis, acute disseminated/haemorrhagic encephalomyelitis, and subacute sclerosing panencephalitis; neuromuscular disorders, e.g. myasthenia gravis and Lambert-Eaton syndrome; spinal diorders, e.g. tropical spastic paraparesis, and stiff-man syndrome: paraneoplastic syndromes, e.g. cerebellar degeneration and encephalomyelitis; CNS trauma; migraine; stroke and correctum diseases such as meningitis
**(6) (other tissues and systemic disease)** hepatitis, vasculitis, spondyloarthopathies, vaginitis, glomerulonephritis, myositis, atherosclerosis, Acquired Immunodeficiency Syndrome (AIDS), lupus erythematosus, systemic lupus, erythematosus, Hashimoto's thyroiditis, type I diabetes, nephrotic syndrome, eosinophilia fascitis, hyper IgE syndrome, lepromatous leprosy, and idiopathic thrombocytopenia pupura; post-operative adhesions, and sepsis.
(7) (allograft and xenograft rejection) acute and chronic following, for example, transplantation of kidney, heart, liver, lung, bone marrow, skin and cornea; and chronic graft versus host disease;
(8) Cancer, carcinoma & tumour metastasis, including that of the bladder, breast, colon, kidney, liver, lung, ovary, pancreas, stomach, cervix, thyroid and skin, especially non-small cell lung cancer (NSCLC), malignant melanoma, prostate cancer and squamous sarcoma. Hematopoietic tumors of lymphoid lineage, including acute lymphocytic leukemia, B cell lymphoma and Burketts lymphoma, Hodgkins Lymphoma, Acute Lymphoblastic Leukemia. Hematopoietic tumors of myeloid lineage, including acute and chronic myelogenous leukemias and promyelocytic leukemia. Tumors of mesenchymal origin, including fibrosarcoma and rhabdomyosarcoma, and other tumors, including melanoma, seminoma, tetratocarcinoma, neuroblastoma and glioma.
(9) All diseases that result from a general inbalance of the immune system and resulting in increased atopic inflammatory reactions.
(10)Cystic fibrosis, re-perfusion injury in the heart, brain, peripheral limbs and other organs.
(11) Bum wounds & chronic skin ulcers
(12) Reproductive Diseases (e.g. Disorders of ovulation, menstruation and implantation, Pre-term labour, Endometriosis)
(13) thrombosis
(14) infectious diseases such as HIV infection and other viral infections, bacterial infections.

Thus, the present invention provides a compound of formula (I), or a pharmaceutically-acceptable salt or solvate thereof, as hereinbefore defined for use in therapy.

Preferably the compound of the invention are used to treat diseases in which the chemokine receptor belongs to the CC chemokine receptor subfamily, more preferably the target chemokine receptor is the CCR4 receptor.

Particular conditions which can be treated with the compound of the invention are asthma, rhinitis and inflammatory skin disorders, diseases in which there are raised TARC, MDC or CCR4 levels. It is preferred that the compound of the invention is used to treat asthma and rhinitis, especially asthma.

In a further aspect, the present invention provides the use of a compound of formula (I), or a pharmaceutically acceptable salt or solvate thereof, as hereinbefore defined in the manufacture of a medicament for use in therapy.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of chemokine receptor activity, particularly CCR4 activity, is beneficial.

In the context of the present specification, the term "therapy" also includes "prophylaxis" unless there are specific indications to the contrary. The terms "therapeutic" and "therapeutically" should be construed accordingly.

The invention still further provides the use of a compound of formula (I), or a pharmaceutically acceptable salt or solvate thereof, as herein before described in the manufacture of a medicament for use in treating asthma.

For the above-mentioned therapeutic uses the dosage administered will, of course, vary with the compound employed the mode of administration, the treatment desired and the disorder indicated.

The compound of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations-, or systemically, e.g. by oral administration in the form of tablets, capsules, syrups, powders or granules, or by parenteral administration in the form of solutions or suspensions, or by subcutancous administration or by rectal administration in the form of suppositories or transdermally. Preferably the compound of the invention is administered orally.

The invention further relates to combination therapies for the treatment of any one of rheumatoid arthritis, osteoarthritis, osteoporosis, psoriasis, inflammatory bowel diseases, COPD asthma, allergic rhinitis, atopic dermatitis or cancer or the neurodegenerative diseases such as multiple sclerosis, Alzheimer's disease or stroke.

For the treatment of rheumatoid arthritis, the compounds of the invention may be combined with "biological agents" such as TNF-α inhibitors such as anti-TNF monoclonal antibodies (such as Remicade, CDP-870 and Humira) and soluble TNF receptor immunoglobulin molecules (such as Enbrel.reg.). IL-1 receptor antagonist (such as Anakinra) and IL-1 trap, IL-18 receptor, anti-IL-6 Ab, anti-CD20 Ab, anti-IL-15 Ab and CTLA4Ig.

Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin. The cyclooxygenase-2 (COX-2) inhibitors (such as meloxicam, celecoxib , rofecoxib, valdecoxib and etoricoxib) and the cyclo-oxygenase inhibiting nitric oxide donors (CINOD's) and the "disease modifying agents" (DMARDs) such as methotrexate, sulphasalazine, cyclosporine A, lefunomide; ciclesonide; hydroxychloroquine, d-penicillamine, auranofin or parenteral or oral gold.

The present invention still further relates to the combination of a compound of the invention together with a leukotriene biosynthesis inhibitor, 5-lipoxygenase (5-LO) inhibitor or 5-lipoxygenase activating protein (FLAP) antagonist selected from the group consisting of zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; the compound SB-210661; pyridinyl-substituted 2n cyanonaphthalene compounds such as L-739,010; 2-cyanoquinoline compounds such as L-746,530; indole and quinoline compounds such as MK-591, MK-886, and BAY x 1005.

The present invention still further relates to the combination of a compound of the invention together with a receptor antagonists for leukotrienes LTB₄, LTC₄, LTD₄, and LTE₄ selected from the group consisting of the phenothiazin-3-ones such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a phosphodiesterase-4 (PDE4) inhibitor including inhibitors of the isoform PDE4D.

The present invention still further relates to the combination of a compound of the invention together with histaminic H₁ receptor antagonists including cetirizine, loratadine, desloratadine, fexofenadine, astemizole, azelastine, and chlorpheniramine.

The present invention still further relates to the combination of a compound of the invention together with a gastroprotective histaminic H₂ receptor antagonist or the proton pump inhibitors (such as omeprazole)

The present invention still further relates to the combination of a compound of the invention together with an α₁- and α₂-adrenoceptor agonist vasoconstrictor sympathomimetic agent, including propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride.

The present invention still further relates to the combination of a compound of the invention together with anticholinergic agents including ipratropium bromide; tiotropium bromide; oxitropium bromide; pirenzepine; and telenzepine.

The present invention still further relates to the combination of a compound of the invention together with a β₁- to β₄-adrenoceptor agonists including metaproterenol isoproterenol, isoprenaline, albuterol, salbutamol, formoterol, salmeterol, terbutaline, orciprenaline, bitolterol mesylate, and pirbuterol; or methylxanthanines including theophylline and aminophylline; sodium cromoglycate; or muscarinic receptor (M1, M2, and M3) antagonist.

The present invention still further relates to the combination of a compound of the invention together with other modulators of chemokine receptor function such as CCR1, CCR2, CCR2A, CCR2B, CCR3, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family.

The present invention still further relates to the combination of a compound of the invention together with an insulin-like growth factor type I (IGF-1) mimetic.

The present invention still further relates to the combination of compound of the invention together with an inhaled glucocorticoid with reduced systemic side effects, including prednisone, prednisolone, flunisolide, triamcinolone acetonide, beclomethasone dipropionate, budesonide, fluticasone propionate, and mometasone furoate.

The present invention still further relates to the combination of a compound of the invention together with (a) tryptase inhibitors; (b) platelet activating factor (PAF) antagonists; (c) interleukin converting enzyme (ICE) inhibitors; (d) IMPDH inhibitors; (e) adhesion molecule inhibitors including VLA-4 antagonists; (f) cathepsins; (g) MAP kinase inhibitors; (h) glucose-6 phosphate dehydrogenase inhibitors; (i) kinin-B₁ - and B₂ - receptor antagonists; (j) anti-gout agents, e.g., colchicine; (k) xanthine oxidase inhibitors, e.g., allopurinol; (I) uricosuric agents, e.g., probenecid, sulfinpyrazone, and benzbromarone; (m) growth hormone secretagogues; (n) transforming growth factor (TGFβ); (o) platelet-derived growth factor (PDGF); (p) fibroblast growth factor, e.g., basic fibroblast growth factor (bFGF); (q) granulocyte macrophage colony stimulating factor (GM-CSF); (r) capsaicin cream; (s) Tachykinin NK₁ and NK₃ receptor antagonists selected from the group consisting of NKP-608C; SB-233412 (talnetant); and D-4418; and (t) elastase inhibitors selected from the group consisting of UT-77 and ZD-0892 (u) induced nitric oxide synthase inhibitors (iNOS) or (v) chemoattractant receptor-homologous molecule expressed on TH2 cells, (CRTH2 antagonists).

The present invention still further relates to the combination of a compound of the invention together with an inhibitor of matrix metalloproteases (MMPs), i.e., the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), stromelysin-3 (MMP-11), and MMP12 inhibitors.

The compounds of the invention can also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, induced nitric oxide synthase inhibitors (iNOS inhibitors), COX-2 inhibitors such as celecoxib, valdecoxib, rofecoxib and etoricoxib, and the cyclo-oxygenase inhibiting nitric oxide donors (CINOD's) analgesics (such as paracetamol and tramadol), cartilage sparing agents such as diacerein, doxycyline and glucosamine, and intra-articular therapies such as corticosteroids and hyaluronic acids such as hyalgan and synvisc and P2X7 antagonists.

The compounds of the invention can also be used in combination with existing therapeutic agents for the treatment of inflammatory bowel diseases (Ulcerative colitis and Crohn's disease). Suitable agents to be used include sulphasalazine, 5-amino-salicylates, the thiopurines, azathioprine and 6-mecaptorurine and corticosteroids such as budesonide.

The compounds of the invention may also be used in combination with antiviral agents such as Viracept, AZT, aciclovir and famciclovir, and antisepsis compounds such as Valant.

The compounds of the present invention may also be used in combination with cardiovascular agents such as calcium channel blockers, lipid lowering agents such as statins, fibrates, beta-blockers, angiotensin converting enzyme (ACE) inhibitors, Angiotensin-2 receptor antagonists and platelet aggregation inhibitors.

The compounds of the present invention may also be used in combination with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti Alzheimer's drugs such as donepezil, tacrine, COX-2 inhibitors, propentofylline or metryfonate.

The compounds of the present invention may also be used in combination with osteoporosis agents such as roloxifene, droloxifene, lasofoxifene or fosomax and immunosuppressant agents such as FK-506, rapamycin, cyclosporine, azathioprine, and methotrexate.

The compounds of the invention can also be used in combination with existing therapeutic agents for the treatment of cancer. Suitable agents to be used in combination include:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside, hydroxyurea, gemcitabine and paclitaxel (Taxol®); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5a-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD 1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin^{™}], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO99/02166, WO00/40529, WO00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The following examples illustrate the invention. The title and sub-title compounds of the examples and methods were named using the Index naming programme verion 4.53/07 April 200 from Advanced Chemistry Development Inc.

### Example 1

### S-[5-[[(2,3-Dichlorophenyl)sulfonyl]amino]-6-methoxypyrazinyl]-D-cysteine, methyl ester

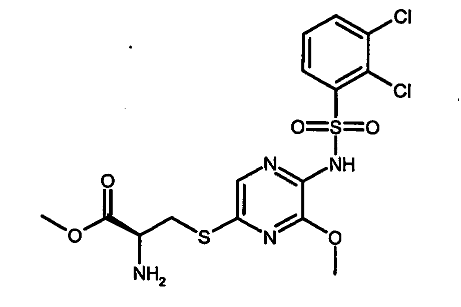

a) 2,3-Dichloro*-N*-(3,5-dichloro-2-pyrazinyl)benzenesulphonamide 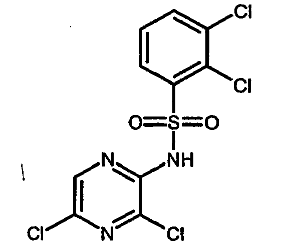 Sodium hydride (1.45g of 60%) was added to 3,5-dichloro-2-pyrazinamine (2.0g) in 1,2-dimethoxyethane (25mL) under nitrogen at room temperature. After 1 hour at room temperature, 2,3-dichlorobenzenesuphonyl chloride (2.94g) was added. After stirring for 30 minutes, 5% aqueous citric acid was added and the product extracted with ethyl acetate (x3). The combined extracts were washed with saturated brine, dried (MgSO₄) and the solvent was evaporated. Chromatography on silica eluting with dichloromethane/methanol mixtures gave the subtitled compound as a white solid (3.0g).
   m/e 372 (M-1⁻, 100%)
   ¹H NMR (D6 DMSO) δ 8.29 (1H, s), 8.06 (1H, dd), 7.94 (1H, dd), 7.57 (1H, t) MP 181-182°C
b) 2,3-Dichloro-*N*-(5-chloro-3-methoxypyrazinyl)benzenesulfonamide 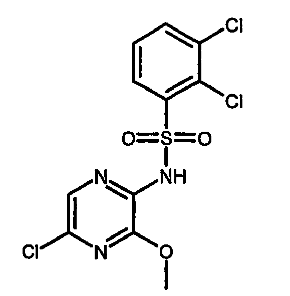 A solution of 2,3-dichloro-*N*-(3,5-dichloro-2-pyrazinyl)benzenesulphonamide (20.0g) and sodium methoxide (7.0g) in methanol (300mL) was heated at reflux for 24 hours. The solvent was evaporated and the residue redissolved in water (500mL). The mixture was acidified to pH~1 with concentrated hydrochloric acid giving a white precipitate which was isolated by filtration and dried to afford the subtitled compound as a white solid (19.0g).
   m/e 368 (M-1⁻, 100%)
   ¹H NMR (CDCl₃) δ 8.27 (1H, d), 7.84 (1H, br s), 7.70 (1H, d), 7.62 (1H, s), 7.41 (1H, t), 4.06 (3H, s).
c) 2,3-Dichloro-*N*-(5-chloro-3-methoxypyrazinyl)-*N*-[[2-(trimethylsilyl)ethoxy]methyl]benzenesulfonamide 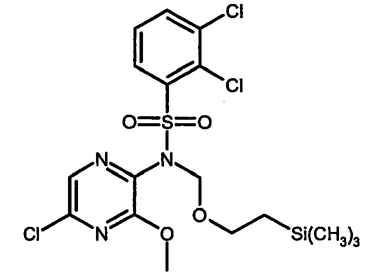 To a stirred solution of 2,3-dichloro-*N*-(5-chloro-3-methoxypyrazinyl)benzenesulfonamide (2.2g) and diisopropylethylamine (1.3mL) in dichloromethane (100mL) was added 2-(trimethylsilyl)ethoxymethyl chloride (1.3mL). After stirring for 30min at room temperature, the reaction mixture was washed with water and brine, and the organic phase dried over magnesium sulphate, filtered and evaporated to give a white solid. This was purified by silica gel chromatography, eluting with 10:1 isohexane:ethyl acetate, to afford the subtitled compound as a white solid (2.8g).
   m/e 498 (M+1⁺, 100%)
   ¹H NMR (CDCl₃) δ 8.00 (1H, s), 7.97 (1H, dd), 7.67 (1H, dd), 7.29 (1H, t), 5.24 (2H, s), 3.91 (3H, s), 3.79-3.73 (2H, m), 0.87-0.82 (2H, m), 0.00 (9H, s).
d) *S*-[5-[[(2,3-Dichlorophenyl)sulfonyl][[2-(trimethylsilyl)ethoxy]methyl]amino]-6-methoxypyrazinyl]-D-cysteine, methyl ester 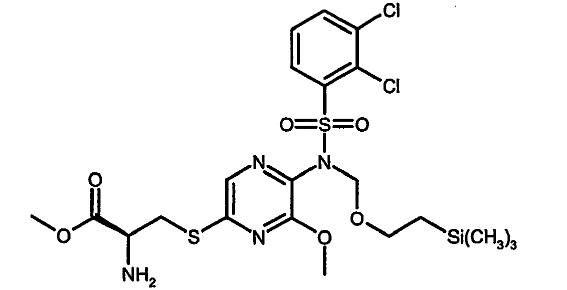 To a stirred solution of 2,3-Dichloro-*N*-(5-chloro-3-methoxypyrazinyl)-*N*-[[2-(trimethylsilyl)ethoxy]methyl]benzenesulfonamide (0.15g) and D-cysteine methyl ester hydrochloride (0.1g) in acetonitrile (5mL) was added caesium carbonate (0.39g) and the reaction mixture stirred at room temperature under an atmosphere of nitrogen for 18 hours. After evaporation of solvent the residue was partitioned between brine and dichloromethane, and the organic phase dried over magnesium sulphate, filtered and evaporated. The crude product was purified by silica gel chromatography, eluting with 2:1 dichloromethane:ethyl acetate, to afford the subtitled compound as a colourless oil (0.09g). m/e 597 (M+1⁺, 100%)
   ¹H NMR (CDCl₃) δ 7.97 (1H, d), 7.89 (1H, s), 7.65 (1H, d), 7.27 (1H, t), 5.23 (2H, s), 3.91 (3H, s), 3.81-3.68 (3H, m), 3.72 (3H, s), 3.26-3.19 (2H, m), 0.89-0.83 (2H, m), 0.00 (9H, s).
e) S-[5-[[(2,3-Dichlorophenyl)sulfonyl]amino]-6-methoxypyrazinyl]-D-cysteine, methyl ester 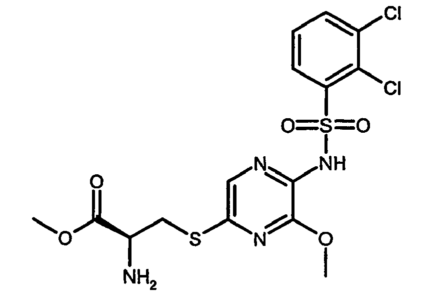 A solution of *S*-[5-[[(2,3-dichlorophenyl)sulfonyl][[2-(trimethylsilyl)ethoxy]methyl]amino]-6-methoxypyrazinyl]-D-cysteine, methyl ester (0.08g) in a mixture of dichloromethane (5mL) and trifluoroacetic acid (3mL) was stirred at room temperature for 1 hour. After evaporation of solvent the residue was purified by silica gel chromatography, eluting with 10:1 dichloromethane:methanol, to afford the title product as a white solid (0.05g).
   m/e 465 (M-1⁻, 100%)
   ¹H NMR (CDCl₃) δ 8.05 (1H, d), 7.94 (1H, d), 7.69 (1H, s), 7.58 (1H, t), 4.36 (1H, t), 3.95 (3H, s), 3.71 (1H, dd), 3.60 (3H, s), 3.46 (1H, dd).

### Example 2

### S-[5-[[(2,3-Dichlorophenyl)sulfonyl]amino]-6-methozypyrazinyl]-L-cysteine, methyl ester

a) S-[5-[[(2,3-Dichlorophenyl)sulfonyl][[2-(trimethylsilyl)ethoxy]methyl]amino]-6-methoxypyrazinyl]-*N*-[(1,1-dimethylethoxy)carbonyl]-L-cysteine, methyl ester 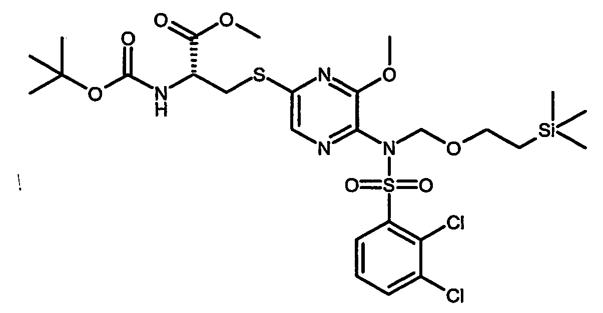 To a solution of 2,3-dichloro-*N*-(5-chloro-3-methoxypyrazinyl)-*N*-[[2-(trimethylsilyl)ethoxy]methyl]benzenesulfonamide (0.300g) and N-[(1,1-dimethylethoxy)carbonyl]-L-cysteine, methyl ester (0.13mL) in acetonitrile (10mL) was added caesium carbonate (0.200g) and the mixture stirred under nitrogen at room temperature for 24 hours. The mixture was added to water and the product extracted with ethyl acetate (x3). The combined extracts were washed with saturated brine, dried (MgSO₄) and the solvent was evaporated to give the subtitled compound as an oil (0.42g). m/e 697 (M-1⁻, 100%)
b) S-[5-[[(2,3-Dichlorophenyl)sulfonyl]amino]-6-methoxypyrazinyl]-L-cysteine, methyl 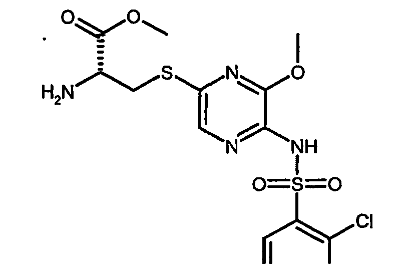 ester
   Procedure as for Example 1 step e) using S-[5-[[(2,3-dichlorophenyl)sulfonyl][[2-(trimethylsilyl)ethoxy]methyl]amino]-6-methoxypyrazinyl]-*N*-[(1,1-dimethylethoxy)carbonyl]-L-cysteine, methyl ester (0.300g). Yield 0.075g. m/e 467 (M+1⁺, 100%)
   ¹H NMR (DMSO) δ 8.17 (2H, bs), 7.96 (1H, dd), 7.68 (1H, dd), 7.40 (1H, t), 7.34 (1H, s), 4.20 (1H, t), 3.83 (3H, s), 3.52 (3H, s), 3.40 (2H, m).
   MP 170-4°C

### Example 3

### S-[5-[[(2,3-Dichlorophenyl)sulfonyl]amino]-6-methozypyrazinyl]-L-cysteine

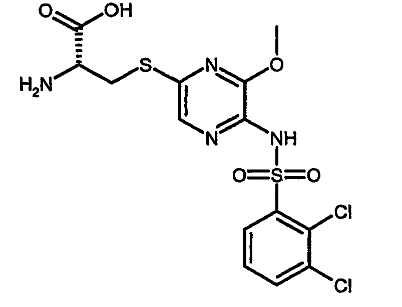

To a stirred solution of S-[5-[[(2,3-dichlorophenyl)sulfonyl]amino]-6-methoxypyrazinyl]-L-cysteine, methyl ester (0.100g) in tetrahydrofuran (20mL) was added a solution of lithium hydroxide (0.090g) in water (5mL).After stirring at room temperature for 4 hours, the reaction mixture was poured into 2M hydrochloric acid and extracted with dichloromethane (x3). The aqueous extract was evaporated to approximately 5mL. The title compound crystallised as a white solid (0.050g).
m/e 453 (M+1⁺, 100%)
¹H NMR (DMSO) δ 8.51 (3H, bs), 8.05 (1H, d), 7.95 (1H, d), 7.71 (1H, s), 7.59 (1H, t), 4.21 (1H, m), 3.96 (3H, s), 3.75 (1H, dd), 3.51 (1H, dd).
MP 180-90°C

### Example 4

### (2R)-2-Amino-3-[[5-[[(2,3-dichlorophenyl)sulfonyl]amino]-6-methoxypyrazinyl]thio]propanamide

a) S-[5-[[(2,3-Dichlorophenyl)sulfonyl][[2-(trimethylsilyl)ethoxy]methyl]amino]-6-methoxypyrazinyl]-*N*-[(1,1-dimethylethoxy)carbonyl]-L-cysteine 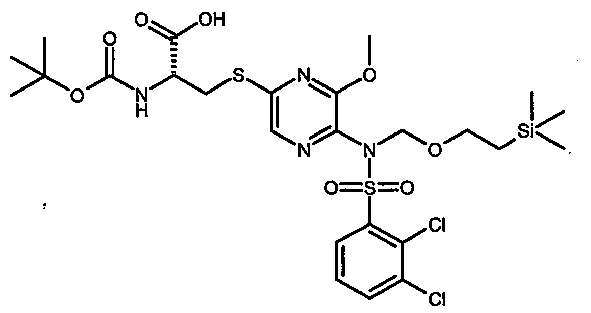 To a stirred solution of S-[5-[[(2,3-dichlorophenyl)sulfonyl][[2-(trimethylsilyl)ethoxy]methyl]amino]-6-methoxypyrazinyl]-*N*-[(1,1-dimethylethoxy)carbonyl]-L-cysteine, methyl ester (0.500g) in tetrahydrofuran (20mL) was added a solution of lithium hydroxide (0.300g) in water (5mL). After stirring at room temperature for 2 days, the reaction mixture was poured into saturated ammonium chloride solution and extracted with ethyl acetate (x2). The combined extracts were washed with saturated brine, dried (MgSO₄) and the solvent was evaporated. The residue was purified by silica gel chromatography, eluting with 3:1 ethyl acetate:methanol, to afford the subtitled product as a solid (0.200g).
   m/e 681 (M-1⁻, 100%)
b) [(1*R*)-2-Amino-1-[[[5-[[(2,3-dichlorophenyl)sulfonyl][[2-(trimethylsilyl)ethoxy]methyl]amino]-6-methoxypyrazinyl]thio]methyl]-2-oxoethyl]carbamic acid, 1,1-dimethylethyl ester. A solution of S-[5-[[(2,3-dichlorophenyl)sulfonyl][[2- 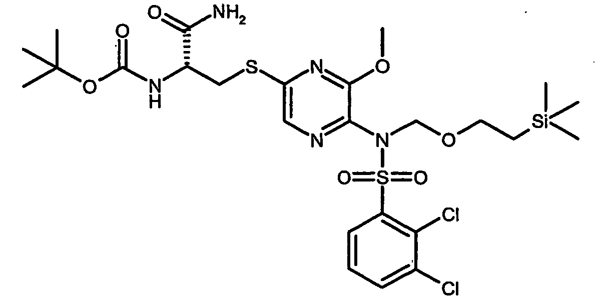 (trimethylsilyl)ethoxy]methyl]amino]-6-methoxypyrazinyl]-*N*-[(1,1-dimethylethoxy)carbonyl]-L-cysteine (0.500g) in tetrahydrofuran (10mL) was cooled to 0°C under nitrogen and isobutyl chloroformate (0.105mL) was added dropwise. After 30 minutes at 0°C 0.88 ammonia solution was added and allowed to warm to room temperature. After stirring at room temperature for 24 hours, the reaction mixture was poured into saturated ammonium chloride solution and extracted with ethyl acetate (x3). The combined extracts were washed with saturated brine, dried (MgSO₄) and the solvent was evaporated to afford the subtitled product as an oil (0.550g).
   m/e 682 (M-1⁻, 100%).
c) (2*R*)-2-Amino-3-[[5-[[(2,3-dichlorophenyl)sulfonyl]amino]-6-methoxypyrazinyl]thio]propanamide 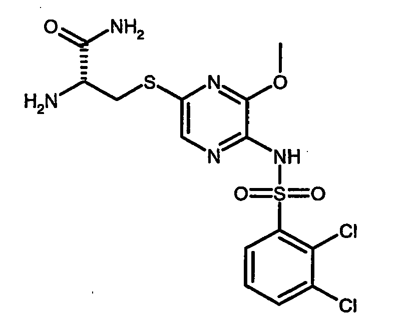 Procedure as for Example 1 step e) using [(1*R*)-2-amino-1-[[[5-[[(2,3-dichlorophenyl)sulfonyl] [[2-(trimethylsilyl)ethoxy]methyl]amino]-6-methoxypyrazinyl]thio]methyl]-2-oxoethyl]carbamic acid, 1,1-dimethylethyl ester (0.250g) Yield 0.150g.
   m/e 452 (M+1⁺, 100%)
   ¹H NMR (DMSO) δ 7.95 (1H, d), 7.77 (1H, s), 7.61 (1H, d), 7.48 (1H, s), 7.36 (2H, bs), 7.35 (1H, t), 7.33 (1H, s), 3.74 (1H, m), 3.28 (1H, m), 3.08 (1H, m).
   MP 148-52°C

### Example 5

### (2R)-2-Amino-3-[[5-[[(2,3-dichlorophenyl)sulfonyl]amino]-6-methoxypyrazinyl]thio]- N-methyl-propanamide

a) [(1*R*)-1-[[[5-[[(2,3-Dichlorophenyl)sulfonyl][[2-(trimethylsilyl)ethoxy]methyl]amino]- 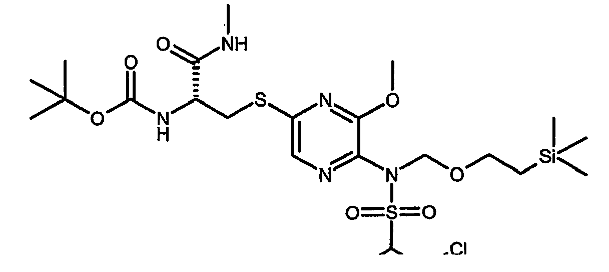 6-methoxypyrazinyl]thio]methyl]-2-(methylamino)-2-oxoethyl]carbamic acid, 1,1-dimethylethyl ester.
   Procedure as for Example 4 step b) using methylamine. Yield 0.230g.
   m/e 696 (M+1⁺, 100%)
b) (2*R*)-2-Amino-3-[[5-[[(2,3-dichlorophenyl)sulfonyl]amino]-6-methoxypyrazinyl]thio]-*N*-methylpropanamide, 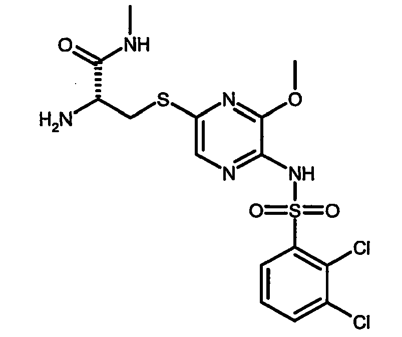 Procedure as for Example 1 step e) using [(1*R*)-1-[[[5-[[(2,3-dichlorophenyl)sulfonyl][[2-(trimethylsilyl)ethoxy]methyl]amino]-6-methoxypyrazinyl]thio]methyl]-2-(metllylamino)-2-oxoethyl]carbamic acid, 1,1-dimethylethyl ester (0.200g). Yield 0.060g.
   m/e 464 (M-1⁻, 100%)
   ¹H NMR (DMSO) δ 8.30 (1H, m), 8.05 (2h, bs), 7.96 (1H, dd), 7.63 (1H, dd), 7.36 (1H, t), 7.31 (1H, s), 3.80 (4H, m), 3.32 (3H, s), 3.25 (1H, m), 3.14 (1H, m).
   MP 160-4°C

### Example 6

### (2R)-2-Amino-3-[[5-[[(2,3-dichlorophenyl)sulfonyl]amino]-6-methoxypyrazinyl]thio]-N,N-dimethylpropanamide

a) [(1*R*)-1-[[[5-[[(2,3-Dichlorophenyl)sulfonyl][[2-(trimethylsilyl)ethoxy]methyl]amino]-6-methoxypyrazinyl]thio]methyl]-2-(dimethylamino)-2-oxoethyl]carbamic acid 1,1- 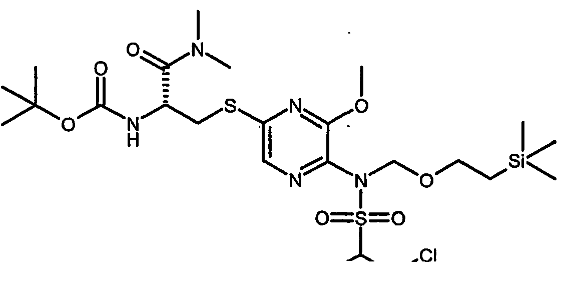 dimethylethyl ester.
   Procedure as for Example 4 step b) using dimethylamine. Yield 0.220g.
   m/e 710 (M-1⁻, 100%)
b) (2*R*)-2-Amino-3-[[5-[[(2,3-dichlorophenyl)sulfonyl]amino]-6-methoxypyrazinyl]thio]-*N,N*-dimethylpropanamide 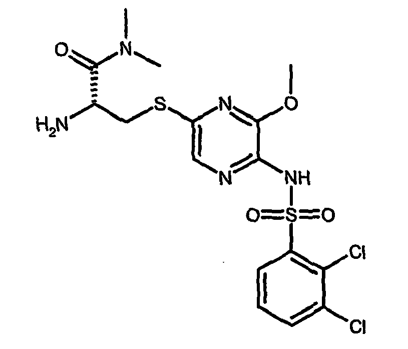 Procedure as for Example 1 step e) using [(1*R*)-1-[[[5-[[(2,3-dichlorophenyl)sulfonyl][[2-(trimethylsilyl)eihoxy]methyl]amino]-6-methoxypyrazinyl]thio]methyl]-2-(dimethylamino)-2-oxoethyl]carbamic acid, 1,1-dimethylethyl ester (0.200g). Yield 0.055g.
   m/e 480 (M+1⁺, 100%)
   ¹H NMR (DMSO) δ 8.05 (2h, bs), 7.95 (1H, d), 7.62 (1H, d), 7.35 (1H, t), 7.28 (1H, s), 4.43 (1H, t), 3.81 (3H, s), 3.26 (1H, m), 3.15 (1H, m), 2.89 (3H,s), 2.66 (3H, s). MP 187-90°C

### Example 7

### N-[5-[[(2R)-2-Amino-3-hydroxypropyl]thio]-3-methoxypyrazinyl]-2,3-dichloro benzenesulfonamide

a) [(1*R*)-2-[[5-[[(2,3-Dichlorophenyl)sulfonyl][[2-(trimethylsilyl)ethoxy]methyl]amino]-6-methoxypyrazinyl]thio]-1-(hydroxymethyl)ethyl]carbamic acid, 1,1-dimethylethyl ester 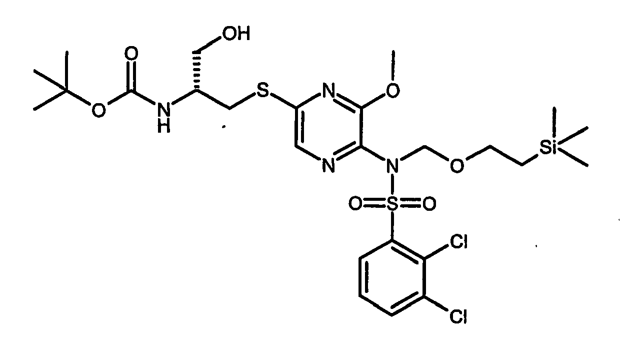 To a stirred solution of S-[5-[[(2,3-dichlorophenyl)sulfonyl][[2-(trimethylsilyl)ethoxy]methyl]amino]-6-methoxypyrazinyl]-*N*-[(1,1-dimethylethoxy)carbonyl]-L-cysteine, methyl ester (0.120g) in tetrahydrofuran (10mL) under nitrogen was added a 1.0M solution of lithium triethylborohydride in tetrahydrofuran (0.7mL). After stirring at room temperature for 1 day, the reaction mixture was poured into saturated ammonium chloride solution and extracted with ethyl acetate (x3). The combined extracts were washed with saturated brine, dried (MgSO₄) and the solvent evaporated. The residue was purified by silica gel chromatography, eluting with 2:3 ethyl acetate:isohexane, to afford the subtitled product as an oil (0.075g).
   m/e 669 (M+1⁺, 100%)
b) *N*-[5-[[(2*R*)-2-Amino-3-hydroxypropyl]thio]-3-methoxypyrazinyl]-2,3-dichlorobenzenesulfonamide 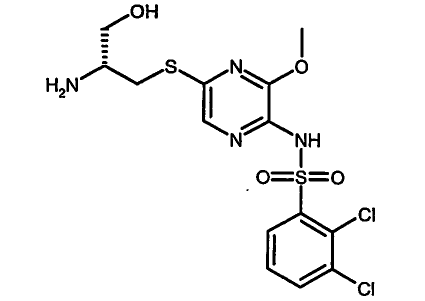 Procedure as for Example 1 step e) using [(1*R*)-2-[[5-[[(2,3-dichlorophenyl)sulfonyl][[2-(trimethylsilyl)ethoxy]methyl]amino]-6-methoxypyrazinyl]thio]-1-(hydroxymethyl)ethyl]carbamic acid, 1,1-dimethylethyl ester (0.075g).
   Yield 0.024g.
   m/e 437 (M-1⁻, 100%)
   ¹H NMR (DMSO) δ 8.05-7.93 (4H, m), 7.71 (1H, s), 7.58 (1H, t), 5.38 (1H, m), 3.94 (3H, s), 3.66-3.23 (5H, m).

### Example 8

### S-[3-Chloro-5-[[(2,3-dichlorophenyl)sulfonyl]amino]-6-methoxypyrazinyl]-L-cysteine, methyl ester

a) 3-Methoxy-5-bromo-6-chloro-2-pyrazinamine 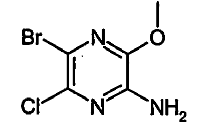 A stirred solution of 2-amino-6-chloropyrazine (2.0g) and *N*-bromosuccinimide (13.71g) in chloroform (100 mL) was heated to reflux for 20 hours. The reaction mixture was cooled and concentrated onto silica gel (20g) and the residue loaded onto a column of silica gel (5cm x 2cm) and the column was eluted with dichloromethane. Concentration afforded 3,5-dibromo-6-chloro-2-aminopyrazine that was dissolved into methanol (200 mL) and sodium methoxide (32g of a 25% solution in methanol) added. The reaction was heated to 70°C for 1.5h, cooled and concentrated to approx. 50 mL capacity. The reaction mixture was poured into water (200mL) and the sub-titled adduct (2.0g) collected as an off-white solid.
   m/e 235, 237 (M+1⁺, 100%)
b) *N*-(5-Bromo-6-chloro-3-methoxypyrazinyl)-2,3-dichlorobenzenesulphonamide 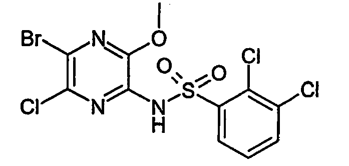 Procedure as for Example 1 step a), (reaction performed at room temperature) using 3-methoxy-5-bromo-6-chloro-2-pyrazinamine (0.5g) and 2,3-dichlorobenzenesulphonyl chloride (2.21g). Yield 3.2g.
   m/e 445, 447 (M-1⁺, 100%)
   ¹H NMR (CDCl₃) δ 8.32 (1H, dd), 7.79 (1H, br), 7.72 (1H, dd), 7.45 (1H, t), 4.05 (3H, s). MP 177-178°C
c) *N*-(5-bromo-6-chloro-3-methoxypyrazinyl)-2,3-dichloro-*N*-[[2-(trimethylsilyl)ethoxy]methyl]benzenesulfonamide 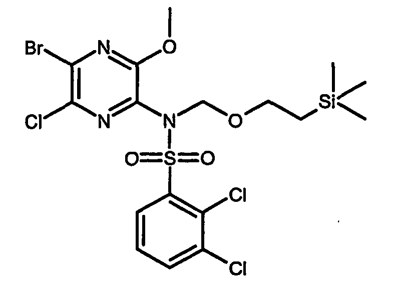 Procedure as for Example 1 step c) using *N*-(5-Bromo-6-chloro-3-methoxypyrazinyl)-2,3-dichlorobenzenesulphonamide (1.75g). Yield 2.20g.
   m/e 447 (M+1-SEM⁺, 100%)
   ¹H NMR (CDCl₃) δ 8.02 (1H, d), 7.71 (1H, d), 7.35 (1H, t), 5.23 (2H, s), 3.96 (3H, s), 3.72 (2H, t), 0.84 (2H, t), 0.00 (9H, s).
d) *S*-[3-Chloro-5-[[(2,3-dichlorophenyl)sulfonyl][[2-(trimethylsilyl)ethoxy]methyl]amino]-6-methoxypyrazinyl]-*N*-[(1,1-dimethylethoxy)carbonyl]-L-cysteine, methyl ester 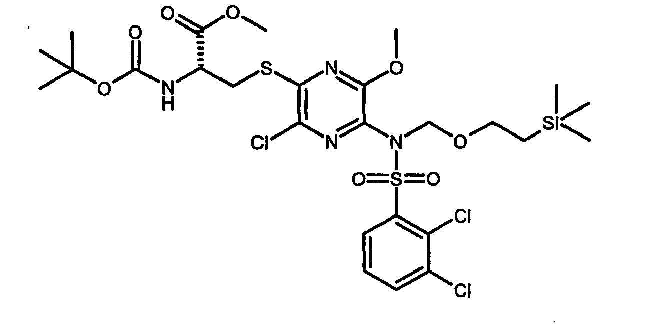 Procedure as for Example 2 step a) using *N*-(5-bromo-6-chloro-3-methoxypyrazinyl)-2,3-dichloro-*N*-[[2-(trimethylsilyl)ethoxy]methyl]benzenesulfonamide (0.400g). Yield 0.480g. m/e 631 ([M-Boc]+1⁺, 100%)
e) *S*-[3-Chloro-5-[[(2,3-dichlorophenyl)sulfonyl]amino]-6-methoxypyrazinyl]-L-cysteine, 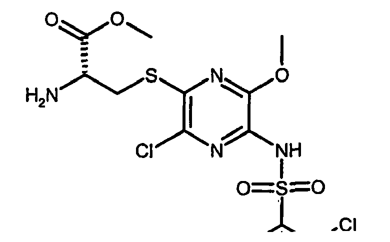 methyl ester
   Procedure as for Example 1 step e) using S-[3-chloro-5-[[(2,3-dichlorophenyl)sulfonyl][[2-(trimethylsilyl)ethoxy]methyl]amino]-6-methoxypyrazinyl]-*N*-[(1,1-dimethylethoxx)carbonyl]-L-cysteine, methyl ester (0.300g). Yield 0.120g. m/e 503 (M+1⁺, 100%)
   ¹H NMR (DMSO) δ 8.40 (2H, bs), 8.02 (1H, d), 7.65 (1H, d), 7.39 (1H, t), 4.30 (1H, m), 3.86 (3H, s), 3.66 (1H, m), 3.61 (3H, s), 3.34 (1H, m).
   MP 170-3°C

### Example 9

### S-[3-Chloro-5-[[(2,3-dichlorophenyl)sulfonyl]amino]-6-methozypyrazinyl]-L-cysteine

### Procedure as for Example 3 using S-[3-chloro-5-[[(2,3-dichlorophenyl)sulfonyl]amino]-6-

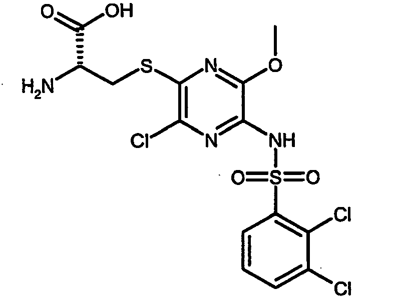

methoxypyrazinyl]-L-cysteine, methyl ester (0.070g). Yield 0.050g
m/e 488 (M+1⁺, 100%)
¹H NMR (DMSO) δ 8.51 (3H, bs), 8.03 (1H, dd), 7.70 (1H, d), 7.42 (1H, t), 4.16 (1H, m), 3.87 (3H, s), 3.74 (1H, dd), 3.30 (1H, m).
MP 170-3°C

### Example 10

### N-[5-[[(2R)-2-Amino-3-hydroxypropyl]thio]-6-chloro-3-methoxypyrazinyl]-2,3-dichlorobenzenesulfonamide

a) [(1*R*)-2-[[3-Chloro-5-[[(2,3-dichlorophenyl)sulfonyl][[2- 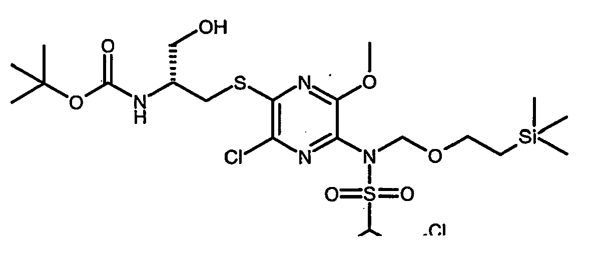 (trimethylsilyl)ethoxy]methyl]amino]-6-methoxypyrazinyl]thio]-1-(hydroxymethyl)ethyl]carbamic acid, 1,1-dimethylethyl ester Procedure as for Example 7 step a) using *S*-[3-chloro-5-[[(2,3-dichlorophenyl)sulfonyl][[2-(trimethylsilyl)ethoxy]methyl]amino]-6-methoxypyrazinyl]-*N*-[(1,1-dimethylethoxy)carbonyl]-L-cysteine, methyl ester (0.175g). Yield 0.170g m/e 603 (M+1-BOC⁺, 100%)
b) *N*-[5-[[(2*R*)-2-Amino-3-hydroxypropyl]thio]-6-chloro-3-methoxypyrazinyl]-2,3-dichlorobenzenesulfonamide 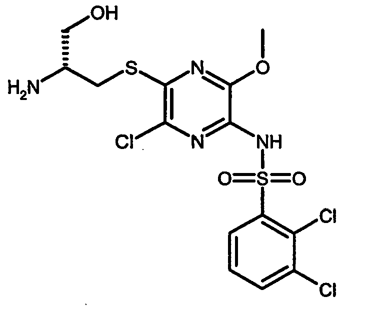 Procedure as for Example 1 step e) using [(1*R*)-2-[[3-chloro-5-[[(2,3-dichlorophenyl)sulfonyl][[2-(trimethylsilyl)ethoxy]methyl]amino]-6-methoxypyrazinyl]thio]-1-(hydroxymethyl)ethyl]carbamic acid, 1,1-dimethylethyl ester (0.170g).
   Yield 0.032g.
   m/e 475 (M+1⁺, 100%)
   ¹H NMR (DMSO) δ 8.01 (1H, dd), 7.91 (3H, bs), 7.63 (1H, t), 5.32 (1H, m), 3.82 (3H, s), 3.65 (1H, m), 3.57 (1H, m), 3.25-3.12 (2H, m).
   MP 255-6°C

### Example 11

### S-[5-[[(2,3-Dichlorophenyl)sulfonyl]amino]-6-methoxy-3-methylpyrazinyl]-L-cysteine, methyl ester

a) 6-Methyl-2-pyrazinamine 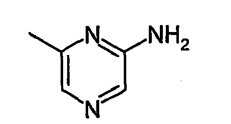 Dimethylzinc (100mL of a 2M solution in toluene) was added dropwise over 0.5h to a stirred solution of 6-chloro-2-pyrazinamine (12.9g) and [1,3-*bis*(diphenylphosphino)propane]nickel(II) chloride (5.4g) in dioxane (200mL) under a nitrogen atmosphere. The reaction mixture was heated at reflux for 18h, then cooled to room temperature and quenched cautiously with *iso*-propanol (30mL) and methanol (50mL). After removal of solvent *in vacuo,* the residue was partitioned between dichloromethane and aqueous ammonium chloride. The organic phase was filtered through celite, dried (MgSO₄), filtered and evaporated to give the crude product as an orange solid. Chromatography on silica gel eluting with ethyl acetate/methanol mixtures gave the sub-title compound (5.1g). Used directly.
b) 3,5-Dibromo-6-methyl-2-pyrazinamine 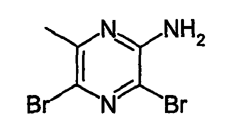 A solution of bromine (1.85g) in chloroform (5mL) was added dropwise to a stirred solution of 2-amino-6-methylpyrazine (0.6g) and pyridine (0.9 ml) in chloroform (50mL). The reaction mixture was stirred at room temperature for 0.5h, then washed twice with water, dried (MgSO₄), filtered and evaporated to give the crude product as an orange solid. Chromatography on silica gel eluting with dichloromethane gave the title compound (0.95g). Used directly.
c) 5-Bromo-3-methoxy-6-methyl-2-pyrazinamine 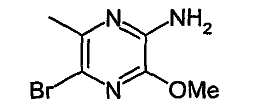 3,5-Dibromo-6-methyl-2-pyrazinamine (0.9g) was added to a solution of sodium (0.39g) in methanol (30mL) and the mixture heated at reflux for 18h. After removal of solvent in *vacuo,* the residue was partitioned between water and dichloromethane, and the organic phase dried (MgSO₄), filtered and evaporated to give the title compound as a pale yellow solid (0.58g).
   m/e 218/220 (M+1⁺, 100%)
   ¹H NMR (CDCl₃) δ 4.70 (2H, br s), 3.97 (3H, s), 2.40 (3H, s)
d) *N*-[5-Bromo-3-methoxy-6-methylpyrazinyl)-2,3-dichlorobenzenesulphonamide 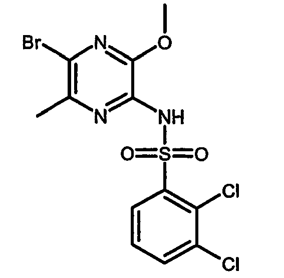 Sodium hydride (0.5g of a 60% dispersion in oil) was added to a solution of 5-bromo-3-methoxy-6-methyl-2-pyrazinamine (0.55g) in *N*-methylpyrrolidinone (25mL). The resultant dark solution was stirred at room temperature for 0.5h before a solution of 2,3-dichlorobenzenesulphonyl chloride (0.67g) in *N*-methylpyrrolidinone (5mL) was added dropwise. The reaction mixture was stirred at room temperature for 3h, then quenched with aqueous ammonium chloride and partitioned between ethyl acetate and aqueous ammonium chloride (x5). The organic phase was dried (MgSO₄), filtered and evaporated to give the crude product. Chromatography on silica gel eluting with dichloromethane/ acetic acid (200:1) gave the sub-title compound as a pale yellow solid (0.38g).
   m/e 424/426/428 (M-1⁻, 100%)
   ¹H NMR (CDCl₃) δ 8.29 (1H, d), 7.69 (2H, d), 7.41 (1H, t), 4.01 (3H, s), 2.27 (3H, s)
   MP 146-148°C
e) *N*-(5-Bromo-3-methoxy-6-methylpyrazinyl)-2,3-dichloro-*N*-[[2-(trimethylsilyl)ethoxy]methyl]-benzenesulfonamide 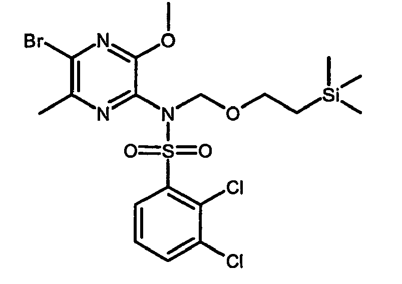 Procedure as for Example 1 step c) using *N*-[5-bromo-3-methoxy-6-methylpyrazinyl)-2,3-dichlorobenzenesulphonamide (7.0g).
   Yield 6.7g
   ¹H NMR (CDCl₃) δ 8.01 (1H, d), 7.68 (1H, d), 7.30 (1H, t), 5.24 (2H, s), 3.89 (3H, s), 3.74 (2H, m), 2.47 (3H, s), 0.84 (2H, m), 0.00 (9H, s).
f) *S*-[5-[[(2,3-Dichlorophenyl)sulfonyl][[2-(trimethylsilyl)ethoxy]methyl]amino]-6-methoxy-3-methylpyrazinyl]-*N*-[(1,1-dimethylethoxy)carbonyl]-L-cysteine, methyl ester 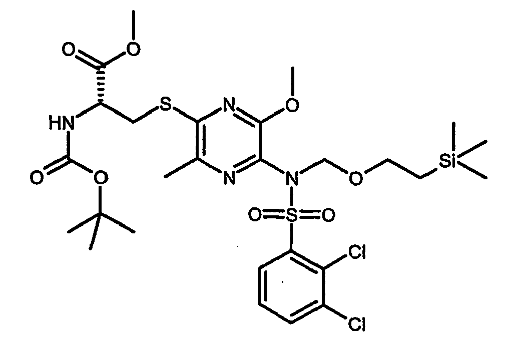 Procedure as for Example 2 step a) using the product of Example 13 step e) (0.1g) and N-[(1,1-dimethylethoxy)carbonyl]-L-cysteine, methyl ester (0.05g).
   Yield 0.045g.
   ¹H NMR (CDCl₃) δ 8.01 (1H, d), 7.66 (1H, d), 7.28 (1H, t), 5.40 (1H, m), 5.21 (2H, AB), 4.65 (1H, m) 3.91 (3H, s), 3.77 (2H, m), 3.70 (3H, s), 3.52 (1H, m), 2.29 (3H, s), 1.43 (9H, s), 0.85 (2H, m), 0.01 (9H, s).
g) *S*-[5-[[(2,3-Dichlorophenyl)sulfonyl]amino]-6-methoxy-3-methylpyrazinyl]-L-cysteine, methyl ester 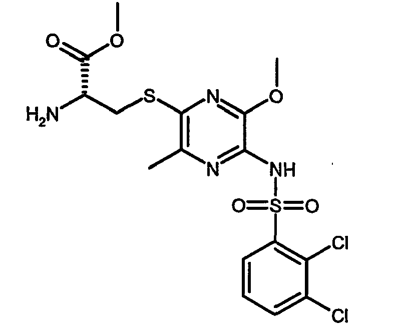 Procedure as for Example 1 step e) using *S*-[5-[[(2,3-dichlorophenyl)sulfonyl][[2-(trimethylsilyl)ethoxy]methyl]amino]-6-methoxy-3-methylpyrazinyl]-*N*-[(1,1-dimethylethoxy)carbonyl]-L-cysteine, methyl ester (0.045g).
   Yield 0.018g.
   m/e 481 (M+1⁺, 100%)
   ¹H NMR (D6-DMSO) δ 8.07 (1H, d), 7.70 (1H, d), 7.43 (1H, t), 4.06 (1H, t), 3.81 (3H, s), 3.51 (3H, s), 3.53-3.49 (1H, m), 3.34-3.29 (1H, m), 1.92 (3H, s).
   MP 164-168°C

### Example 12

### S-[5-[[(2,3-Dichlorophenyl)sulfonyl]amino]-6-methoary-3-methylpyrazinyl]-L-cysteine, oxalate salt

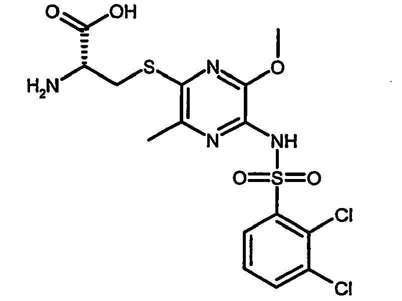

A solution of *S*-[5-[[(2,3-Dichlorophenyl)sulfonyl]amino]-6-methoxy-3-methylpyrazinyl]-L-cysteine, methyl ester (0.065g) and lithium hydroxide (0.011g) in tetrahydrofuran (2mL) and water (2mL) was heated at reflux for 4h. After removal of solvent *in vacuo,* the crude product was purified by reverse phase preparative hplc, followed by treatment with one equivalent of oxalic acid, to give the title compound as a pale brown solid.
Yield 0.021g.
m/e 467 (M+1⁺, 100%)
¹H NMR (D6-DMSO) δ 8.07 (1H, d), 7.66 (1H, d), 7.41 (1H, t), 3.82 (3H, s), 3.73 (1H, dd), 3.51 (1H, d), 3.02 (1H, m), 1.92 (3H, s).

### Example 13

### N-(2-Aminoethyl)-S-[5-[[(2,3-dichlorophenyl)sulfonyl]amino]-6-methoxypyrazinyl]-L-cysteine, ethyl ester

a) *S*-[5-[[(2,3-Dichlorophenyl)sulfonyl][[2-(trimethylsilyl)ethoxy]methyl]amino]-6-methoxypyrazinyl]-L-cysteine, ethyl ester 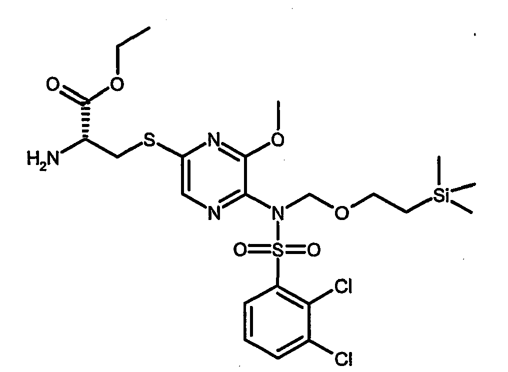 Procedure as for Example 1 step d) using 2,3-dichloro-*N*-(5-chloro-3-methoxypyrazinyl)-*N*-[[2-(trimethylsilyl)ethoxy]methyl]benzenesulfonamide (1.0g) and L-cysteinel ethyl ester hydrochloride (0.45g).
   Yield 0.9g.
   ¹H NMR (CDCl₃) δ 7.97 (1H, d), 7.89 (1H, s), 7.65 (1H, d), 7.27 (1H, t), 5.23 (2H, s), 4.18 (2H, q), 3.91 (3H, s), 3.80-3.71 (4H, m), 3.21-3.16 (1H, m), 1.26 (3H, t), 0.86 (2H, m), 0.01 (9H, s).
b) *N*-(2-Aminoethyl)-*S*-[5-[[(2,3-dichlorophenyl)sulfonyl]amino]-6-methoxypyrazinyl]-L-cysteine, ethyl ester 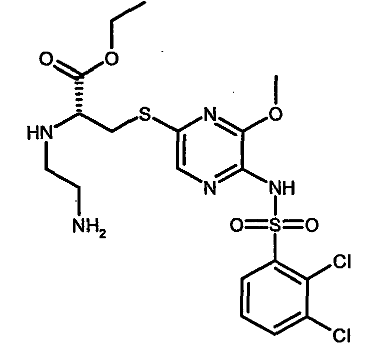 A solution of *S*-[5-[[(2,3-dichlorophenyl)sulfonyl][[2-(trimethylsilyl)ethoxy]methyl]amino]-6-methoxypyrazinyl]-L-cysteine, ethyl ester (0.35g), *tert*-butyl *N*-(2-oxoethyl)carbamate (0.27g) and sodium triacetoxyborohydride (0.36g) in acetonitrile (7mL) was stirred at room temperature for 30min before removal of solvent *in vacuo.* The residue was dissolved in a mixture of dichloromethane (5mL) and trifluoroacetic acid (2mL) and stirred at room temperature for 1h. After removal of solvent *in vacuo,* the crude product was purified by silica gel chromatography, eluting with 10:1 dichloromethane:methanol, to afford the title product as a pale brown solid.
   Yield 0.27g
   m/e 522 (M-1⁻, 100%)
   ¹H NMR (CDCl₃) δ 8.21 (1H, d), 7.67 (1H, d), 7.56 (1H, br s), 7.40 (1H, t), 4.12 (2H, q), 4.03 (3H, s), 3.85 (1H, m), 3.69 (1H, m), 3.35 (1H, m), 3.25 (4H, br s), 1.20 (3H, t).

### Example 14

### N-[5-[[(2R)-2-Amino-2-phenylethyl]oxy]-3-methoxypyrazinyl]-2,3-dichlorobenzenesulfonamide

a) *N*-[5-[[(2R)-2-Amino-2-phenylethyl]oxy]-3-methoxypyrazinyl]-2,3-dichloro-*N*-[[2-(trimethylsilyl)ethoxy]methyl]-benzenesulfonamide 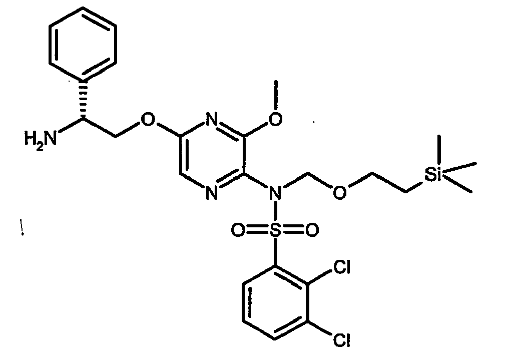 2,3-Dichloro-*N*-(5-chloro-3-methoxypyrazinyl)-*N*-[[2-(trimethylsilyl)ethoxy]methyl]-benzenesulfonamide (0.25g) and R-(-)-2-phenylglycinol (0.075g) were dissolved in dry DMF (10 ml) under an atmosphere of nitrogen and treated with sodium hydride (60% suspension in oil, 0.035g). After stirring at ambient temperature for 20 hr the reaction was poured into brine and extracted with ethyl acetate (3 x 50 ml). The organic extracts were washed with brine, dried (MgSO₄) and evaporated to give the sub-title product as an oil. Yield 0.2g.
   m/e 599 (M⁺, 100%)
b) *N*-[5-[[(2R)-2-Amino-2-phenylethyl]oxy]-3-methoxypyrazinyl]-2,3-dichlorobenzenesulfonamide 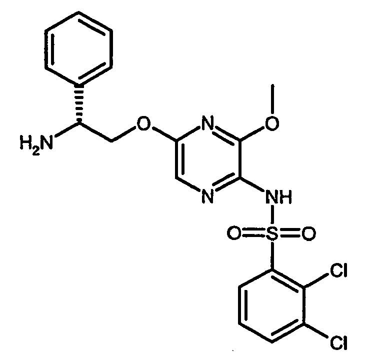 The product from Example 14 step a) (0.2 g) was subjected to the procedure described for Example 1 step e) to afford the title product which crystallised from diethyl ether/hexane. Yield 0.032g
   MP 138-140 °C
   m/e 467 (M-1⁻, 100%)
   ¹H NMR (DMSO-d6) δ 7.92 (1H, dd), 7.64 (1H, dd), 7.32-7.53 (6H, m), 7.08 (1H, s), 4.63 (1H, m), 4.25-4.40 (2H, m), 3.76 (3H, s).

### Example 15

### 2,3-Dichloro-N-[5-[[2-hydroxy-2-(2-thiazolyl)ethyl]thio]-3-methoxypyrazinyl]-benzenesulfonamide

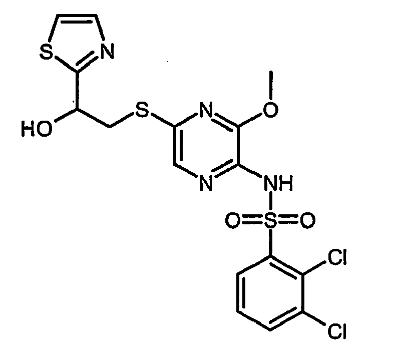

a) 2,3-Dichloro-*N*-[3-methoxy-5-[[2-(4-pyridinyl)ethyl]thio]pyrazinyl]-*N*-[[2-(trimethylsilyl)ethoxy]methyl]-benzenesulfonamide 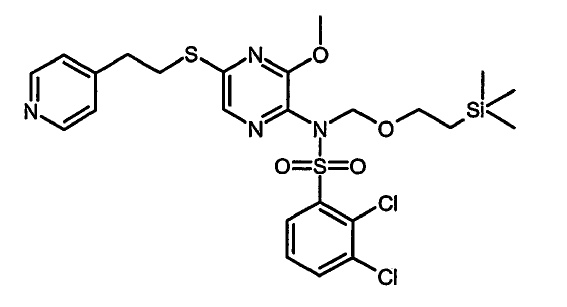 2,3-Dichloro-N-(5-chloro-3-methoxypyrazinyl)-N-[[2-(trimethylsilyl)ethoxy]methyl]-benzenesulfonamide (10 g) and 4-pyridineethanthiol hydrochloride (4 g) were dissolved in acetonitrile (200 ml), treated with caesium carbonate (16 g) and stirred at ambient temperature for 24 hr. The mixture was patitioned between ethyl acetate ( 200 ml) and 2M HCl (200 ml). The ethyl acetate extract was washed with 2 M HCl (100 ml), water (100 ml), brine (100 ml), dried (MgSO4) and evaporated to give the sub-title compound as a solid (9.1 g).
   ¹H NMR (CDCl₃) δ 8.55 (2H, d), 7.99 (1H, m), 7.87 (1H, s), 7.67 (1H, m), 7.28 (1H, m), 7.15 (2H, d), 5.23 (2H, s), 3.90 (3H, s), 3.79 (2H, t), 3.40 (2H, t), 3.03 (2H, t),0.87 (2H, t), 0.00 (9H, s).
b) 2-[[5-[[(2,3-Dichlorophenyl)sulfonyl][[2-(trimethylsilyl)ethoxy]methyl]amino]-6-methoxypyrazinyl]thio]-*N*-methoxy-*N*-methyl-acetamide. 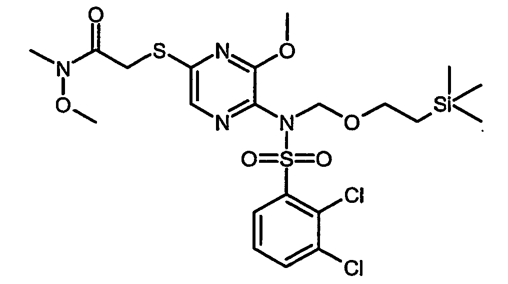 The product from Example 15 step a) (4 g) was dissolved in dry THF (25 ml) under an atmosphere of nitrogen and treated with a 1 M solution of potassium *tert*-butoxide in THF (10 ml), added dropwise. After stirring for 30 min the reaction was treated with a saturated solution of ammonium chloride ( 20 ml) followed by 2M HCl (100 ml) and extracted into ethyl acetate ( 200 ml). The ethyl acetate extract was washed with water ( 2 x100 ml), brine (100 ml), dried (MgSO4) and evaporated to leave a gum (3.5 g). This was dissolved in dry THF (25 ml) and treated with a 1 M solution of potassium *tert*-butoxide in THF (5 ml) and 2-chloro-*N*-methoxy-*N*-methyl-acetamide (1 g) added as a solution in THF (5 ml). The mixture was stirred at ambient temperature for 1 hr and partitioned between ethyl acetate (200 ml) and 2 M HCl (100 ml). The ethyl acetate extract was washed with water ( 2 x100 ml), brine (100 ml), dried (MgSO4) and evaporated to give the sub-titled compound as a yellow gum (3.32 g).
   ¹H NMR (CDCl₃) δ 7.98 (1H, dd), 7.94 (1H, s), 7.66 (1H, dd), 7.24 (1H, m), 5.21 (2H, s), 4.14 (2H, s), 3.87 (3H, s), 3.79 (2H, t), 3.76 (3H, s), 3.22 (3H, s),0.87 (2H, t), 0.00 (9H, s).
c) 2,3-Dichloro-*N*-[3-methoxy-5-[[2-oxo-2-(2-thiazolyl)ethyl]thio]pyrazinyl]-*N*-[[2-(trimethylsilyl)ethoxy]methyl]-benzenesulfonamide 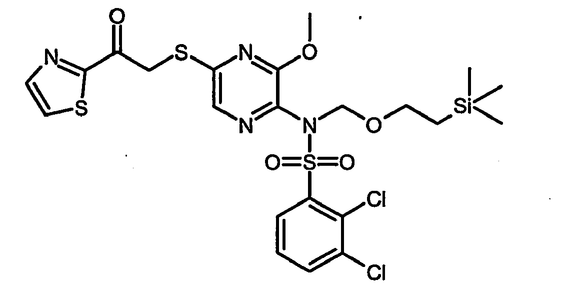 Thiazole (0.17 g) was dissolved in dry THF (10 ml), cooled to -30°C and stirred under an atmosphere of nitrogen. Butyl lithium (1.5 M, 1.5 ml) was added dropwise and the reaction was stirred for 30 mins and became a dark-orange colour. The product of Example 15 step b) (0.3 g) in THF (3 ml) was added dropwise and the reaction mixture was stirred between -20 to -10°C for 2.5 hrs. The mixture was then poured into 2 M HCl ( 25 ml) and extracted into ethyl aceatte (50 ml). The ethyl acetate extract was washed with water ( 2 x 20 ml), brine (20 ml), dried (MgSO₄) and evaporated to give a gum which was purified by silica gel chromatography, using hexane:ethyl acetate (2:1) as eluant to give the sub-titled compound as a yellow gum (0.1 g).
   m/e 622 (M+1)⁺
   ¹H NMR (CDCl₃) δ 8.06 (1H, d), 7.96 (1H, s), 7.88 (1H, dd), 7.76 (1H, d), 7.65 (1H, dd) 7.25 (1H, m), 5.21 (2H, s), 4.75 (2H, s), 3.75 (2H, t), 3.53 (3H, s), 0.85 (2H, t), 0.01 (9H, s).
d) 2,3-Dichloro-*N*-[5-[[2-hydroxy-2-(2-thiazolyl)ethyl]thio]-3-methoxypyrazinyl]-benzenesulfonamide 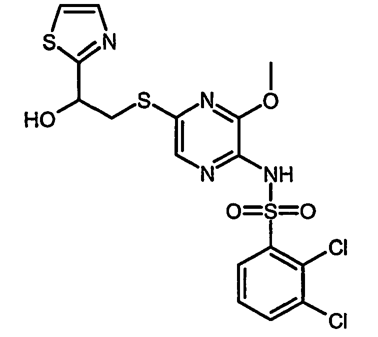 The product of Example 15 step c) was dissolved in acetonitrile (3 ml) and treated with sodium triacetoxyborohydride and stirred at ambient temperature for 48 hr. The reaction mixture was treated with TFA (3 mls) for 30 mins and them partitioned between ethyl acetate (20 ml) and 2 M HCl (10 ml). The ethyl acetate extract was washed with water ( 2 x 20 ml), brine (20 ml), dried (MgSO₄) and evaporated to give a gum which was purified by silica gel chromatography, using hexane:ethyl acetate (1:1) as eluant to give the subtitled compound as a yellow solid (0.03 g).
   m/e 4911493/495 (M-1)⁻
   ¹H NMR (CDCl₃) δ 8.28 (1H, d), 7.6-7.7 (2H, m), 7.39 (1H, d), 7.76 (1H, d), 7.28 (1H, m), 4.25 (1H, m), 4.1 (3H, s), 3.99 (1H, d), 3.7-3.75 (1H, m), 3.41-3.49 (1H, m).

### Example 16

### 2,3-Dichloro-N-[5-[[2-hydrozy-2-(1-methyl-1H-imidazol-2-yl)ethyl]thio]-3-methoxypyrazinyl]-benzenesulfonamide, potassium salt

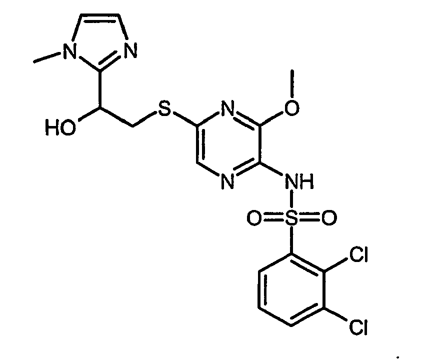

a) 2,3-Dichloro-*N*-[3-methoxy-5-[[(trimethylsilyl)methyl]thio]pyrazinyl]-*N*-[[2-(trimethylsilyl)ethoxy]methyl]-benzenesulfonamide 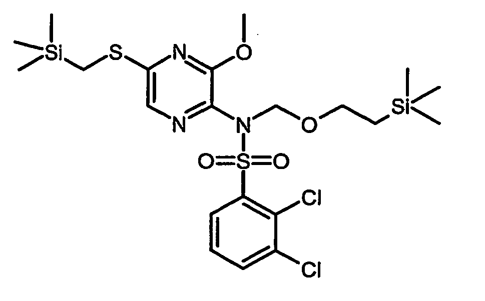 A solution of 2,3-dichloro-*N*-(5-chloro-3-methoxypyrazinyl)-*N*-[[2-(trimethylsilyl)ethoxy]methyl]benzenesulfonamide (3.2g), trimethylsilylmethane thiol (1.0ml) and cesium carbonate (4.2g) in acetonitrile (50ml) was stirred under an atmosphere of nitrogen at room temperature for two hours. The reaction mixture was filtered and evaporated, and the residue purified by silica gel chromatography, using isohexane:ethyl acetate (10:1) as eluant, to give the sub-titled compound as a colourless oil (2.8g).
   ¹H NMR (CDCl₃) δ 7.95 (1H, d), 7.89 (1H, s), 7.64 (1H, d), 7.25 (1H, t), 5.24 (2H, s), 3.86 (3H, s), 3.82-3.76 (2H, m), 2.31 (2H, s), 0.89-0.84 (2H, m), 0.15 (9H, s), 0.01 (9H, s).
b) 2,3-Dichloro-*N*-[5-[[2-hydroxy-2-(1-methyl-1*H*-imidazol-2-yl)ethyl]thio]-3-methoxypyrazinyl]-*N*-[[2-(trimethylsilyl)ethoxy]methyl]-benzenesulfonamide 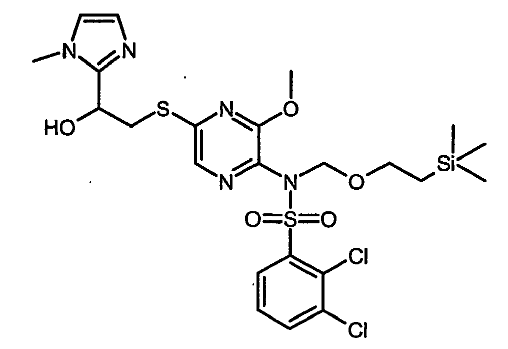 To a solution of 2,3-dichloro-*N*-[3-methoxy-5-[[(trimethylsilyl)methyl]thio]pyrazinyl]-*N-*[[2-(trimethylsilyl)ethoxy]methyl]-benzenesulfonamide (0.5g) and 1-methyl-2-imidazolecarboxaldehyde (94mg) in tetrahydrofuran (2ml) was added tetrabutylammonium fluoride (0.1ml of a 1M solution in THF), and the mixture stirred at room temperature under an atmosphere of nitrogen for 5 minutes. The solvent was *removed in vacuo* and the residue purified by silica gel chromatography, eluting with 1:1 ethyl acetate:dichloromethane and ethyl acetate, to afford the sub-titled product as a colourless
   oil (0.12g).
   ¹H NMR (CDCl₃) δ 7.97 (1H, d), 7.88 (1H, s), 7.65 (1H, d), 7.27 (1H, t), 6.96 (1H, s), 6.81 (1H, s), 5.22 (2H, s), 4.98 (1H, t), 3.88 (3H, s), 3.84-3.74 (5H, m), 3.68 (3H, s), 0.88-0.84 (2H, m), 0.00 (9H, s).
c) 2,3-Dichloro-*N*-[5-[[2-hydroxy-2-(1-methyl-1*H*-imidazol-2-yl)ethyl]thio]-3-methoxypyrazinyl]-benzenesulfonamide, potassium salt 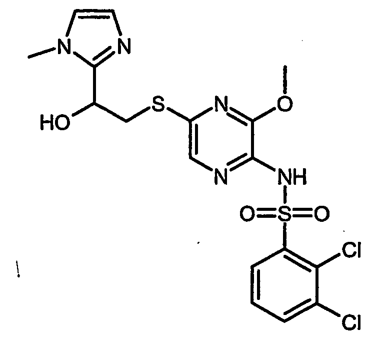 A solution of the product from step b) (75mg) in dichloromethane (4ml) and trifluoroacetic acid (2ml) was stirred at room temperature for 30 minutes. The solvent was removed *in vacuo* and the residue purified by reverse phase preparative hplc, followed by treatment with one equivalent of potassium hydroxide, to give the title compound as a white solid. Yield 0.031g.
   m/e 490/492 (M+1)⁺
   ¹H NMR (DMSO-d6) δ 7.93 (1H, d), 7.60 (1H, d), 7.34 (1H, t), 7.19 (1H, s), 7.01 (1H, s), 6.74 (1H, s), 4.75 (1H, t), 3.79 (3H, s), 3.59 (3H, s), 3.48-3.42 (1H, m), 3.31-3.24 (1H, m).

### Example 17

### 2,3-Dichloro-N-[5-[[2-hydroxy-2-(2-oxazolyl)ethyl]thio]-3-methoxypyrazinyl]-benzenesulfonamide

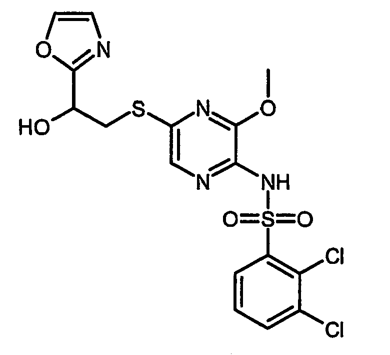

a) 2,3-Dichloro-*N*-[5-[[2-hydroxy-2-(2-oxazolyl)ethyl]thio]-3-methoxypyrazinyl]-*N*-[[2-(trimethylsilyl)ethoxy]methyl]-benzenesulfonamide 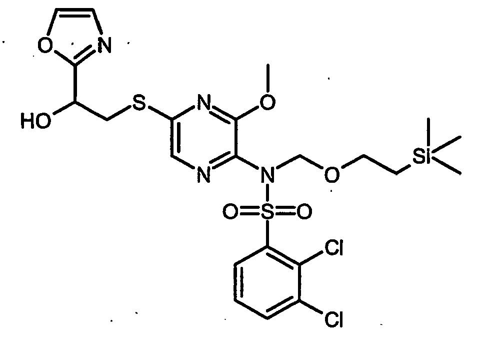 Using the procedure of Example 16 step (b) 2,3-dichloro-*N*-[3-methoxy-5-[[(trimethylsilyl)methyl]thio]pyrazinyl]-*N*-[[2-(trimethylsilyl)ethoxy]methyl]-benzenesulfonamide (0.7g) was reacted with oxazole-2-carboxaldehyde (0. 14g) in the presence of tetrabutylammonium fluoride (0.6ml of a 1M solution in THF) in tetrahydrofuran (3ml).
   Yield 0.080g
   ¹H NMR (CDCl₃) δ 7.97 (1H, d), 7.91 (1H, s), 7.65 (1H, d), 7.61 (1H, s), 7.28 (1H, t), 7.08 (1H, s), 5.22 (2H, s), 5.14-5.09 (1H, m), 3.91 (3H, s), 3.80-3.64 (3H, m), 3.66-3.59 (1H, m), 3.55 (1H, br d), 0.88-0.83 (2H, m), 0.01 (9H, s).
b) 2,3-Dichloro-*N*-[5-[[2-hydroxy-2-(2-oxazolyl)ethyl]thio]-3-methoxypyrazinyl]-benzenesulfonamide 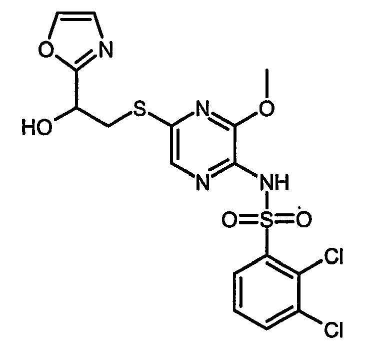 Using the procedure of Example 16 step (c) the title compound was obtained by treating 2,3-dichloro-*N*-[5-[[2-hydroxy-2-(2-oxazolyl)ethyl]thio]-3-methoxypyrazinyl]-*N*-[[2-(trimethylsilyl)ethoxy]methyl]-benzenesulfonamide (80mg) with trifluoroacetic acid (2ml) in dichloromethane (2ml).
   Yield 0.056g
   m/e 475/477 (M-1)⁻
   ¹H NMR (DMSO-d6) 7.97 (1H, s), 7.93 (1H, d), 7.60 (1H, d), 7.34 (1H, t), 7.14 (1H, s), 7.10 (1H, s), 4.75 (1H, t), 3.79 (3H, s), 3.35-3.21 (2H, m).

### Example 18

### N-[5-[[2-Amino-2-(2-oxazolyl)ethyl]thio]-3-methozypyrazinyl]-2,3-dichlorobenzenesulfonamide

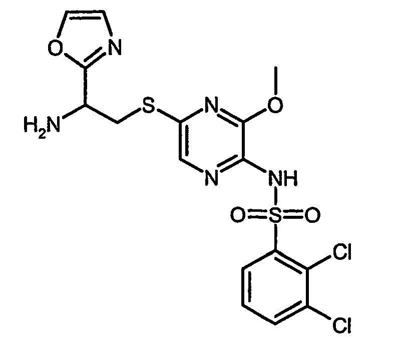

a) 2,3-Dichloro*-N*-[5-[[2-chloro-2-(2-oxazolyl)ethyl]thio]-3-methoxypyrazinyl]-*N*-[[2-(trimethylsilyl)ethoxy]methyl]-benzenesulfonamide 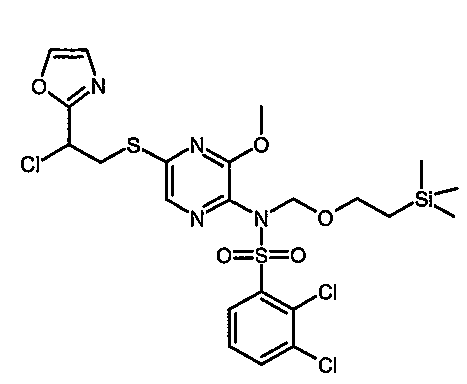 To a solution of 2,3-dichloro-*N*-[5-[[2-hydroxy-2-(2-oxazolyl)ethyl]thio]-3-methoxypyrazinyl]-*N*-[[2-(trimethylsilyl)ethoxy]methyl]-benzenesulfonamide (0.35g) in dichloromethane (10ml) was added methanesulphonyl chloride (0.45ml) and triethylamine (1.2ml). The reaction was stirred at room temperature for two hours then diluted with ethyl acetate, washed with brine, dried over magnesium sulphate, filtered and evaported to give the sub-titled compound as a yellow oil (0.41g).
   m/e 627/629 (M+1)⁺
b) *N*-[5-[[2-Azido-2-(2-oxazolyl)ethyl]thio]-3-methoxypyrazinyl]-2,3-dichloro-*N*-[[2-(trimethylsilyl)ethoxy]methyl]-benzenesulfonamide 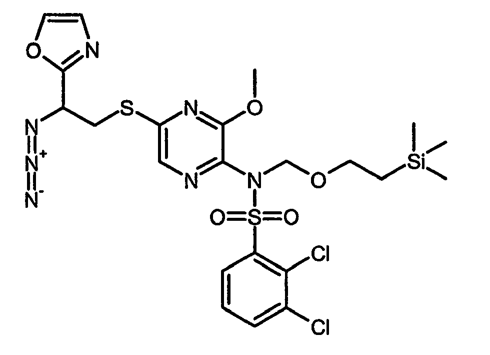 A solution of 2,3-dichloro-*N*-[5-[[2-chloro-2-(2-oxazolyl)ethyl]thio]-3-methoxypyrazinyl]-*N*-[[2-(trimethylsilyl)ethoxy]methyl]-benzenesulfonamide (0.41g) and sodium azide (0.11g) in dimethylformamide (10ml) was heated at 65°C for two hours. The cooled reaction mixture was diluted with diethyl ether and washed once with brine and three times with saturated aqueous ammonium chloride. The organic phase was dried over magnesium sulphate, filtered and evaporated to give the sub-titled compound as a yellow oil (0.24g). m/e 502/504 (M-130+1)⁺ (loss of [(trimethylsilyl)ethoxy]methyl)
c) *N*-[5-[[2-Azido-2-(2-oxazolyl)ethyl]thio]-3-methoxypyrazinyl]-2,3-dichlorobenzenesulfonamide 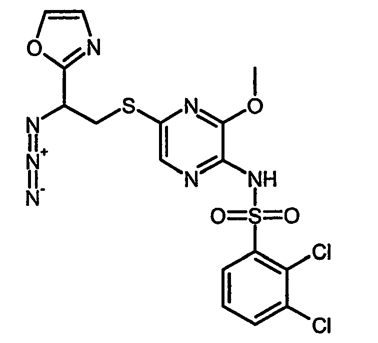 A solution of *N*-[5-[[2-azido-2-(2-oxazolyl)ethyl]thio]-3-methoxypyrazinyl]-2,3-dichloro-*N*-[[2-(trimethylsilyl)ethoxy]methyl]-benzenesulfonamide (0.24g) in dichloromethane (10ml) and trifluoroacetic acid (3ml) was stirred at room temperature for one hour. The solvent was removed *in vacuo* and the residue purified by silica gel chromatography, eluting with 1:1 ethyl acetate:isohexane/0.5% acetic acid to give the sub-titled compound as a colourless oil (0.16g).
   m/e 500/502 (M-1)⁻
d) *N*-[5-[[2-Amino-2-(2-oxazolyl)ethyl]thio]-3-methoxypyrazinyl]-2,3-dichlorobenzenesulfonamide 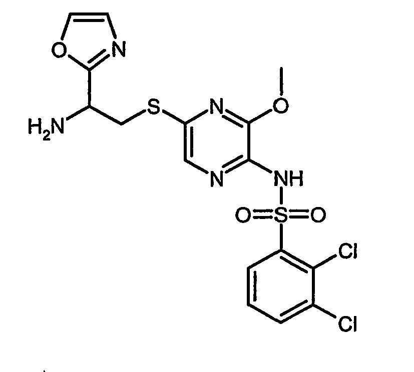 A solution of *N*-[5-[[2-azido-2-(2-oxazolyl)ethyl]thio]-3-methoxypyrazinyl]-2,3-dichlorobenzenesulfonamide (0.16g) and triphenylphosphine (0.16g) in tetrahydrofuran (10ml) and water (2ml) was stirred at room temperature for 20 hours. Methanol (10ml) and 10% aqueous sodium hydroxide (2ml) were added and the reaction mixture stirred at room temperature a further 24 hours. The reaction mixture was concentrated, acidifed with 2M aqueous hydrochloric acid and extracted with dichloromethane (5x). The combined organic phases were dried over magnesium sulphate, filtered and evaporated to give a yellow solid which was purified by reverse phase preparative hplc to give the title compound as a white solid.
   Yield 0.08g
   m.p. 168-170°C
   m/e 476/478 (M+1)⁺
   ¹H NMR (DMSO-d6) 8.12 (1H, s), 7.99 (1H, d), 7.68 (1H, d), 7.40 (1H, t), 7.26 (1H, s), 7.24 (1H, s), 4.59 (1H, m), 3.75 (3H, s), 3.57-3.52 (1H, m), 3.45-3.39 (1H, m).

### Example 19

### 2,3-Dichloro-N-[5-[(2,3-dihydrozypropyl)thio]-3-methozypyrazinyl] benzenesulfonamide

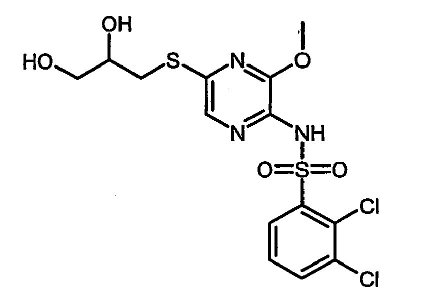

a) 2,3-Dichloro-*N*-[5-[(2,3-dihydroxypropyl)thio]-3-methoxypyrazinyl]-*N*-[[2-(trimethylsilyl)ethoxy]methyl]benzenesulfonamide. 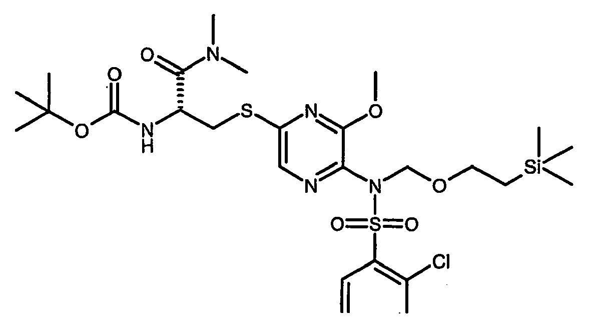 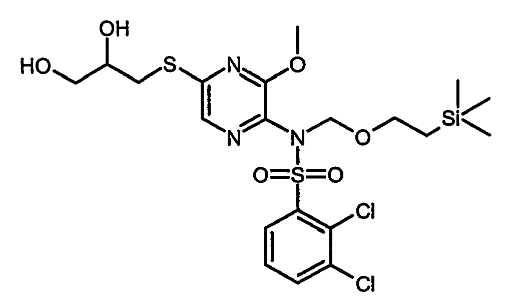 Procedure as for Example 1 step d) using 3-mercaptopropane-1,2-diol. Yield 0.350g.
   m/e 570 (M+1⁺)
b) 2,3-Dichloro-*N*-[5-[(2,3-dihydroxypropyl)thio]-3-methoxypyrazinyl] benzenesulfonamide. 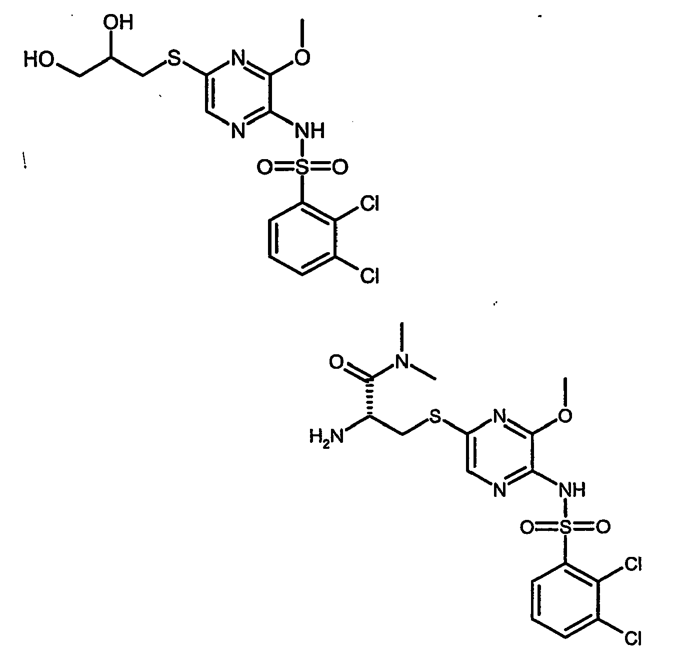 Procedure as for Example 1 step e) using 2,3-dichloro-*N*-[5-[(2,3-dihydroxypropyl)thio]-3-methoxypyrazinyl]-*N*-[[2-(trimethylsilyl)ethoxy]methyl]benzenesulfonamide.
   Yield 0.018g.
   m/e 440 (M+1⁺, 100%)
   ¹H NMR (DMSO) δ 11.27 (1H, bs), 8.02 (1H, d), 7.93 (1H, d), 7.67 (1H, s), 7.57 (1H, t), 3.89 (3H, s), 3.62 (1H, m), 3.40 (3H, m), 2.98 (1H, m).
   MP 136-8°C

### Example 20

### 2,3-Dichloro-N-[5-[(2-hydroxy-2-phenylethyl)thio]-3-methoxypyrazinyl] benzenesulfonamide

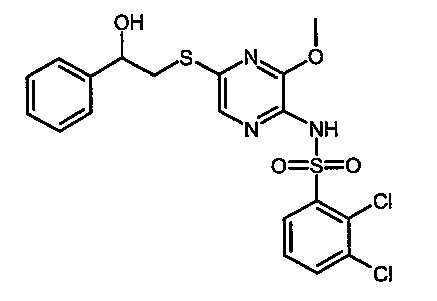

a) 2,3-Dichloro-*N*-[3-methoxy-5-[(2-oxo-2-phenylethyl)thio]pyrazinyl]-*N*-[[2-(trimethylsilyl)ethoxy]methyl]benzenesulfonamide. 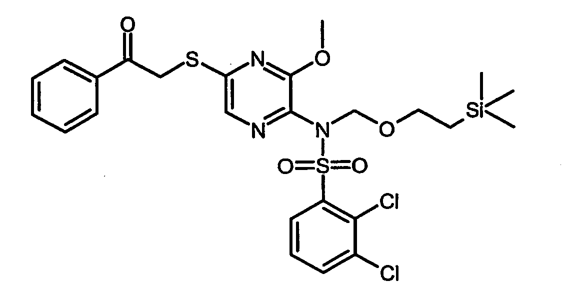 The product from Example 15 step a) (1.0 g) was dissolved in dry THF (20 ml) under an atmosphere of nitrogen and treated with a 1 M solution of potassium *tert*-butoxide in THF (2.5 ml), added dropwise. After stirring for 60 min the reaction was treated with phenacyl bromide (0.37 g) and allowed to stir for 20 hrs. The reaction mixture was poured into saturated aqueous ammonium chloride solution and extracted into ethyl acetate (x3). The combined ethyl acetate extracts were washed with water, brine and dried (MgSO4) and evaporated to leave an oil. The residue was purified by silica gel chromatography, eluting with 1:4 ethyl acetate:isohexane, to afford the sub-titled product as a colourless oil (0.87g). m/e 614 (M+1⁺, 100%)
   ¹H NMR (CDCl₃) δ 8.02 (2H, m), 7.95 (2H, m), 7.63 (2H, m), 7.52 (2H, m), 7.25 (1H, m), 5.20 (2H, s), 4.59 (2H, s), 3.76 (2H, m), 3.56 (3H, s),0.85 (2H, t), 0.00 (9H, s).
b) 2,3-Dichloro*-N*-[5-[(2-hydroxy-2-phenylethyl)thio]-3-methoxypyrazinyl]-*N-*[[2-(trimethylsilyl)ethoxy]methyl]benzenesulfonamide. 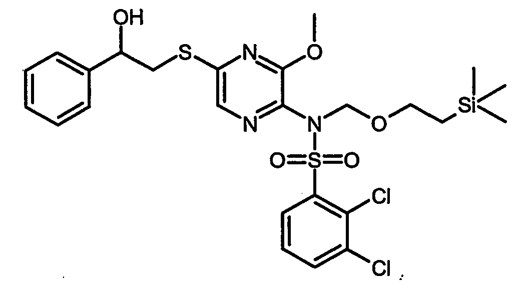 The product from Example 20 step a) (0.33 g) was dissolved in EtOH (5 ml) under an atmosphere of nitrogen and treated with sodium borohydride (0.042 g). The reaction was stirred for 24 hrs then poured into water and extracted into ethyl acetate (x3). The combined ethyl acetate extracts were washed with brine, dried (MgSO4) and evaporated to afford the sub-titled product as a colourless oil (0.30g).
   m/e 616 (M+1)⁺
c) 2,3-Dichloro-*N*-[5-[(2-hydroxy-2-phenylethyl)thio]-3-methoxypyrazinyl] benzenesulfonamide. 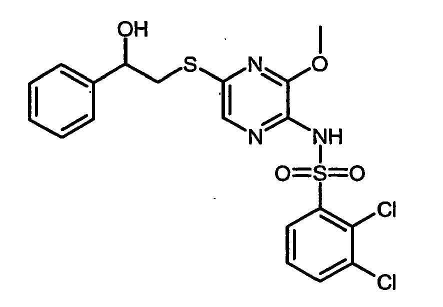 Procedure as for Example 1 step e) using the product from Example 20 step b) (0.30 g). Yield 0.022g.
   m/e 484 (M-1)⁻
   ¹H NMR (DMSO) δ 8.02 (1H, dd), 7.94 (1H, dd), 7.58 (2H, m), 7.35 (2H, d), 7.17-7.29 (3H, m), 5.64 (1H, s), 4.75 (1H, t), 3.89 (3H, s), 3.27-3.46 (2H, m).
   MP 141-3°C

### Example 21

### 2,3-Dichloro-N-[5-[[2-hydrozy-2-(3-pyridinyl)ethyl]thio]-3-methozypyrazinyl] benzenesulfonamide

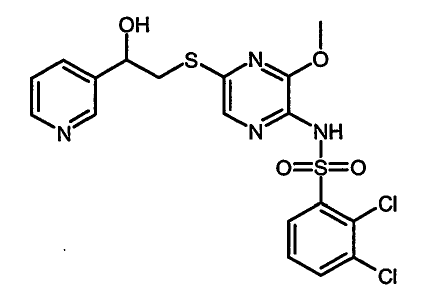

a) 2,3-Dichloro-*N*-[3-methoxy-5-[[2-oxo-2-(3-pyridinyl)ethyl]thio]pyrazinyl]-*N*-[[2-(trimethylsilyl)ethoxy]methyl]benzenesulfonamide 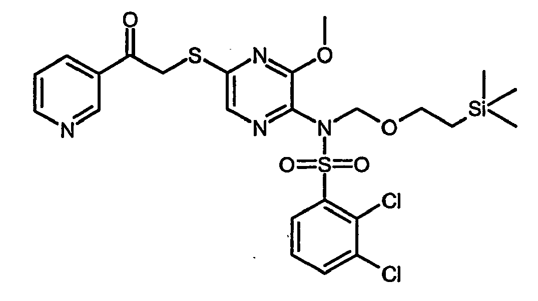 Procedure as for Example 20 step a) using 3-(bromoacetyl)pyridine hydrobromide (0.70 g).
   Yield 1.55g
   m/e 615 (M+1⁺)
b) 2,3-Dichloro-*N*-[3-methoxy-5-[[2-oxo-2-(3-pyridinyl)ethyl]thio]pyrazinyl]benzenesulfonamide 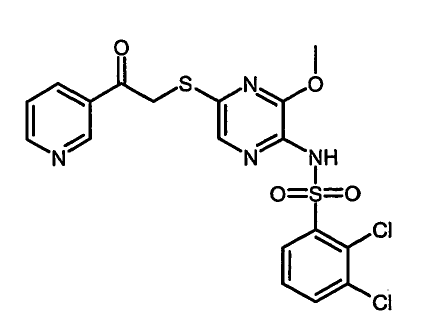 Procedure as for Example 1 step e) using the product from Example 21 step a) (0.16 g). Yield 0.122g.
   m/e 483 (M-1)⁻
   ¹H NMR (DMSO) δ 11.32 (1H, bs), 9.22 (1H, m), 8.81 (1H, m), 8.37 (1H, m), 8.02 (1H, dd), 7.93 (1H, dd), 7.74 (1H, s), 7.58 (2H, m), 4.84 (2H, s), 3.64 (3H, s).
   MP 202-3°C
c) 2,3-Dichloro-*N*-[5-[[2-hydroxy-2-(3-pyridinyl)ethyl]thio]-3-methoxypyrazinyl]benzenesulfonamide. 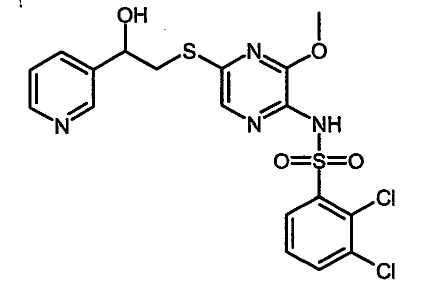 Procedure as for Example 20 step b) using the product from Example 21 step b) (0.10 g). Yield 0.013g.
   m/e 486 (M-1)⁻
   ¹H NMR (DMSO) δ 8.54 (1H, s), 8.39 (1H, d), 8.02 (1H, d), 7.92 (1H, d), 7.74 (1H, d), 7.57 (2H, m), 7.26 (1H, m), 5.81 (1H, d), 4.83 (1H, m), 3.89 (3H, s), 3.42 (2H, m).
   MP 188-90°C

### Example 22

### N-[5-[[2-Amino-2-(3-pyridinyl)ethyl]thio]-3-methoxypyrazinyl]-2,3-dichloro benzenesulfonamide

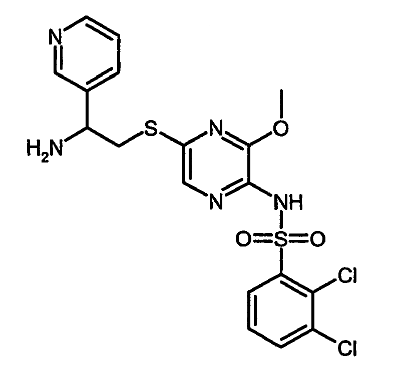

a) 2,3-Dichloro-*N*-[5-[[2-hydroxy-2-(3-pyridinyl)ethyl]thio]-3-methoxypyrazinyl]-*N*-[[2-(trimethylsilyl)ethoxy]methyl]benzenesulfonamide. 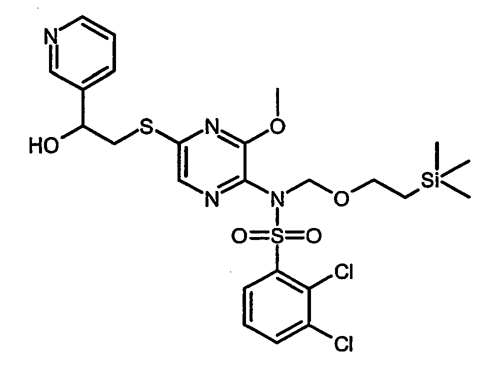 Procedure as for Example 20 step b) using the product from Example 21 step a) (1.55 g). Yield 0.92 g
   m/e 617 (M+1⁺)
b) 2,3-Dichloro-*N*-[5-[[2-chloro-2-(3-pyridinyl)ethyl]thio]-3-methoxypyrazinyl]-*N*-[[2-(trimethylsilyl)ethoxy]methyl]benzenesulfonamide 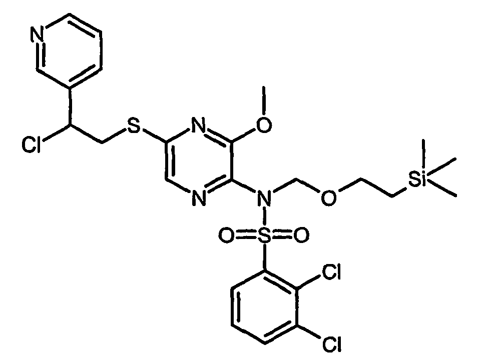 Procedure as for Example 18 step a) using the product from Example 22 step a) (0.74 g). Yield 0.32 g
   m/e 635 (M+1⁺)
   ¹H NMR (CDCl₃) δ 8.58 (2H, m), 7.98 (1H, d), 7.83 (1H, s), 7.75 (1H, d), 7.66 (1H, d), 7.32 (2H, m), 5.22 (2H, s), 5.10 (1H, m), 4.03 (1H, m), 3.98 (3H, m), 3.78 (2H, m), 3.64 (1H, m), 0.86 (2H, m), 0.00 (9H, s).
c) *N*-[5-[[2-Azido-2-(3-pyridinyl)ethyl]thio]-3-methoxypyrazinyl]-2,3-dichloro-*N*-[[2-(trimethylsilyl)ethoxy]methyl]benzenesulfonamide 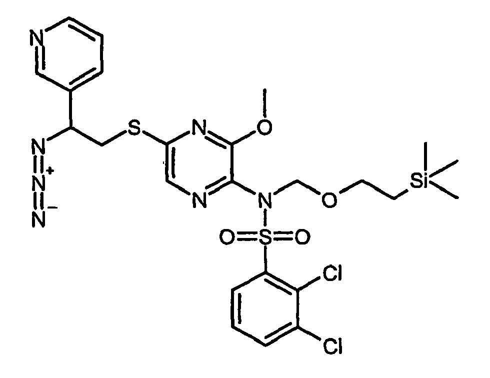 Procedure as for Example 18 step b) using the product from Example 22 step b) (0.29g). Yield 0.29g
   m/e 642 (M+1⁺)
d) *N*-[5-[[2-Azido-2-(3-pyridinyl)ethyl]thio]-3-methoxypyrazinyl]-2,3-dichlorobenzenesulfonamide 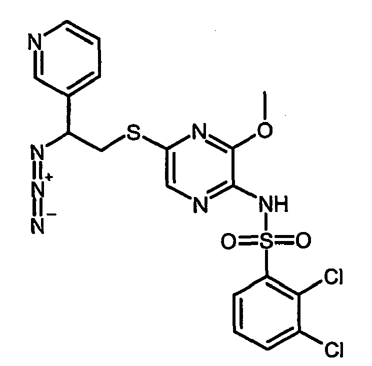 Procedure as for Example 1 step e) using the product from Example 22 step c) (0.29 g). Yield 0.23 g
   m/e 510 (M-1)⁻
e) *N*-[5-[[2-Amino-2-(3-pyridinyl)ethyl]thio]-3-methoxypyrazinyl]-2,3-dichloro benzenesulfonamide 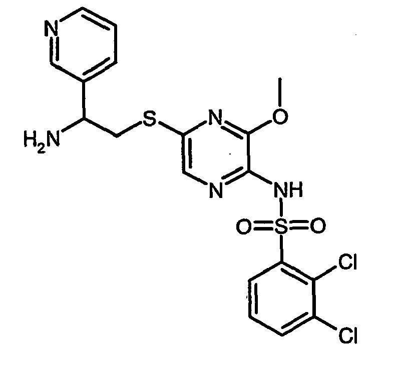 Procedure as for Example 18 step d) using the product from Example 22 step d) (0.23 g). Yield 0.025g.
   m/e 484 (M-1)⁻
   ¹H NMR (DMSO) δ 8.63 (1H, s), 8.53 (1H, d), 8.00 (1H, d), 7.87 (1H, d), 7.80 (1H, d), 7.48 (1H, t), 7.39 (2H, m), 4.53 (1H, t), 3.85 (3H, s), 3.55 (2H, m).
   MP 175-8°C

### Example 23

### 2,3-Dichloro-N-[5-[[2-hydroxy-2-(4-pyridinyl)ethyl]thio]-3-methoxypyrazinyl]benzenesulfonamide

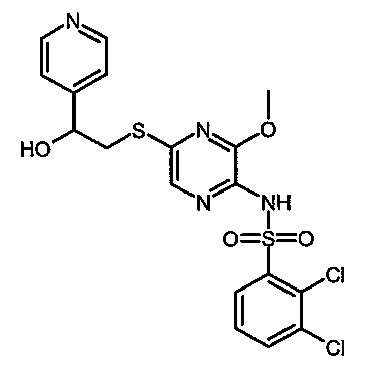

a) 2,3-Dichloro-*N*-[5-[[2-hydroxy-2-(4-pyridinyl)ethyl]thio]-3-methoxypyrazinyl]-*N*-[[2-(trimethylsilyl)ethoxy]methyl]benzenesulfonamide 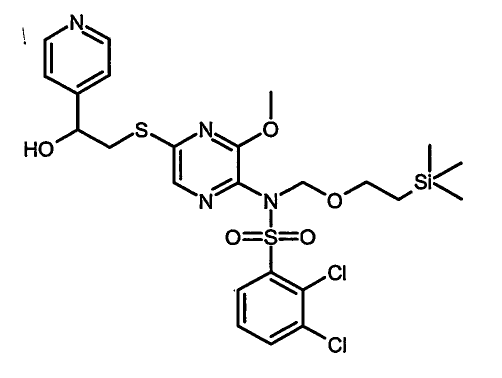 Procedure as for Example 16 step b) using 4-pyridinecarboxaldehyde (0.066 ml).
   Yield 0.180g
   m/e 617 (M+1⁺)
b) 2,3-Dichloro-*N*-[5-[[2-hydroxy-2-(4-pyridinyl)ethyl]thio]-3-methoxypyrazinyl]benzenesulfonamide 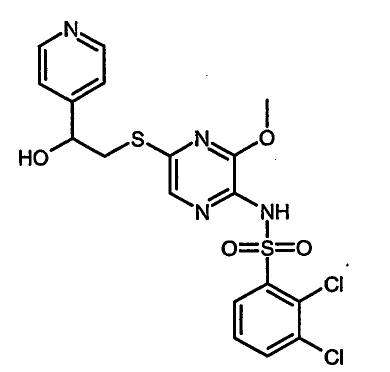 Procedure as for Example 1 step e) using the product from Example 23 step a) (0.14 g). Yield 0.055 g
   m/e 485 (M-1)⁻
   ¹H NMR (DMSO) δ 8.48 (2H, dd), 8.02 (1H, dd), 7.93 (1H, dd), 7.63 (1H, s), 7.57 (1H, t), 7.39 (2H, dd), 5.90 (1H, d), 4.81 (1H, m), 3.90 (3H, s), 3.46 (1H, dd), 3.35 (1H, dd).
   MP 176-7°C

### Example 24

### 2,3-Dichloro-N-[5-[[2-hydrozy-2-(2-pyridinyl)ethyl]thio]-3-methozypyrazinyl]benzenesulfonamide

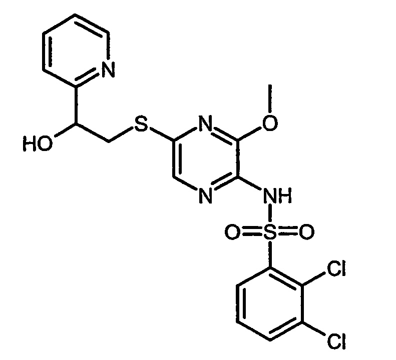

a) 2,3-Dichloro-*N*-[5-[[2-hydroxy-2-(2-pyridinyl)ethyl]thio]-3-methoxypyrazinyl]-*N*-[[2-(trimethylsilyl)ethoxy]methyl]benzenesulfonamide 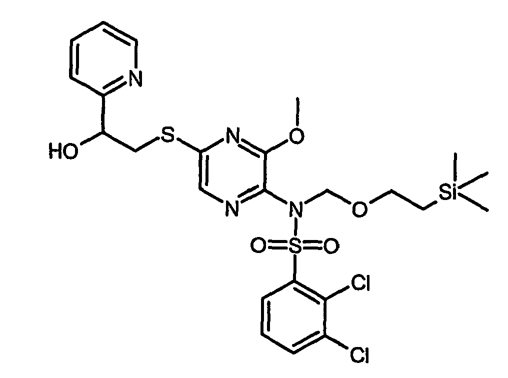 Procedure as for Example 16 step b) using 2-pyridinecarboxaldehyde (0.066 ml).
   Yield 0.10g
   m/e 617 (M+1⁺)
b) 2,3-Dichloro-*N*-[5-[[2-hydroxy-2-(2-pyridinyl)ethyl]thio]-3-methoxypyrazinyl]benzenesulfonamide 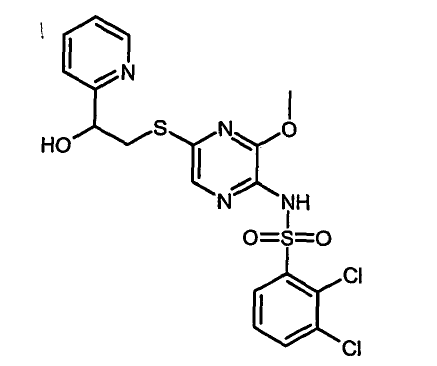 Procedure as for Example 1 step e) using the product from Example 24 step a) (0.10 g). Yield 0.016 g
   m/e 485 (M-1)⁻
   ¹H NMR (DMSO) δ 8.44 (1H, d), 8.02 (1H, d), 7.94 (1H, d), 7.73 (1H, t), 7.58 (2H, m), 7.49 (1H, d), 7.22 (1H, t), 5.82 (1H, s), 4.83 (1H, m), 3.91 (3H, s), 3.65 (1H, m), 3.35 (1H, m).
   MP 143-5°C

### Example 25

### 3-[[5-[[(2,3-Dichlorophenyl)sulfonyl]amino]-6-methozypyrazinyl]thio]-(2R)-2-hydroxypropanoic acid, methyl ester

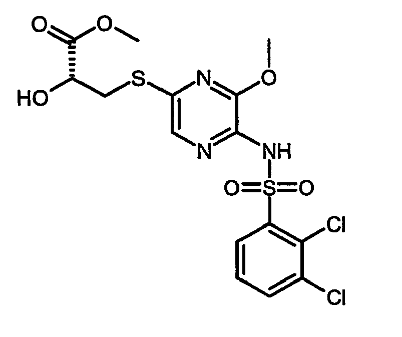

a) 3-[[5-[[(2,3-Dichlorophenyl)sulfonyl][[2-(trimethylsilyl)ethoxy]methyl]amino]-6-methoxypyrazinyl]thio]-(2R)-2-hydroxypropanoic acid, methyl ester. 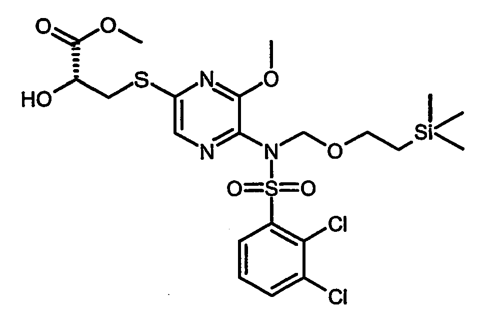 The product from Example 15 step a) (1.24 g) was dissolved in dry THF (10 ml) under an atmosphere of nitrogen and treated with a 1 M solution of potassium *tert*-butoxide in THF (2.3 ml), added dropwise. After stirring for 30 min the reaction was treated with (2S)-2-oxiranecarboxylic acid, methyl ester (1.0 ml) and allowed to stir for 20 hrs. The reaction mixture was poured into saturated aqueous ammonium chloride solution and extracted into ethyl acetate (x3). The combined ethyl acetate extracts were washed with water, brine and dried (MgSO4) and evaporated to leave an oil. The residue was purified by silica gel chromatography, eluting with 1:1 ethyl acetate:isohexane, to afford the sub-titled product as a colourless oil (0.23g).
   m/e 598 (M+1⁺)
b) 3-[[5-[[(2,3-Dichlorophenyl)sulfonyl]amino]-6-methoxypyrazinyl]thio]-(2R)-2-hydroxypropanoic acid, methyl ester. 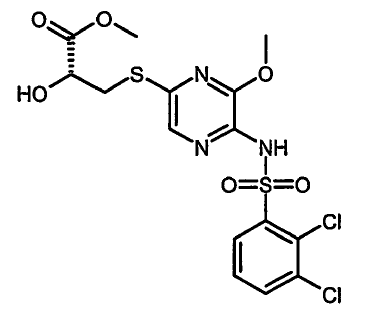 Procedure as for Example 1 step e) using the product from Example 25 step a) (0.23 g). Yield 0.012g.
   m/e 466 (M-1)⁻
   ¹H NMR (DMSO) δ 7.95 (1H, dd), 7.61 (1H, dd), 7.35 (1H, t), 7.19 (1H, s), 4.18 (1H, t), 3.80 (3H, s), 3.50 (3H, s), 3.21 (1H, dd), 3.04 (1H, dd).
   MP 88-90°C

### Example 26

### N-[5-[[2-Amino-2-(2-pyridinyl)ethyl]thio]-3-methozypyrazinyl]-2,3-dichlorobenzenesulfonamide

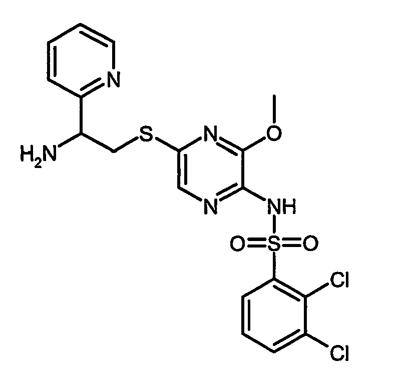

a) 2,3-Dichloro-*N*-[5-[[2-chloro-2-(2-pyridinyl)ethyl]thio]-3-methoxypyrazinyl]-*N*-[[2-(trimethylsilyl)ethoxy]methyl]benzenesulfonamide . 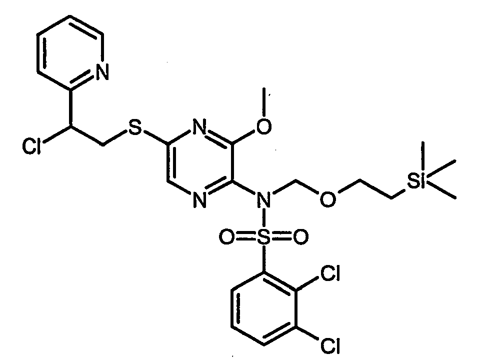 Procedure as for Example 18 step a) using the product from Example 24 step a) (0.70 g). Yield 0.72 g m/e 635 (M+1⁺)
b) *N*-[5-[[2-Azido-2-(2-pyridinyl)ethyl]thio]-3-methoxypyrazinyl]-2,3-dichloro-*N*-[[2-(trimethylsilyl)ethoxy]methyl]benzenesulfonamide 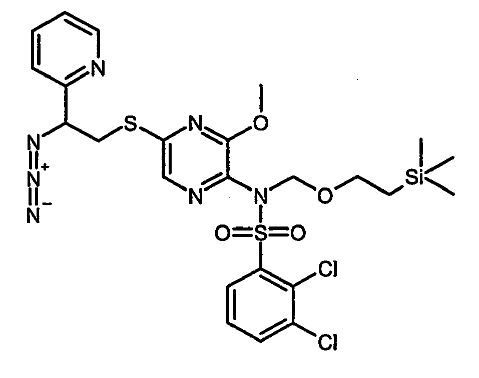 Procedure as for Example 18 step b) using the product from Example 26 step a) (0.72g). Yield 0.70 g m/e 642 (M+1⁺)
c) *N*-[5-[[2-Azido-2-(2-pyridinyl)ethyl]thio]-3-methoxypyrazinyl]-2,3-dichloro benzenesulfonamide 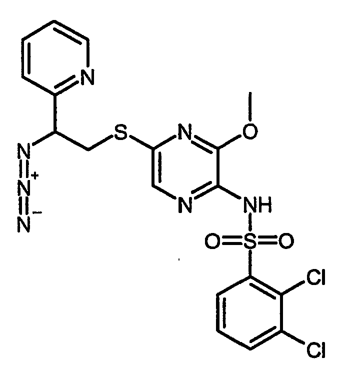 Procedure as for Example 1 step e) using the product from Example 26 step b) (0.70 g).
   Yield 0.34 g
   m/e 510 (M-1)⁻
   ¹H NMR (DMSO) δ 8.57 (1H, m), 8.04 (1H, d), 7.94 (1H, d), 7.81 (1H, t), 7.65 (1H, s), 7.58 (1H, t), 7.47 (1H, d), 7.35 (1H, m), 4.94 (1H, t), 3.94 (3H, s), 3.75 (1H, m), 3.65 (1H, m).
d) *N*-[5-[[2-Amino-2-(2-pyridinyl)ethyl]thio]-3-methoxypyrazinyl]-2,3-dichlorobenzenesulfonamide 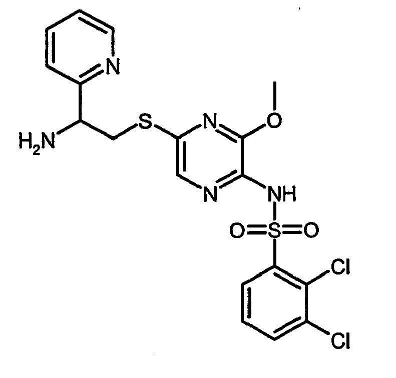 Procedure as for Example 18 step d) using the product from Example 26 step c) (0.15 g). Yield 0.018g.
   m/e 484 (M-1)⁻
   ¹H NMR (DMSO) δ 8.54 (1H, d), 7.95 (1H, d), 7.76 (1H, t), 7.62 (1H, d), 7.45 (1H, d), 7.33 (2H, m), 7.19 (1H, s), 4.42 (1H, t), 3.79 (3H, s), 3.30 (2H, m).
   MP 172-4°C

### Example 27

### N-[5-[[2-Amino-2-(1-methyl-1H-imidazol 2-yl)ethyl]thio]-3-methoxypyrazinyl]-2,3-dichlorobenzenesulfonamide

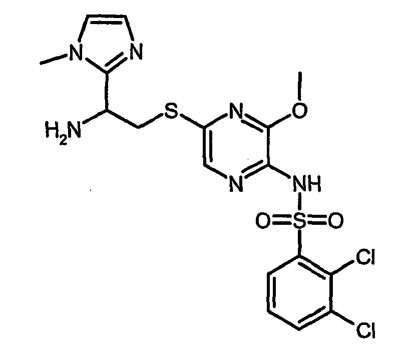

a) 2,3-Dichloro-*N*-[5-[[2-chloro-2-(1-methyl-1*H*-imidazol-2-yl)ethyl]thio]-3-methoxypyrazinyl]-*N*-[[2-(trimethylsilyl)ethoxy]methyl]benzenesulfonamide 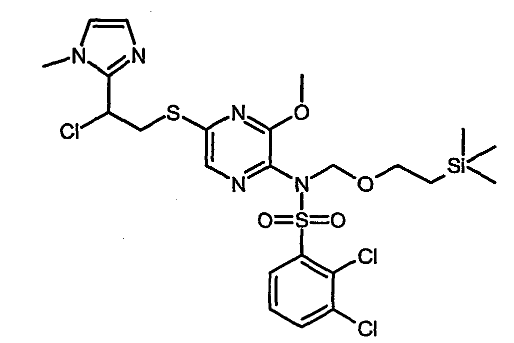 Procedure as for Example 18 step a) using the product from Example 16 step b) (0.44 g). Yield 0.45 g m/e 638 (M+1⁺)
b) *N*-[5-[[2-Azido-2-(1-methyl-1*H*-imidazol-2-yl)ethyl]thio]-3-methoxypyrazinyl]-2,3-dichloro-*N*-[[2-(trimethylsilyl)ethoxy]methyl]benzenesulfonamide 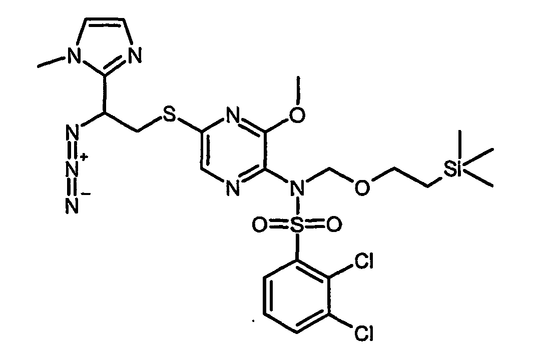 Procedure as for Example 18 step b) using the product from Example 27 step a) (0.72g). Yield 0.455 g m/e 645 (M+1⁺)
c) *N*-[5-[[2-Azido-2-(1-methyl-1*H*-imidazol-2-yl)ethyl]thio]-3-methoxypyrazinyl]-2,3-dichlorobenzenesulfonamide 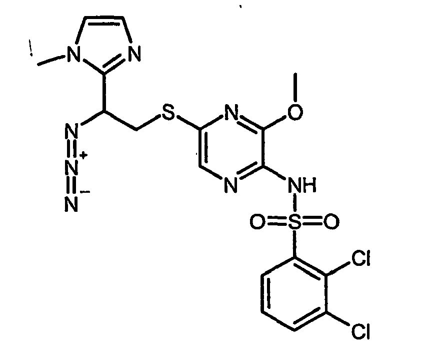 Procedure as for Example 1 step e) using the product from Example 27 step b) (0.45 g). Yield 0.36 g
   m/e 513 (M-1)⁻
d) *N*-[5-[[2-Amino-2-(1-methyl-1*H*-imidazol-2-yl)ethyl]thio]-3-methoxypyrazinyl]-2,3-dichlorobenzenesulfonamide 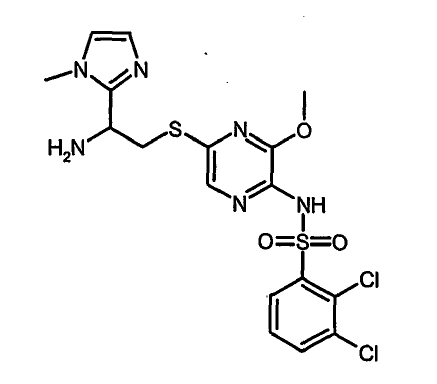 Procedure as for Example 18 step d) using the product from Example 27 step c) (0.36 g). Yield 0.038g.
   m/e 489 (M+1⁺)
   ¹H NMR (DMSO) δ 8.04 (1H, d), 7.94 (1H, d), 7.59 (2H, m), 7.05 (1H, s), 6.84 (1H, s), 4.77 (1H, t), 3.95 (3H, s), 3.58 (3H, s), 3.5-3.7 (2H, m).
   MP 187-9°C

### Example 28

### (2R)-2-Amino-3-[[3-chloro-5-[[(2,3-dichlorophenyl)sulfonyl]amino]-6-methozypyrazinyl]thio]-N-methylpropanamide

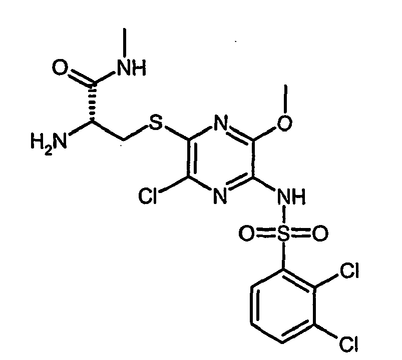

a) *N,N'*-bis[(1,1-dimethylethoxy)carbonyl]-*N,N'*-dimethyl-L-cystinamide 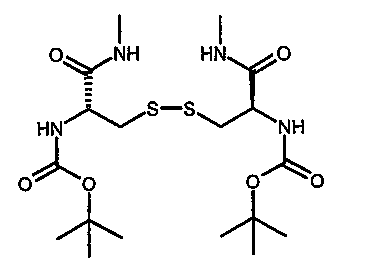 A solution of *N,N*'-bis[(1,1-dimethylethoxy)carbonyl]-L-cystine (4.08g) in tetrahydrofuran (100 mL) was cooled to 0°C under nitrogen and isobutyl chloroformate (2.9 mL) was added dropwise followed by triethylamine (3.1 ml) dropwise. After 30 minutes at 0°C a 2.0M solution of methylamine in THF (20 ml) was added. After stirring at room temperature for 24 hours, the reaction mixture was poured into saturated ammonium chloride solution and extracted with ethyl acetate (x3). The combined extracts were washed with saturated brine, dried (MgSO₄) and the solvent was evaporated to afford the subtitled product as a white solid (2.6 g). m/e 467 (M+1⁺)
b) [(1*R*)-1-(Mercaptomethyl)-2-(methylamino)-2-oxoethyl]carbamic acid, 1,1-dimethylethyl ester 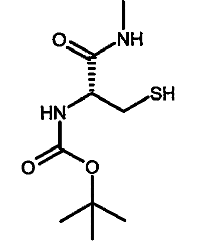 To a suspension of the product from Example 28 step a) (2.15 g) in 1,1,1-trifluoroethanol (5 mL) and water (0.6 ml) was added dropwise tributylphosphine (2.25 ml) followed by triethylamine (0.10 ml). After stirring at room temperature for 24 hours, ethyl acetate and silica were added and the mixture evaporated. The residue was purified by silica gel chromatography, eluting with 1:1 ethyl acetate:isohexane, to afford the sub-titled product as a white solid (1.85 g).
   m/e 233 (M-1)⁻
   ¹H NMR (CDCl₃) δ 6.33 (1H, bs), 5.38 (1H, bs), 4.32 (1H, bs), 3.15 (1H, m), 2.85 (3H, d), 2.70 (1H, m), 1.55 (1H, t), 1.46 (9H, s).
c) [(1*R*)-1-[[[3-Chloro-5-[[(2,3-dichlorophenyl)sulfonyl][[2-(trimethylsilyl)ethoxy]methyl]amino]-6-methoxypyrazinyl]thio]methyl]-2-(methylamino)-2-oxoethyl]carbamic acid, 1,1-dimethylethyl ester 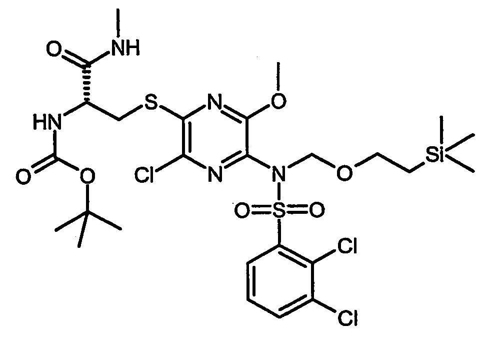 Procedure as for Example 2 step a) using *N*-(5-bromo-6-chloro-3-methoxypyrazinyl)-2,3-dichloro-*N*-[[2-(trimethylsilyl)ethoxy]methyl]benzenesulfonamide (0.25 g) and [(1*R*)-1-(mercaptomethyl)-2-(methylamino)-2-oxoethyl]carbamic acid, 1,1-dimethylethyl ester (0.105 g).
   Yield 0.24 g.
d) (2R)-2-Amino-3-[[3-chloro-5-[[(2,3-dichlorophenyl)sulfonyl]amino]-6-methoxypyrazinyl]thio]-*N*-methylpropanamide 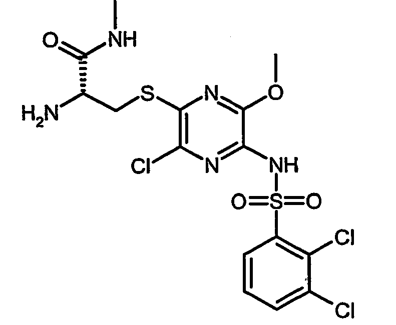 Procedure as for Example 1 step e) using the product from Example 28 step c) (0.24 g). Yield 0.03 g.
   m/e 498 (M-1)⁻
   ¹H NMR (DMSO) δ 8.25 (1H, d), 8.02 (1H, d), 7.63 (1H, d), 7.60 (2H, bs), 7.37 (1H, t), 3.80 (4H, m), 3.30 (2H, m), 2.55 (3H, d).
   MP 150-5°C

### Example 29

### (2R)-2-Amino-3-[[5-[[(2,3-dichlorophenyl)sulfonyl]amino]-6-methoxy-3-methylpyrazinyl]thio]-N-methylpropanamide

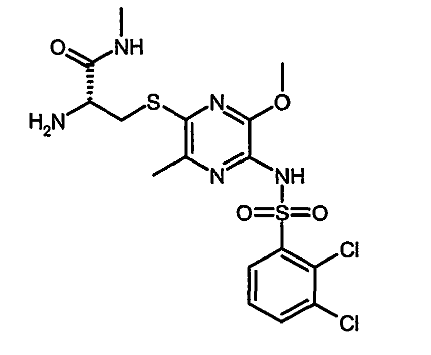

a) [(1*R*)-1-[[[5-[[(2,3-Dichlorophenyl)sulfonyl][[2-(trimethylsilyl)ethoxy]methyl]amino]-6-methoxy-3-methylpyrazinyl]thio]methyl]-2-(methylamino)-2-oxoethyl]carbamic acid, 1,1-dimethylethyl ester 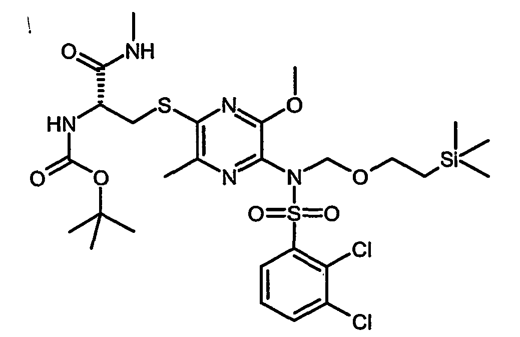 Procedure as for Example 2 step a) using the product from Example 11 step e) (0.25 g) and [(1*R*)-1-(mercaptomethyl)-2-(methylamino)-2-oxoethyl]carbamic acid, 1,1-dimethylethyl ester (0.11 g).
   Yield 0.32 g.
b) (2*R*)-2-Amino-3-[[5-[[(2,3-dichlorophenyl)sulfonyl]amino]-6-methoxy-3-methylpyrazinyl]thio]-*N*-methylpropanamide 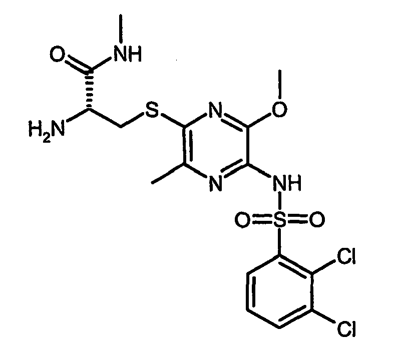 Procedure as for Example 1 step e) using the product from Example 29 step a) (0.32 g). Yield 0.115 g.
   m/e 478 (M-1)⁻
   ¹H NMR (DMSO) δ 8.06 (1H, dd), 7.80 (1H, m), 7.58 (1H, dd), 7.35 (1H, t), 3.77 (3H, s), 3.27 (2H, m), 2.93 (1H, m), 2.50 (3H, d), 1.87 (3H, s).
   MP 68-80°C

### Example 30

### N-[5-[[2-Amino-2-(2-thiazolyl)ethyl]thio]-3-methozypyrazinyl]-2,3-dichlorobenzenesulfonamide

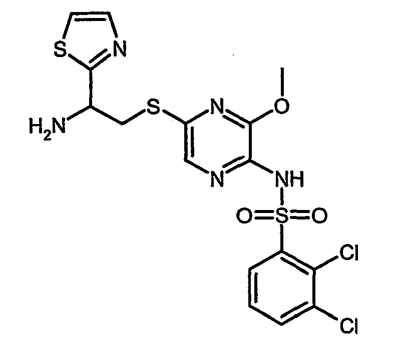

a) 2,3-Dichloro-*N*-[5-[[2-hydroxy-2-(2-thiazolyl)ethyl]thio]-3-methoxypyrazinyl]-*N*-[[2-(trimethylsilyl)ethoxy]methyl]benzenesulfonamide 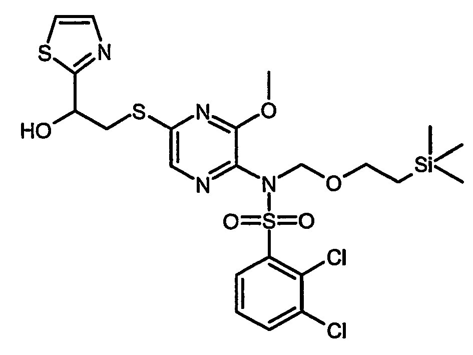 Procedure as for Example 16 step (b) using thiazole-2-carboxaldehyde (1.0 g).
   Yield 0.75 g m/e 623 (M+1⁺)
b) 2,3-Dichloro-*N*-[5-[[2-chloro-2-(2-thiazolyl)ethyl]thio]-3-methoxypyrazinyl]-*N*-[[2-(trimethylsilyl)ethoxy]methyl]benzenesulfonamide 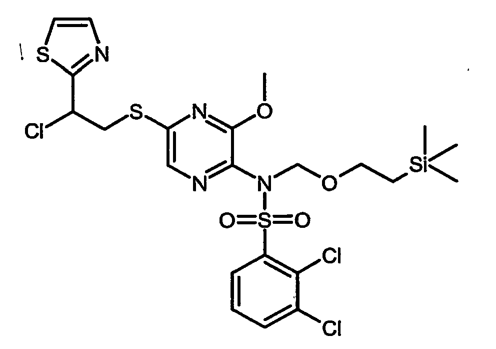 Procedure as for Example 18 step a) using the product from Example 30 step a) (0.75 g). Yield 0.77 g m/e 639 (M+1⁺)
c) *N*-[5-[[2-Azido-2-(2-thiazolyl)ethyl]thio]-3-methoxypyrazinyl]-2,3-dichloro-*N*-[[2-(trimethylsilyl)ethoxy]methyl]benzenesulfonamide 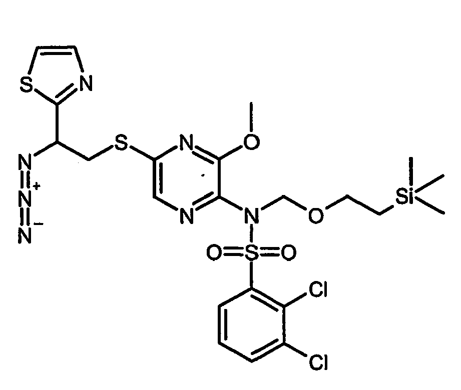 Procedure as for Example 18 step b) using the product from Example 30 step b) (0.77 g). Yield 0.78 g m/e 648 (M+1⁺)
d) *N*-[5-[[2-Azido-2-(2-thiazolyl)ethyl]thio]-3-methoxypyrazinyl]-2,3-dichlorobenzenesulfonamide 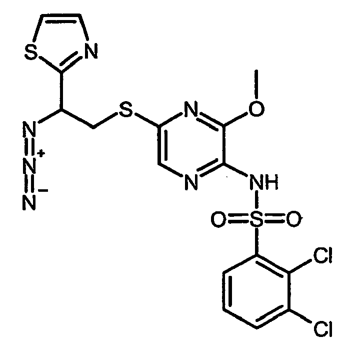 Procedure as for Example 1 step e) using the product from Example 30 step c) (0.78 g). Yield 0.62 g
   m/e 516 (M-1)⁻
e) *N*-[5-[[2-Amino-2-(2-thiazolyl)ethyl]thio]-3-methoxypyrazinyl]-2,3-dichlorobenzenesulfonamide 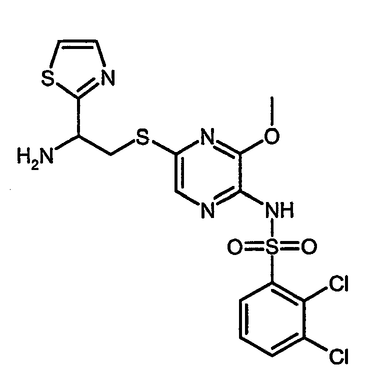 Procedure as for Example 18 step d) using the product from Example 30 step d) (0.62 g). Yield 0.15 g. m/e 492 (M+1⁺)
   ¹H NMR (DMSO) δ 8.16 (2H, bs), 7.98 (1H, d), 7.85 (1H, d), 7.77 (1H, d), 7.68 (1H, d), 7.41 (1H, t), 7.32 (1H, s), 4.77 (1H, t), 4.09 (1H, bs), 3.78 (3H, s), 3.4-3.6 (2H, m).
   MP 168-9°C

### Example 31

### N-[5-[[(2R)-2-Amino-3-hydroxypropyl]oxy]-3-methoxypyrazinyl]-2,3-dichlorobenzenesulfonamide, monohydrochloride

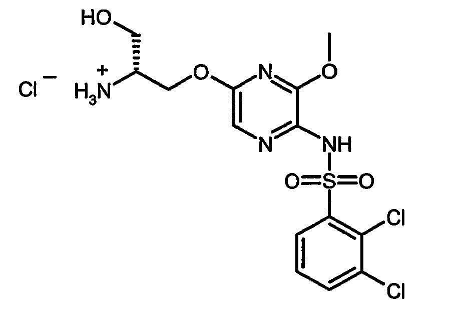

2,3-Dichloro-*N*-(5-chloro-3-methoxypyrazinyl)-*N*-[[2-(trimethylsilyl)ethoxy]methyl]-benzenesulfonamide (0.25g) and (4*S*)-4-(hydroxymethyl)-2,2-dimethyl-3-oxazolidinecarboxylic acid 1,1-dimethylethyl ester (0. 142g) were dissolved in dry 1,2-dimethoxyethane (5ml) under an atmosphere of nitrogen and treated with sodium hydride (60% suspension in oil, 0.026g). After stirring at ambient temperature overnight the reaction was poured into water and extracted twice with ethyl acetate. The organic extracts were washed with brine, dried (MgSO₄) and evaporated to give a pale yellow oil (0.413g). The oil was dissolved in dichloromethane (5ml) and treated with trifluoroacetic acid (1ml). After stirring at ambient temperature overnight, methanol (5ml) was added and the solution was stirred for 1 hour. The mixture was diluted with dichloromethane, flash silica (5g) was added and the the solvents were removed *in vacuo.* The resulting powder was purified by flash chromatography on silica, eluting with 5% and 10% methanol in dichloromethane to afford a colourless residue (0.164g). After dissolving the residue in methanol and treating it with 4M HCl solution in 1,4-dioxane (1ml), the solvents were removed *in vacuo.* Trituration with diethyl ether afforded a solid that was removed by filtration and dried in *vacuo* at 40° to give the title compound as an off-white powder.
Yield 0.108g
m.p. 130-150°C (decomposes)
m/e 423/425 (M+1)⁺
¹H NMR (D₂O) 8.06 (1H, m), 7.95 (1H, m), 7.55 (2H, m), 4.69 (1H, m), 4.57 (1H, m), 4.02 (1H, m), 3.95 (3H, s), 3.90 (2H, m).

### Example 32

### 2,3-Dichloro-N-[5-[((2S)-2,3-dihydrozypropyl]oxy]-3-methozypyrazinyl]-benzenesulfonamide

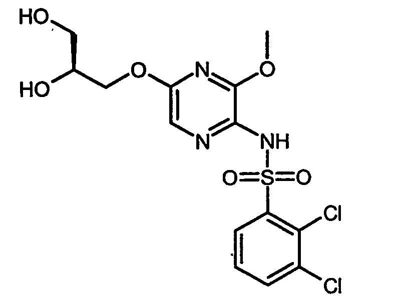

2,3-Dichloro-*N*-(5-chloro-3-methoxypyrazinyl)-*N*-[[2-(trimethylsilyl)ethoxy]methyl]-benzenesulfonamide (0.25g) and (4*R*)-2,2-dimethyl-1,3-dioxolane-4-methanol (0.079g) were dissolved in dry 1,2-dimethoxyethane (5ml) under an atmosphere of nitrogen and treated with sodium hydride (60% suspension in oil, 0.026g). After stirring at ambient temperature overnight the reaction was poured into water and extracted twice with ethyl acetate. The organic extracts were washed with brine, dried (MgSO₄) and evaporated to give a colourless gum (0.230g). The gum was dissolved in dichloromethane (5ml) and treated with trifluoroacetic acid (1ml). After stirring at ambient temperature for 2 hours, methanol (5ml) was added and the solution was stirred for 2.5 hours. More trifluoroacetic acid (2ml) was then added and the mixture was stirred for a further 3 hours. Flash silica (5g) was added and the the solvents were removed *in vacuo.* The resulting powder was purified by flash chromatography on silica, eluting with 5% methanol in dichloromethane to afford a white foam (0.055g). Further purification by reversed phase preparative hplc gave the title compound as a white foam.
Yield 0.026g
m/e 424/426 (M+1)⁺
¹H NMR (DMSO-d6) 7.95 (1H, m), 7.85 (1H, m), 7.49 (1H, m), 7.34 (1H, s), 4.93 (1H, d), 4.63 (1H, t), 4.23 (1H, m), 4.08 (1H, m), 3.80 (3H, s), 3.76 (1H, m), 3.40 (2H, m).

### Example 33

### 2,3-Dichloro-N-[5-[[(2R)-2,3-dihydrozypropyl]ozy]-3-methogypyrazinyl]-benzenesulfonamide

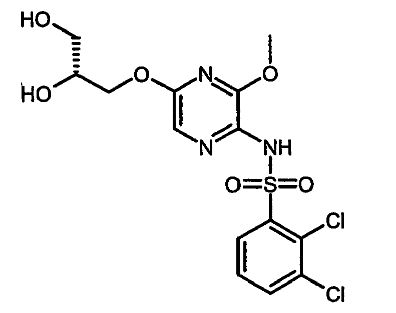

2,3-Dichloro-*N*-(5-chloro-3-methoxypyrazinyl)-*N*-[[2-(trimethylsilyl)ethoxy]methyl]-benzenesulfonamide (0.25g) and (4*S*)-2,2-dimethyl-1,3-dioxolane-4-methanol, (0.080g) were dissolved in dry 1,2-dimethoxyethane (5ml) under an atmosphere of nitrogen and treated with sodium hydride (60% suspension in oil, 0.026g). After stirring at ambient temperature overnight the reaction was poured into water and extracted twice with ethyl acetate. The organic extracts were washed with brine, dried (MgSO₄) and evaporated to give a colourless gum (0.346g). The gum was dissolved in dichloromethane (5ml) and treated with trifluoroacetic acid (1ml). After stirring at ambient temperature for 2 hours, methanol (5ml) was added and the solution was stirred for 2.5 hours. More trifluoroacetic acid (2ml) was then added and the mixture was stirred for a further 3 hours. Flash silica (5g) was added and the the solvents were removed *in vacuo.* The resulting powder was purified by flash chromatography on silica, eluting with 4% methanol in dichloromethane to afford a white foam (0.060g). Further purification by reversed phase preparative hplc gave the title compound as a white foam.
Yield 0.035g
m/e 424/426 (M+1)⁺
¹H NMR (DMSO-d6) 7.94 (1H, m), 7.74 (1H, m), 7.42 (1H, m), 7.18 (1H, s), 4.89 (1H, d), 4.61 (1H, t), 4.16 (1H, m), 4.00 (1H, m), 3.78 (3H, s), 3.75 (1H, m), 3.39 (2H, m).

### Example 34

### N-[5-[[(2R)-2-Amino-2-(3-methyl-1,2,4-oxadiazol-5-yl)ethyl]thio]-3-methoxypyrazinyl]-2,3-dichlorobenzenesulfonamide

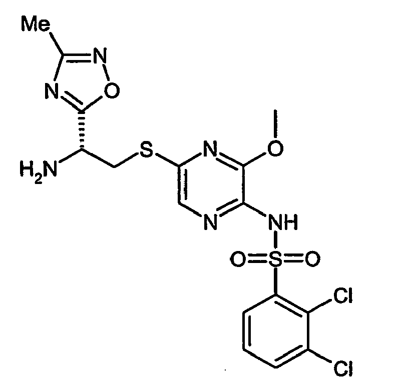

*N*-(5-Bromo-3-methoxypyrazinyl)-2,3-dichloro-*N*-[[2-(trimethylsilyl)ethoxy]methyl]-benzenesulfonamide (0.25g), [(1*R*)-2-mercapto-1-(3-methyl-1,2,4-oxadiazol-5-yl)ethyl]-carbamic acid 1,1-dimethylethyl ester (0.133g) and caesium carbonate (0.166g) in acetonitrile (5ml) were stirred at ambient temperature overnight. The reaction was poured into a mixture of water and brine and extracted twice with ethyl acetate. The organic extracts were washed with brine, dried (MgSO₄) and evaporated to give a yellow gum (0.385g). The gum was dissolved in dichloromethane (5ml) and treated with trifluoroacetic acid (2.5ml). After stirring at ambient temperature for 1 hour the solvents were removed *in vacuo* and the residue was purified by reversed phase preparative hplc to afford the title compound as a pale orange powder.
Yield 0.062g
m/e 491/493 (M+1)⁺
¹H NMR (DMSO-d6) 8.05 (1H, m), 7.95 (1H, m), 7.63 (1H, s), 7.59 (1H, m), 5.11 (1H, t), 3.97 (3H, s), 3.78 (2H, broad d), 2.26 (3H, s).

### Example 35

### N-[5-[[(2R)-2-Amino-2-(3-methyl-1,2,4-oacadiazol-5-yl)ethyl]thio]-3-methozy-6-methylpyrazinyl]-2,3-dichlorobenzenesulfonamide

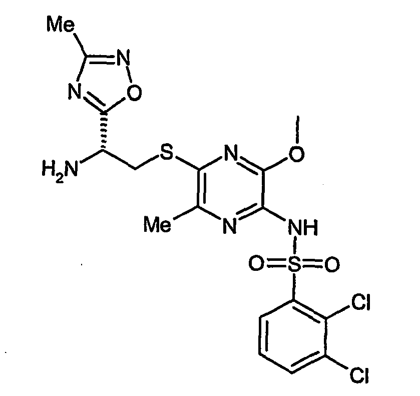

*N*-(5-Bromo-3-methoxy-6-methylpyrazinyl)-2,3-dichloro-*N*-[[2-(trimethylsilyl)ethoxy]methyl]-benzenesulfonamide (0.251g), [(1*R*)-2-mercapto-1-(3-methyl-1,2,4-oxadiazol-5-yl)ethyl]-carbamic acid 1,1-dimethylethyl ester (0.143g) and caesium carbonate (0.184g) in acetonitrile (5ml) were stirred at ambient temperature overnight. More [(1*R*)-2-mercapto-1-(3-methyl-1,2,4-oxadiazol-5-yl)ethyl]-carbamic acid 1,1-dimethylethyl ester (0.082g), caesium carbonate (0.089g) and acetonitrile (5ml) were added and the mixture was stirred at ambient temperature for 2 more days. The reaction was poured into a mixture of water and brine and extracted three times with ethyl acetate. The organic extracts were washed with brine, dried (MgSO₄) and evaporated to give a brown gum (0.441g). The gum was dissolved in dichloromethane (5ml) and treated with trifluoroacetic acid (2.5ml). After stirring at ambient temperature for 2.75 hours, flash silica (5g) was added and the solvents were removed *in vacuo.* The resulting powder was purified by flash chromatography on silica, eluting with 2% and 5% methanol in dichloromethane to afford a pale brown foam (0.095g). Further purification by reversed phase preparative hplc afforded the titled compound as a pale yellow powder.
Yield 0.021g
m/e 505/507 (M+1)⁺
¹H NMR (DMSO-d6) 8.09 (1H, d), 7.83 (1H, d), 7.52 (1H, t), 4.48 (1H, t), 3.85 (3H, s), 3.45-3.60 (2H, m), 2.22 (3H, s), 1.95 (3H, s).

### Example 36

### N-[5-[[(2R)-2-Amino-2-(3-methyl-1,2,4-ozadiazol-5-yl)ethyl]thio]-6-chloro-3-methoxypyrazinyl]-2,3-dichlorobenzenesulfonamide

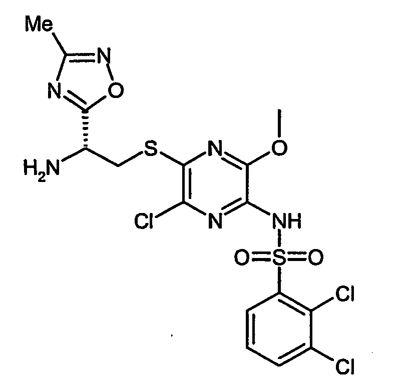

A solution of *N*-(5-Bromo-6-chloro-3-methoxypyrazinyl)-2,3-dichloro-*N*-[[2-(trimethylsilyl)ethoxy]methyl]benzenesulfonamide (0.203g), [(1*R*)-2-mercapto-1-(3-methyl-1,2,4-oxadiazol-5-yl)ethyl]-carbamic acid 1,1-dimethylethyl ester (0.158g) and *N*,*N*-diisopropylethylamine (0.060ml) in *N*-methylpyrrolidinone (3ml) was stirred at 50° overnight. The reaction mixture was concentrated *in vacuo* to give a brown residue that was dissolved in dichloromethane (5ml) and treated with trifluoroacetic acid (2.5ml). After stirring at ambient temperature for 1.25 hours the solvents were removed *in vacuo* and the residue was purified by reversed phase preparative hplc to afford a white solid. Recrystallisation from aqueous methanol gave the title compound an off-white powder.
Yield 0.035g
m/e 527 (M+1)⁺
¹H NMR (DMSO-d6) 8.24 (1H, br), 8.02 (1H, d), 7.70 (1H, d), 7.42 (1H, t), 4.94 (1H, t), 3.86 (3H, s), 3.66 (1H, m), 3.53 (1H, m), 2.29 (3H, s).

### Pharmacological Analysis

### FMAT Whole cell binding assay

### Cells

CHO-Kl cells stably expressing the human recombinant CCR4 receptor (Euroscreen; Brussels, Belgium) were cultured in NUT.MIX.F_12(HAM) medium with glutamax-1, containing 10% (v/v) foetal bovine serum and 400 µg ml⁻¹ geneticin.

Cells were harvested at approximately 70% confluence by treatment with a cell dissociation buffer, and seeded at 5x10³ cells/100µl culture medium into wells of a black Costar clear-bottomed 96-well microtitre plates. Plates were incubated overnight at 37°C in 5% CO₂ and used the following day.

### ASSAY

Before use, the cell plates were washed twice with 100 µl Hanks balanced salt solution (HBSS). To each well was then added 65µl of HBSS, 10 µL of 10% DMSO in HBSS ± test compound and then 25 µL of 2.8 nM FB-MDC (Applied Biosystems). This fluorescent probe was prepared from a 10µM stock in 0.08% (v/v) TFA/16% (v/v) acetonitrile, diluted into HBSS.

After two hours incubation in the dark at room temperature, the plates were analysed in an FMAT8100 reader (Applied Biosystems) to measure fluorescence that was associated with binding of FB-MDC to the cells. Compound activity was determined as an pIC₅₀ [log(concentration of compound that results in 50% inhibition)], comparing fluorescence in control and background wells.

### Typical Data

Fluorescence (ctrl) =1200
Fluorescence (bkg) = 0

The compounds of the examples all have a pIC₅₀ of greater than 5.0.

Data for specific compounds is given below.

| | Mean | |
|---|---|---|
| Example 4 | pIC₅₀ | 6.2 |
| Example 15 | pIC₅₀ | 6.4 |

## Claims

1. A compound of formula (I) or a pharmaceutically acceptable salt or solvate thereof: 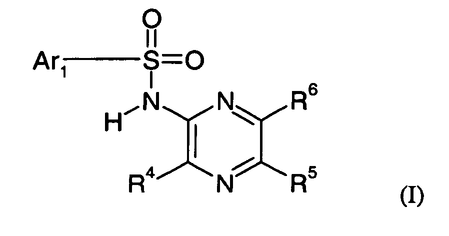 in which:
Ar¹ is phenyl or thienyl, each of which is optionally substituted by one to three substituents R¹, R² and R³ selected from halogen, cyano, CF₃, OCF₃, OC₁₋₆ alkyl or C₁₋₆ alkyl;
R⁴ is C₁₋₆ alkoxy where the alkyl group may form a 3-6 membered saturated ring or may be substituted with 1-3 fluorine atoms or a cyano group; or
OC₁₋₆ alkylR¹¹, or OC₂₋₆ alkyl-X-R¹¹ where the alkyl group may form a 3-6 membered saturated ring and is optionally substituted with 1-3 groups selected from hydroxy, halogen, NR¹⁴R¹⁵, SR¹³, S(O)₂R¹³, S(O)R¹³ or COR¹³;
one of R⁵ or R⁶ is XCH₂C₁₋₄ alkyl where the alkyl group is substituted at one position by R¹¹ and either NR¹⁴R¹⁵ or hydroxy, or R⁵/R⁶ is XR¹⁶ where R¹⁶ is a 4-8 membered saturated ring containing 1-3 heteroatoms selected from nitrogen, oxygen or sulphur and optionally substituted with 1-3 groups selected from hydroxy, cyano, halogen and =O, and R¹⁶ is substituted by R¹¹;
and the other is hydrogen, halogen, amino, NHC₁₋₆ alkyl, N(C₁₋₆ alkyl)₂, C₁₋₆ alkoxy or C₁₋₆ alkyl optionally substituted by one or more fluoro or hydroxyl groups;
X is NR¹³, O, S, S(O) or S(O)₂;
R¹¹ is an aryl group or a 5-7 membered heteroaromatic ring containing 1-4 heteroatoms selected from nitrogen, oxygen or sulphur, which aryl group or heteroaromatic ring can be optionally substituted by 1-3 groups selected from halogen, C(O)NR¹⁴R¹⁵, C(O)OR¹², hydroxy, =O, =S, CN, NO₂, COR¹³, NR¹⁴R¹⁵, X(CH₂)qNR¹⁴R¹⁵, (CH₂)nNR¹⁴R¹⁵, (CH₂)nOH, SR¹³, S(O)R¹³, S(O)₂R¹³
C₁₋₆ alkyl-X-C₁₋₆ alkyl, C₁₋₆ alkyl or C₁₋₆ alkoxy where the alkyl group may form a 3-6 membered ring or is optionally substituted with 1-3 groups selected from hydroxy, halogen, NR¹⁴R¹⁵, SR¹³, S(O)R¹³, S(O)₂R¹³; or
R¹¹ is C(O)NR¹⁴R¹⁵, C(O)OR¹², CH₂OR¹²
R¹² and R¹³ are independently hydrogen or C₁₋₆ alkyl where the alkyl group may be substituted with 1-3 fluorine atoms or may form a saturated 3-6 membered ring;
R¹⁴ and R¹⁵ are independently hydrogen, C₁₋₆ alkyl, C₃₋₆ cycloalkyl, (CH₂)qOH or (CH₂)_{q}NH₂,
or R¹⁴ and R¹⁵ together with the nitrogen atom to which they are attached form a 4-8 membered saturated ring containing 1-3 heteroatoms selected from nitrogen, oxygen and sulphur and optionally substituted by C₁₋₆ alkyl, C₁₋₆ alkyl-OH, or hydroxy; and
n is 1 ,2, 3, 4 or 5; and
q is 2, 3, 4, 5 or 6.

2. A compound according to claim 1 in which Ar¹ is phenyl optionally substituted by one or more halogen atoms.

3. A compound according to claim 1 or 2 in which R⁴ is methoxy.

4. A compound according to any one of claims 1 to 3 in which R⁵ is XCH₂CH(R¹¹)NR¹⁴R¹⁵ where R¹¹ is CO₂Me or CONHMe or a 5 or 6-membered heterocycle and NR¹⁴R¹⁵ is NH₂ or NHMe and X is S or O.

5. A compound according to any one of claims 1 to 4 in which R⁶ is hydrogen, chloro or methyl.

6. A compound of formula (I) selected from:
S-[5-[[(2,3-Dichlorophenyl)sulfonyl]amino]-6-methoxypyrazinyl]-D-cysteine, methyl ester
S-[5-[[(2,3-Dichlorophenyl)sulfonyl]amino]-6-methoxypyrazinyl]-L-cysteine, methyl ester
S-[5-[[(2,3-dichlorophenyl)sulfonyl]amino]-6-methoxypyrazinyl]-L-cysteine
(2*R*)-2-amino-3-[[5-[[(2,3-dichlorophenyl)sulfonyl]amino]-6-methoxypyrazinyl]thio]propanamide
(2*R*)-2-amino-3-([5-[[(2,3-dichlorophenyl)sulfonyl]amino]-6-methoxypyrazinyl]thio]propanamide
(2*R*)-2-amino-3-[[5-[[(2,3-dichlorophenyl)sulfonyl]amino]-6-methoxypyrazinyl]thio]-*N,N-*dimethylpropanamide
*N*-[5-[[(2*R*)-2-amino-3-hydroxypropyl]thio]-3-methoxypyrazinyl]-2,3-dichloro benzenesulfonamide
*S*-[3-chloro-5-[[(2,3-dichlorophenyl)sulfonyl]amino]-6-methoxypyrazinyl]-L-cysteine, methyl ester
*S*-[3-chloro-5-[[(2,3-dichlorophenyl)sulfonyl]amino]-6-methoxypyrazinyl]-L-cysteine *N*-[5-[[(2*R*)-2-amino-3-hydroxypropyl]thio]-6-chloro-3-methoxypyrazinyl]-2,3-dichlorobenzenesulfonamide
*S*-[5-[[(2,3-Dichlorophenyl)sulfonyl]amino]-6-methoxy-3-methylpyrazinyl]-L-cysteine, methyl ester
*S*-[5-[[(2,3-dichlorophenyl)sulfonyl]amino]-6-methoxy-3-methylpyrazinyl]-L-cysteine,
*N*-(2-Aminoethyl)-*S*-[5-[[(2,3-dichlorophenyl)sulfonyl]amino]-6-methoxypyrazinyl]-L-cysteine, ethyl ester
*N*-[5-[(2R)-2-amino-2-phenylethoxy]-3-methoxypyrazinyl]-2,3-dichlorobenzenesulfonamide
2,3-Dichloro-*N*-[5-[[2-hydroxy-2-(2-thiazolyl)ethyl]thio]-3-methoxypyrazinyl]-benzenesulfonamide,
2,3-Dichloro-*N*-[5-[[2-hydroxy-2-(1-methyl-1*H*-imidazol-2-yl)ethyl]thio]-3methoxypyrazinyl]-benzenesulfonamide, potassium salt
2,3-Dichloro-*N*-[5-[[2-hydroxy-2-(2-oxazolyl)ethyl]thio]-3-methoxypyrazinyl]-benzenesulfonamide
*N*-[5-[[2-amino-2-(2-oxazolyl)ethyl]thio]-3-methoxypyrazinyl]-2,3-dichlorobenzenesulfonamide
2,3-dichloro-*N*-[5-[(2,3-dihydroxypropyl)thio]-3-methoxypyrazinyl] benzenesulfonamide
2,3-dichloro-*N*-[5-[(2-hydroxy-2-phenylethyl)thio]-3-methoxypyrazinyl] benzenesulfonamide
2,3-dichloro-*N*-[5-[[2-hydroxy-2-(3-pyridinyl)ethyl]thio]-3-methoxypyrazinyl] benzenesulfonamide
*N*-[5-[[2-amino-2-(3-pyridinyl)ethyl]thio]-3-methoxypyrazinyl]-2,3-dichloro benzenesulfonamide
2,3-dichloro-N-[5-[[2-hydroxy-2-(4-pyridinyl)ethyl]thio]-3-methoxypyrazinyl]benzenesulfonamide
2,3-dichloro-*N*-[5-[[2-hydroxy-2-(2-pyridinyl)ethyl]thio]-3-methoxypyrazinyl]benzenesulfonamide
3-[[5-[[(2,3-dichlorophenyl)sulfonyl]amino]-6-methoxypyrazinyl]thio]-(2R)-2-hydroxypropanoic acid, methyl ester
*N*-[5-[[2-amino-2-(2-pyridinyl)ethyl]thio]-3-methoxypyrazinyl]-2,3-dichlorobenzenesulfonamide
*N*-[5-[[2-amino-2-(1-methyl-1*H*-imidazol-2-yl)ethyl]thio]-3-methoxypyrazinyl]-2,3-dichlorobenzenesulfonamide
(2*R*)-2-amino-3-[[3-chloro-5-[[(2,3-dichlorophenyl)sulfonyl]amino]-6-methoxypyrazinyl]thio]-*N*-methylpropanamide
(2*R*)-2-amino-3-[[5-[[(2,3-dichlorophenyl)sulfonyl]amino]-6-methoxy-3-methylpyrazinyl]thio]-*N*-methylpropanamide
*N*-[5-[[2-amino-2-(2-thiazolyl)ethyl]thio]-3-methoxypyrazinyl]-2,3-dichlorobenzenesulfonamide
*N*-[5-[[(2R)-2-Amino-3-hydroxypropyl]oxy]-3-methoxypyrazinyl]-2,3-dichlorobenzenesulfonamide, monohydrochloride
2,3-Dichloro-*N*-[5-[[(2*S*)-2,3-dihydroxypropyl]oxy]-3-methoxypyrazinyl]-benzenesulfonamide
2,3-Dichloro-*N*-[5-[[(2*R*)-2,3-dihydroxypropyl]oxy]-3-methoxypyrazinyl]-benzenesulfonamide
*N*-[5-[[(2*R*)-2-Amino-2-(3-methyl-1,2,4-oxadiazol-5-yl)ethyl]thio]-3-methoxypyrazinyl]-2,3-dichlorobenzenesulfonamide
*N*-[5-[[(2*R*)-2-Amino-2-(3-methyl-1,2,4-oxadiazol-5-yl)ethyl]thio]-3-methoxy-6-methylpyrazinyl]-2,3-dichlorobenzenesulfonamide
*N*-[5-[[(2*R*)-2-Amino-2-(3-methyl-1,2,4-oxadiazol-5-yl)ethyl]thio]-6-chloro-3-methoxypyrazinyl]-2,3-dichlorobenzenesulfonamide
and pharmaceutically acceptable salts thereof.

7. A pharmaceutical composition comprising a compound of formula (I), or a pharmaceutically acceptable salt or solvate thereof, as claimed any one of claims 1 to 6 in association with a pharmaceutically acceptable adjuvant, diluent or carrier.

8. A compound of formula (I), or a pharmaceutically acceptable salt or solvate thereof, as claimed in any one of claims 1 to 6 for use in therapy.

9. Use of a compound of formula (I), or a pharmaceutically acceptable salt or solvate thereof, as claimed in any one of claims 1 to 6 in the manufacture of a medicament for use in therapy.

10. Use of a compound of formula (I), or a pharmaceutically acceptable salt or solvate thereof, as claimed in any one of claims 1 to 6 in the manufacture of a medicament for use as a CCR4 antagonist.

11. A compound of formula (I), or a pharmaceutically acceptable salt or solvate thereof, as claimed in any one of claims 1 to 6 for use in the treatment of a disease where modulation of CCR4 activity is beneficial.

12. Use of a compound of formula (I), or a pharmaceutically acceptable salt or solvate thereof, as claimed in any one of claims 1 to 6 in the manufacture of a medicament for use in treating asthma.

13. A process for the preparation of a compound of formula (I) which comprises either
(a) reacting of a compound of formula (II): 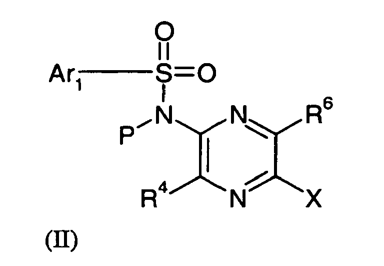 , wherein Ar¹, R⁴ and R⁶ are as defined above, P is a protecting group and X is a leaving group,
with a compound R⁵-H in the presence of a base, and optionally thereafter,
• removing any protecting groups
• forming a pharmaceutically acceptable salt:
(b) where the compound of formula (I) is of formula (Ia) 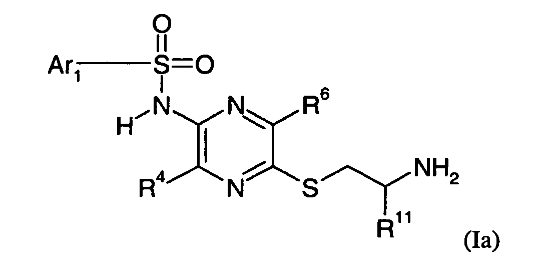 , wherein Ar¹, R⁴, R⁶ and R¹¹ are as defined above, reacting a compound of formula (X) 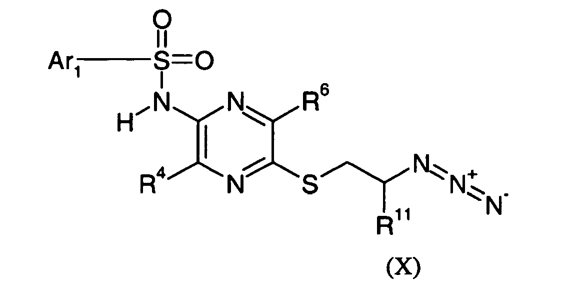 with a reducing agent such as triphenylphosphine in the presence of water, and optionally thereafter,
• removing any protecting groups
• forming a pharmaceutically acceptable salt.

## Patentansprüche

1. Verbindung der Formel (I) oder ein pharmazeutisch annehmbares Salz oder Solvat davon: 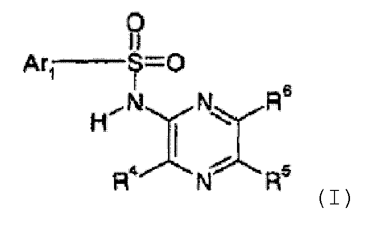 worin:
Ar¹ für Phenyl oder Thienyl steht, jeweils gegebenenfalls substituiert durch einen bis drei unter Halogen, Cyano, CF₃, OCF₃, O-C₁₋₆-Alkyl oder C₁₋₆-Alkyl ausgewählte Substituenten R¹, R² und R³;
R⁴ für C₁₋₆-Alkoxy, wobei die Alkylgruppe einen 3- bis 6-gliedrigen gesättigten Ring bilden kann oder durch 1 bis 3 Fluoratome oder eine Cyanogruppe substituiert sein kann, oder
O-C₁₋₆-Alkyl-R¹¹ oder O-C₂₋₆-Alkyl-X-R¹¹, wobei die Alkylgruppe einen 3-6-gliedrigen gesättigten Ring bilden kann und gegebenenfalls durch 1 bis 3 unter Hydroxy, Halogen, NR¹⁴R¹⁵, SR¹³, S(O)₂R¹³, S(O)R¹³ oder COR¹³ ausgewählte Gruppen substituiert ist, steht;
eine der Gruppen R⁵ oder R⁶ für XCH₂-C₁₋₄-Alkyl steht, wobei die Alkylgruppe in einer Position durch R¹¹ und entweder NR¹⁴R¹⁵ oder Hydroxy substituiert ist, oder R⁵/R⁶ für XR¹⁶, worin R¹⁶ einen 4-8-gliedrigen gesättigten Ring mit 1-3 unter Stickstoff, Sauerstoff oder Schwefel ausgewählten Heteroatomen, der gegebenenfalls durch 1-3 unter Hydroxy, Cyano, Halogen und =O ausgewählte Gruppen substituiert ist, bedeutet und durch R¹¹ substituiert ist, steht;
und die andere für Wasserstoff, Halogen, Amino, NH-C₁-₆-Alkyl, N(C₁₋₆-Alkyl)₂, C₁₋₆-Alkoxy oder C₁₋₆-Alkyl, gegebenenfalls substituiert durch eine oder mehrere Fluor- oder Hydroxylgruppen, steht;
X für NR¹³, O, S, S(O) oder S(O)₂ steht;
R¹¹ für eine Arylgruppe oder einen 5- bis 7-gliedrigen heteroaromatischen Ring mit 1-4 unter Stickstoff, Sauerstoff oder Schwefel ausgewählten Heteroatomen, jeweils gegebenenfalls substituiert durch 1 bis 3 unter Halogen, C(O)NR¹⁴R¹⁵, C(O)OR¹², Hydroxy, =O, =S, CN, NO₂, COR¹³, NR¹⁴R¹⁵, X-(CH₂)_{q}NR¹⁴R¹⁵, (CH₂)ₙNR¹⁴R¹⁵, (CH₂)ₙOH, SR¹³, S(O)R¹³, S(O)₂R¹³, C₁₋₆-Alkyl-X-C₁₋₆-alkyl, C₁₋₆-Alkyl oder C₁₋₆-Alkoxy, wobei die Alkylgruppe einen 3-6-gliedrigen Ring bilden kann oder gegebenenfalls durch 1-3 unter Hydroxy, Halogen, NR¹⁴R¹⁵, SR¹³, S(O)R¹³ und S(O)₂R¹³ ausgewählte Gruppen substituiert ist, ausgewählte Gruppen, steht; oder
R¹¹ für C(O)NR¹⁴R¹⁵, C(O)OR¹², CH₂OR¹² steht;
R¹² und R¹³ unabhängig voneinander für Wasserstoff oder C₁₋₆-Alkyl, wobei die Alkylgruppe durch 1-3 Fluoratome substituiert sein kann oder einen 3-6-gliedrigen gesättigten Ring bilden kann, stehen;
R¹⁴ und R¹⁵ unabhängig voneinander für Wasserstoff, C₁₋₆-Alkyl, C₃₋₆-Cycloalkyl, (CH₂)_{q}OH oder (CH₂)_{q}NH₂ stehen;
oder R¹⁴ und R¹⁵ gemeinsam mit dem Stickstoffatom, an das sie gebunden sind, einen 4-8-gliedrigen gesättigten Ring, der 1-3 unter Stickstoff, Sauerstoff und Schwefel ausgewählte Heteroatome enthält und gegebenenfalls durch C₁₋₆-Alkyl, C₁₋₆-Alkyl-OH oder Hydroxy substituiert ist, bilden und
n für 1, 2, 3, 4 oder 5 steht und
q für 2, 3, 4, 5 oder 6 steht.

2. Verbindung nach Anspruch 1, worin Ar¹ für gegebenenfalls durch ein oder mehrere Halogenatome substituiertes Phenyl steht.

3. Verbindung nach Anspruch 1 oder 2, worin R⁴ für Methoxy steht.

4. Verbindung nach einem der Ansprüche 1 bis 3, worin R⁵ für XCH₂CH(R¹¹)NR¹⁴R¹⁵, worin R¹¹ CO₂Me oder CONHMe oder einen 5- oder 6-gliedrigen Heterocyclus bedeutet und NR¹⁴R¹⁵ NH₂ oder NHMe bedeutet und X S oder O bedeutet, steht.

5. Verbindung nach einem der Ansprüche 1 bis 4, worin R⁶ für Wasserstoff, Chlor oder Methyl steht.

6. Verbindung der Formel (I), ausgewählt unter:
S-[5-[[(2,3-Dichlorphenyl)sulfonyl]amino]-6-methoxypyrazinyl]-D-cysteinmethylester
S-[5-[[(2,3-Dichlorphenyl)sulfonyl]amino]-6-methoxypyrazinyl]-L-cysteinmethylester
S-[5-[[(2,3-Dichlorphenyl)sulfonyl]amino]-6-methoxypyrazinyl]-L-cystein
(2*R*)-2-Amino-3-[[5-[[(2,3-dichlorphenyl)sulfonyl]-amino]-6-methoxypyrazinyl]thio]propanamid
(2*R*)-2-Amino-3-[[5-[[(2,3-dichlorphenyl)sulfonyl]-amino]-6-methoxypyrazinyl]thio]propanamid
(2*R*)-2-Amino-3-[[5-[[(2,3-dichlorphenyl)sulfonyl]-amino]-6-methoxypyrazinyl]thio]-*N*,*N*-dimethylpropanamid
*N*-[5-[[(2*R*)-2-Amino-3-hydroxypropyl]thio]-3-methoxypyrazinyl]-2,3-dichlorbenzolsulfonamid
*S*-[3-Chlor-5-[[(2,3-dichlorphenyl)sulfonyl]amino]-6-methoxypyrazinyl]-L-cysteinmethylester
*S*-[3-Chlor-5-[[(2,3-dichlorphenyl)sulfonyl]amino]-6-methoxypyrazinyl]-L-cystein
*N*-[5-[[(2*R*)-2-Amino-3-hydroxypropyl]thio]-6-chlor-3-methoxypyrazinyl]-2,3-dichlorbenzolsulfonamid
*S*-[5-[[(2,3-Dichlorphenyl)sulfonyl]amino]-6-methoxy-3-methylpyrazinyl]-L-cysteinmethylester
*S*-[5-[[(2,3-Dichlorphenyl)sulfonyl]amino]-6-methoxy-3-methylpyrazinyl]-L-cystein
*N*-(2-Aminoethyl)-*S*-[5-[[(2,3-dichlorphenyl)-sulfonyl]amino]-6-methoxypyrazinyl]-L-cysteinethylester
*N*-[5-[(2*R*)-2-Amino-2-phenylethoxy]-3-methoxypyrazinyl]-2,3-dichlorbenzolsulfonamid
2,3-Dichlor-*N*-[5-[[2-hydroxy-2-(2-thiazolyl)-ethyl]thio]-3-methoxypyrazinyl]benzolsulfonamid
2,3-Dichlor-*N*-[5-[[2-hydroxy-2-(1-methyl-1H-imidazol-2-yl)ethyl]thio]-3-methoxypyrazinyl]-benzolsulfonamid-Kaliumsalz
2,3-Dichlor-*N*-[5-[[2-hydroxy-2-(2-oxazolyl)-ethyl]thio]-3-methoxypyrazinyl]benzolsulfonamid
*N*-[5-[[2-Amino-2-(2-oxazolyl)ethyl]thio]-3-methoxypyrazinyl]-2,3-dichlorbenzolsulfonamid
2,3-Dichlor-*N*-[5-[(2,3-dihydroxypropyl)thio]-3-methoxypyrazinyl]benzolsulfonamid
2,3-Dichlor-*N*-[5-[(2-hydroxy-2-phenylethyl)thio]-3-methoxypyrazinyl]benzolsulfonamid
2,3-Dichlor-*N*-[5-[[2-hydroxy-2-(3-pyridinyl)-ethyl]thio]-3-methoxypyrazinyl]benzolsulfonamid
*N*-[5-[[2-Amino-2-(3-pyridinyl)ethyl]thio]-3-methoxypyrazinyl]-2,3-dichlorbenzolsulfonamid
2,3-Dichlor-*N*-[5-[[2-hydroxy-2-(4-pyridinyl)-ethyl]thio]-3-methoxypyrazinyl]benzolsulfonamid
2,3-Dichlor-*N*-[5-[[2-hydroxy-2-(2-pyridinyl)-ethyl]thio]-3-methoxypyrazinyl]benzolsulfonamid
3-[[5-[[(2,3-Dichlorphenyl)sulfonyl]amino]-6-methoxypyrazinyl]thio]-(2R)-2-hydroxypropansäuremethylester
*N*-[5-[[2-Amino-2-(2-pyridinyl)ethyl]thio]-3-methoxypyrazinyl]-2,3-dichlorbenzolsulfonamid
*N*-[5-[[2-Amino-2-(1-methyl-1*H*-imidazol-2-yl)-ethyl]thio]-3-methoxypyrazinyl]-2,3-dichlorbenzolsulfonamid
(2*R*)-2-Amino-3-[[3-chlor-5-[[(2,3-dichlorphenyl)-sulfonyl]amino]-6-methoxypyrazinyl]thio]-*N*-methylpropanamid
(2*R*)-2-Amino-3-[[5-[[(2,3-dichlorphenyl)sulfonyl]-amino]-6-methoxy-3-methylpyrazinyl]thio]-*N*-methylpropanamid
*N*-[5-[[2-Amino-2-(2-thiazolyl)ethyl]thio]-3-methoxypyrazinyl]-2,3-dichlorbenzolsulfonamid
*N*-[5-[[(2*R*)-2-Amino-3-hydroxypropyl]oxy]-3-methoxypyrazinyl]-2,3-dichlorbenzolsulfonamidmonohydrochlorid
2,3-Dichlor-*N*-[5-[[(2*S*)-2,3-dihydroxypropyl]oxy]-3-methoxypyrazinyl]benzolsulfonamid
2,3-Dichlor-*N*-[5-[[(2*R*)-2,3-dihydroxypropyl]oxy]-3-methoxypyrazinyl]benzolsulfonamid
*N*-[5-[[(2*R*)-2-Amino-2-(3-methyl-1,2,4-oxadiazol-5-yl)ethyl]thio]-3-methoxypyrazinyl]-2,3-dichlorbenzolsulfonamid
*N*-[5-[[(2*R*)-2-Amino-2-(3-methyl-1,2,4-oxadiazol-5-yl)ethyl]thio]-3-methoxy-6-methylpyrazinyl]-2,3-dichlorbenzolsulfonamid
*N*-[5-[[(2*R*)-2-Amino-2-(3-methyl-1,2,4-oxadiazol-5-yl)ethyl]thio]-6-chlor-3-methoxypyrazinyl]-2,3-dichlorbenzolsulfonamid
und pharmazeutisch annehmbaren Salzen davon.

7. Pharmazeutische Zusammensetzung, die eine Verbindung der Formel (I) oder ein pharmazeutisch annehmbares Salz oder Solvat davon nach einem der Ansprüche 1 bis 6 zusammen mit einem pharmazeutisch annehmbaren Hilfsstoff, Verdünnungsmittel oder Träger enthält.

8. Verbindung der Formel (I) oder ein pharmazeutisch annehmbares Salz oder Solvat davon nach einem der Ansprüche 1 bis 6 zur Verwendung bei der Therapie.

9. Verwendung einer Verbindung der Formel (I) oder eines pharmazeutisch annehmbaren Salzes oder Solvats davon nach einem der Ansprüche 1 bis 6 bei der Herstellung eines Arzneimittels zur Verwendung bei der Therapie.

10. Verwendung einer Verbindung der Formel (I) oder eines pharmazeutisch annehmbaren Salzes oder Solvats davon nach einem der Ansprüche 1 bis 6 bei der Herstellung eines Arzneimittels zur Verwendung als CCR4-Antagonist.

11. Verbindung der Formel (I) oder ein pharmazeutisch annehmbares Salz oder Solvat davon nach einem der Ansprüche 1 bis 6 zur Verwendung bei der Behandlung einer Krankheit, bei der die Modulierung der CCR4-Aktivität von Vorteil ist.

12. Verwendung einer Verbindung der Formel (I) oder eines pharmazeutisch annehmbaren Salzes oder Solvats davon nach einem der Ansprüche 1 bis 6 bei der Herstellung eines Arzneimittels zur Verwendung bei der Behandlung von Asthma.

13. Verfahren zur Herstellung einer Verbindung der Formel (I), bei dem man entweder
(a) eine Verbindung der Formel (II): 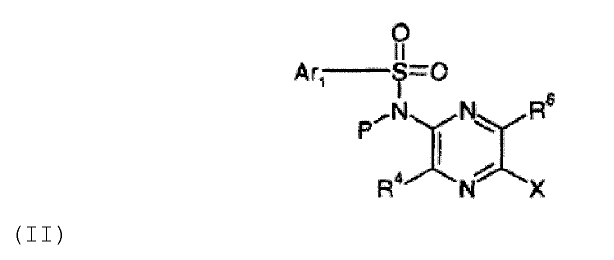 worin Ar¹, R⁴ und R⁵ die oben angegebene Bedeutung besitzen, P für eine Schutzgruppe steht und X für eine Abgangsgruppe steht,
in Gegenwart einer Base mit einer Verbindung R⁵-H umsetzt und gegebenenfalls danach
• jegliche Schutzgruppen abspaltet;
• ein pharmazeutisch annehmbares Salz bildet;
(b) wo die Verbindung der Formel (I) die Formel (Ia) 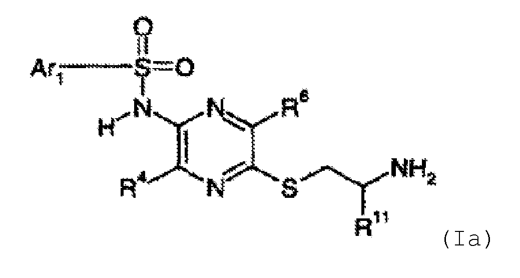
worin Ar¹, R⁴, R⁶ und R¹¹ die oben angegebene Bedeutung besitzen, aufweist, eine Verbindung der Formel (X) 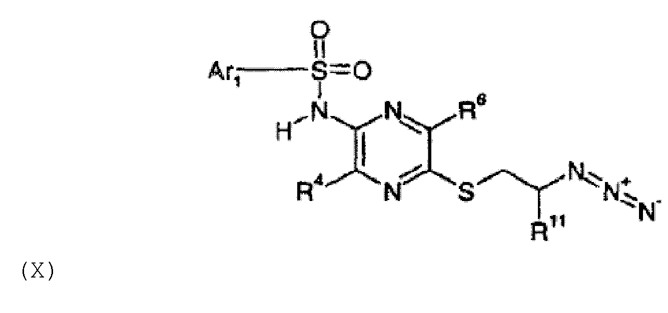 mit einem geeigneten Reduktionsmittel wie Triphenylphosphin in Gegenwart von Wasser umsetzt und gegebenenfalls danach
• jegliche Schutzgruppen abspaltet;
• ein pharmazeutisch annehmbares Salz bildet.

## Revendications

1. Composé de formule (I) ou un sel ou solvate pharmaceutiquement acceptable de celui-ci : 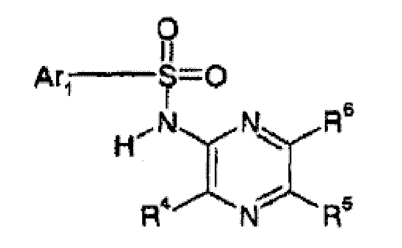 dans lequel :
Ar¹ est un phényle ou un thiényle, dont chacun est facultativement substitué par un à trois substituants R¹, R² et R³ choisis parmi un halogène, un cyano, CF₃, OCF₃, O-(alkyle en C₁₋₆) ou un alkyle en C₁₋₆ ;
R⁴ est un alcoxy en C₁₋₆ où le groupe alkyle peut former un cycle saturé de 3 à 6 chaînons ou peut être substitué avec 1 à 3 atomes de fluor ou un groupe cyano ; ou
O-(alkyle en C₁₋₆)-R¹¹, ou O(alkyle en C₂₋₆)-X-R¹¹ où le groupe alkyle peut former un cycle saturé de 3 à 6 chaînons et est facultativement substitué avec 1 à 3 groupes choisis parmi un hydroxy, un halogène, NR¹⁴R¹⁵, SR¹³, S(O)₂R¹³, S(O)R¹³ ou COR¹³;
l'un de R⁵ ou R⁶ est XCH₂-(alkyle en C₁₋₄) où le groupe alkyle est substitué à une position quelconque par les deux groupes R¹¹ et NR¹⁴R¹⁵ ou un hydroxy, ou R⁵/R⁶ est XR¹⁶ où R¹⁶ est un cycle saturé de 4 à 8 chaînons contenant de 1 à 3 hétéroatomes choisis parmi l'azote, l'oxygène ou le soufre et facultativement substitué avec 1 à 3 groupes choisis parmi un hydroxy, un cyano, un halogène et =O, et R¹⁶ est substitué par R¹¹ ;
et l'autre est un hydrogène, un halogène, un amino, NH-(alkyle en C₁₋₆), N-(alkyle en C₁₋₆)₂, un alcoxy en C₁₋₆ ou un alkyle en C₁₋₆ facultativement substitué par un ou plusieurs groupes fluoro ou hydroxyle ;
X est NR¹³, O, S, S(O), S(O)₂, ou une liaison ;
R¹¹ est un groupe aryle ou un cycle hétéroaromatique de 5 à 7 chaînons contenant de 1 à 4 hétéroatomes choisis parmi l'azote, l'oxygène ou le soufre, ledit groupe aryle ou cycle hétéroaromatique pouvant être facultativement substitué par 1 à 3 groupes choisis parmi un halogène, C(O)NR¹⁴R¹⁵, C(O)OR¹², un hydroxy, =O, =S, CN, NO₂, COR¹³, NR¹⁴R¹⁵, X(CH₂)_{q}NR¹⁴R¹⁵, (CH₂)ₙNR¹⁴R¹⁵, (CH₂)ₙOH, SR¹³, S(O)R¹³, S(O)₂R¹³, (alkyle en C₁₋₆) -X-(alkyle en C₁₋₆), un alkyle en C₁₋₆ ou un alcoxy en C₁₋₆ où le groupe alkyle peut former un cycle de 3 à 6 chaînons ou est facultativement substitué avec 1 à 3 groupes choisis parmi un hydroxy, un halogène, NR¹⁴R¹⁵, SR¹³, S(O)R¹³, S(O)₂R¹³; ou
R¹¹ est C(O)NR¹⁴R¹⁵, C(O)OR¹², CH₂OR¹² ;
R¹² et R¹³ sont indépendamment un hydrogène ou un alkyle en C₁₋₆ où le groupe alkyle peut être substitué avec 1 à 3 atomes de fluor ou peut former un cycle de 3 à 6 chaînons saturé ;
R¹⁴ et R¹⁵ sont indépendamment un hydrogène, un alkyle en C₁₋₆, un cycloalkyle en C₃₋₆, (CH₂)_{q}OH ou (CH₂)_{q}NH₂,
ou R¹⁴ et R¹⁵ conjointement avec l' atome d'azote auquel ils sont liés forment un cycle saturé de 4 à 8 chaînons contenant 1 à 3 hétéroatomes choisis parmi l'azote, l'oxygène et le soufre et facultativement substitué par un alkyle en C₁₋₆, (alkyle en C₁₋₆)-OH, ou un hydroxy ; et
n est 1, 2, 3, 4 ou 5 ; et
q est 2, 3, 4, 5 ou 6.

2. Composé selon la revendication 1 dans lequel Ar¹ est un phényle facultativement substitué par un ou plusieurs atomes d'halogène.

3. Composé selon la revendication 1 ou 2 dans lequel R⁴ est un méthoxy.

4. Composé selon l'une quelconque des revendications 1 à 3 dans lequel R⁵ est XCH₂CH(R¹¹)NR¹⁴R¹⁵ où R¹¹ est CO₂Me ou CONHMe ou un hétérocycle de 5 ou 6 chaînons et NR¹⁴R¹⁵ est NH₂ ou NHMe et X est S ou O.

5. Composé selon l'une quelconque des revendications 1 à 4 dans lequel R⁶ est un hydrogène, un chloro ou un méthyle.

6. Composé de formule (I) choisi parmi:
ester méthylique de S-[5-[[(2,3-dichlorophényl)-sulfonyl]amino]-6-méthoxypyrazinyl]-D-cystéine,
ester méthylique de S-[5-[[(2,3-dichlorophényl)-sulfonyl]amino]-6-méthoxypyrazinyl]-L-cystéine,
S-[5-[[(2,3-dichlorophényl)sulfonyl]amino]-6-méthoxypyrazinyl]-L-cystéine,
(2*R*)-2-amino-3-[[5-[[(2,3-dichlorophényl)-sulfonyl]amino]-6-méthoxypyrazinyl]thio]propanamide,
(2*R*)-2-amino-3-[[5-[[(2,3-dichlorophényl)-sulfonyl]amino]-6-méthoxypyrazinyl]thio]-propanamide,
(2*R*)-2-amino-3-[[5-[[(2,3-dichlorophényl)-sulfonyl]amino]-6-méthoxypyrazinyl]thio]-N,N-diméthylpropanamide,
*N*-[5-[[(2*R*)-2-amino-3-hydroxypropyl]thio]-3-méthoxypyrazinyl]-2,3-dichloro-benzènesulfonamide,
ester méthylique de *S*-[3-chloro-5-[[(2,3-dichlorophényl)sulfonyl]amino]-6-méthoxypyrazinyl]-L-cystéine,
*S*-[3-chloro-5-[[(2,3-dichlorophényl)sulfonyl]-amino]-6-méthoxypyrazinyl]-L-cystéine,
*N*-[5-[[(2*R*)-2-amino-3-hydroxypropyl]thio]-6-chloro-3-méthoxypyrazinyl]-2,3-dichlorobenzènesulfonamide,
ester méthylique de S-[5-[[(2,3-dichlorophényl)-sulfonyl]amino]-6-méthoxy-3-méthylpyrazinyl]-L-cystéine,
*S*-[5-[[(2,3-dichlorophényl)sulfonyl]amino]-6-méthoxy-3-méthylpyrazinyl]-L-cystéine,
ester éthylique de N-(2-aminoéthyl)-S-[5-[[(2,3-dichlorophényl)sulfonyl]amino]-6-méthoxypyrazinyl]-L-cystéine,
*N*-[5-[(2*R*)-2-amino-2-phényléthoxy]-3-méthoxypyrazinyl]-2,3-dichlorobenzènesulfonamide,
2,3-dichloro-N-[5-[[2-hydroxy-2-(2-thiazolyl)-éthyl]thio]-3-méthoxypyrazinyl]-benzènesulfonamide,
2,3-dichloro-*N*-[5-[[2-hydroxy-2-(1-méthyl-1*H-*imidazol-2-yl)éthyl]thio]-3-méthoxypyrazinyl]-benzènesulfonamide, sel de potassium,
2,3-dichloro-N-[5-[[2-hydroxy-2-(2-oxazolyl)-éthyl]thio]-3-méthoxypyrazinyl]-benzènesulfonamide,
*N*-[5-[[2-amino-2-(2-oxazolyl)éthyl]thio]-3-méthoxypyrazinyl]-2,3-dichlorobenzènesulfonamide,
2,3-dichloro-*N*-[5-[(2,3-dihydroxypropyl)thio]-3-méthoxypyrazinyl]benzènesulfonamide,
2,3-dichloro-*N*-[5-[(2-hydroxy-2-phényléthyl)thio]-3-méthoxypyrazinyl]benzènesulfonamide,
2,3-dichloro-*N*-[5-[[2-hydroxy-2-(3-pyridinyl)-éthyl]thio]-3-méthoxypyrazinyl]benzènesulfonamide,
*N*-[5-[[2-amino-2-(3-pyridinyl)éthyl]thio]-3-méthoxypyrazinyl]-2,3-dichlorobenzènesulfonamide,
2,3-dichloro-*N*-[5-[[2-hydroxy-2-(4-pyridinyl)-éthyl]thio]-3-méthoxypyrazinyl]benzènesulfonamide,
2,3-dichloro-*N*-[5-[[2-hydroxy-2-(2-pyridinyl)-éthyl]thio]-3-méthoxypyrazinyl]benzènesulfonamide,
ester méthylique d'acide 3-[[5-[[(2,3-dichlorophényl)sulfonyl]amino]-6-méthoxypyrazinyl]thio]-(2R)-2-hydroxypropanoïque,
*N*-[5-[[2-amino-2-(2-pyridinyl)éthyl]thio]-3-méthoxypyrazinyl]-2,3-dichlorobenzènesulfonamide,
*N*-[5-[[2-amino-2-(1-méthyl-1H-imidazol-2-yl)éthyl]thio]-3-méthoxypyrazinyl]-2,3-dichlorobenzènesulfonamide,
(2R)-2-amino-3-[[3-chloro-5-[[(2,3-dichlorophényl)sulfonyl]amino]-6-méthoxypyrazinyl]thio]-N-méthylpropanamide,
(2*R*)-2-amino-3-[[5-[[(2,3-dichlorophényl)-sulfonyl]amino]-6-méthoxy-3-méthylpyrazinyl]thio]-N-méthylpropanamide,
*N*-[5-[[2-amino-2-(2-thiazolyl)éthyl]thio]-3-méthoxypyrazinyl]-2,3-dichlorobenzènesulfonamide,
*N*-[5-[[(2*R*)-2-amino-3-hydroxypropyl]oxy]-3-méthoxypyrazinyl]-2,3-dichlorobenzènesulfonamide, monochlorhydrate,
2,3-dichloro-N-[5-[[(2S)-2,3-dihydroxypropyl]oxy]-3-méthoxypyrazinyl]-benzènesulfonamide,
2,3-dichloro-N-[5-[[(2R)-2,3-dihydroxypropyl]oxy]-3-méthoxypyrazinyl]-benzènesulfonamide,
*N*-[5-[[(2*R*)-2-amino-2-(3-méthyl-1,2,4-oxadiazol-5-yl)éthyl]thio]-3-méthoxypyrazinyl]-2,3-dichlorobenzènesulfonamide,
*N*-[5-[[(2*R*)-2-amino-2-(3-méthyl-1,2,4-oxadiazol-5-yl)éthyl]thio]-3-méthoxy-6-méthylpyrazinyl]-2,3-dichlorobenzènesulfonamide,
*N*-[5-[[(2*R*)-2-amino-2-(3-méthyl-1,2,4-oxadiazol-5-yl)éthyl]thio]-6-chloro-3-méthoxypyrazinyl]-2,3-dichlorobenzènesulfonamide
et des sels pharmaceutiquement acceptables de ceux-ci.

7. Composition pharmaceutique comprenant un composé de formule (I), ou un sel ou solvate pharmaceutiquement acceptable de celui-ci, selon l'une quelconque des revendications 1 à 6 en association avec un adjuvant, diluant ou véhicule pharmaceutiquement acceptable.

8. Composé de formule (I), ou un sel ou solvate pharmaceutiquement acceptable de celui-ci, selon l'une quelconque des revendications 1 à 6 pour utilisation en thérapie.

9. Utilisation d'un composé de formule (I), ou un sel ou solvate pharmaceutiquement acceptable de celui-ci, selon l'une quelconque des revendications 1 à 6 dans la fabrication d'un médicament pour utilisation en thérapie.

10. Utilisation d'un composé de formule (I), ou un sel ou solvate pharmaceutiquement acceptable de celui-ci, selon l'une quelconque des revendications 1 à 6 dans la fabrication d'un médicament pour utilisation en tant qu'antagoniste de CCR4.

11. Composé de formule (I), ou un sel ou solvate pharmaceutiquement acceptable de celui-ci, selon l'une quelconque des revendications 1 à 6 pour utilisation dans le traitement d'une maladie dans laquelle la modulation de l'activité CCR4 est bénéfique.

12. Utilisation d'un composé de formule (I), ou un sel ou solvate pharmaceutiquement acceptable de celui-ci, selon l'une quelconque des revendications 1 à 6 dans la fabrication d'un médicament pour utilisation dans le traitement de l'asthme.

13. Procédé pour la préparation d'un composé de formule (I) qui comprend
(a) la réaction d'un composé de formule (II) : 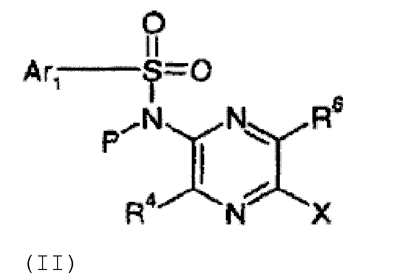 dans lequel Ar¹, R⁴ et R⁶ sont comme défini ci-dessus, P est un groupe protecteur et X est un groupe partant,
avec un composé R⁵-H en présence d'une base, et facultativement ensuite,
• l'élimination des groupes protecteurs éventuels,
• la formation d'un sel pharmaceutiquement acceptable ;
(b) lorsque le composé de formule (I) est de formule (Ia) 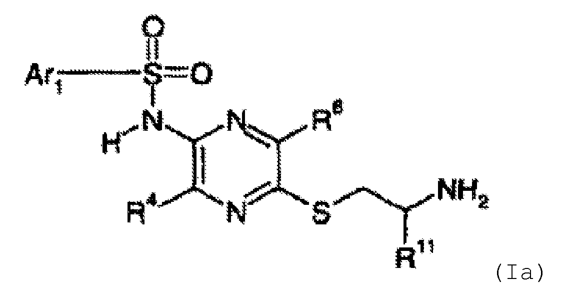
dans lequel Ar¹, R⁴, R⁶ et R¹¹ sont comme défini ci-dessus, la réaction d'un composé de formule (X) 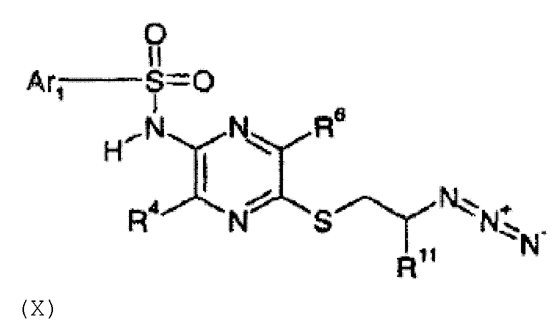 avec un agent réducteur tel que la triphénylphosphine en présence d'eau, et facultativement ensuite,
• l'élimination des groupes protecteurs éventuels,
• la formation d'un sel pharmaceutiquement acceptable.
